(19) Europäisches Patentamt
European Patent Office
Office européen des brevets

(11) EP 1 732 935 B1

(12) **EUROPÄISCHE PATENTSCHRIFT**

(45) Veröffentlichungstag und Bekanntmachung des Hinweises auf die Patenterteilung:
**12.12.2007 Patentblatt 2007/50**

(21) Anmeldenummer: **05746647.6**

(22) Anmeldetag: **30.04.2005**

(51) Int Cl.:
*C07F 5/00* (2006.01) *G01N 21/64* (2006.01)
*C09B 44/00* (2006.01) *C07C 237/04* (2006.01)
*C07D 471/04* (2006.01)

(86) Internationale Anmeldenummer:
**PCT/DE2005/000804**

(87) Internationale Veröffentlichungsnummer:
**WO 2005/108405 (17.11.2005 Gazette 2005/46)**

(54) **LANTHANOIDCHELATE UND IHRE ANWENDUNG IN DER BIOANALYTIK**

LANTHANIDE CHELATES AND USE THEREOF IN BIOANALYSIS

CHELATES DE LANTHANIDE ET LEUR APPLICATION EN BIOANALYTIQUE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **07.05.2004 DE 102004022628**

(43) Veröffentlichungstag der Anmeldung:
**20.12.2006 Patentblatt 2006/51**

(73) Patentinhaber: **Sensient Imaging Technologies GmbH**
**06766 Wolfen (DE)**

(72) Erfinder:
- **MARX, Jörg**
**D-06847 Dessau (DE)**
- **SCHUMER, Frank**
**04103 Leipzig (DE)**
- **LISCHEWSKI, Regina**
**06766 Wolfen (DE)**
- **ZECKERT, Kornelia**
**04157 Leipzig (DE)**
- **BÖHME, Hans-Joachim**
**04329 Leipzig (DE)**
- **HENNING, Horst**
**04103 Leipzig (DE)**

(74) Vertreter: **Nenning, Peter**
**Riesaer Strasse 164**
**04319 Leipzig (DE)**

(56) Entgegenhaltungen:
**WO-A-00/01663**
**WO-A-03/035655**
**US-A- 4 808 541**
**US-A- 4 920 195**
**US-A- 5 032 677**

EP 1 732 935 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft Lanthanoidchelate, ihre Herstellung und Charakterisierung und ihre Verwendung in der Bioanalytik, bevorzugt in der Fluoreszenzspektroskopie.

Es ist bereits bekannt, Lanthanoidkomplexe in der Fluoreszenzspektroskopie einzusetzen. US 4,374,120 stellt Eu- und Tb-Chelate als Fluoreszenzmarker vor mit einer relativ langen Fluoreszenzzeit von 50 bis 1000 Mikrosekunden, Liganden sind u.a. Aminopolycarbonsäuren. Es ist weiterhin bekannt, dass einige Lanthanoid-Fluoreszenz-Chelatkomplexe sich für die zeitaufgelöste Fluorometrie besonders eignen, wobei Tb(III)-BPTA-NHS und Eu(III)-Östrogen bevorzugt sind und ersteres in einem DNA-Hybridisierungs-Assay Verwendung findet (Matsumoto, K. et al., RIKEN Review 35, (May, 2001). LanthanoidChelate finden nach WO 00/01663 Verwendung in der HTRF (homogeneous time-resolved fluoreszenz)-Assays. Bekannt sind Cyanin- und Indocyanin-Farbstoffe zur Verwendung in der Biomedizin (US 6,217,848, US 6,190,641 mit weiteren Nachweisen). DE 42 22 255 beschreibt Markierungsreagenzien mit Lanthanoidionen-chelatisierender Struktur zum Einsatz in der Gensondendiagnostik. Bevorzugt als Lanthanoidionenchelatisierende Struktur sind Pyridinderivate ***(u.a.*** US 5,032,677***),*** Spacer sind Polyalkylamin, Polyethylenglykol, fotosensibel sind Furocumarinderivate. In einem Artikel im Journal of Alloys and Compounds 1995, 225, 511-14 beschreiben Takalo,H. et al. auf Seite 112 Tb(III) und Eu(III)-chelate und ihre Luminszenzausbeute. Zur Bestimmung von Phosphorylierungsaktivitäten werden in der DE 698 13 850 Kryptate verwendet, die ein Seltenerdmolekül wie Tb, Eu, Sm, Dy, Nd enthalten, einen Komplexbildner wie Bispyridin und die als fluoreszierende Donatorverbindung eingesetzt werden. Hemmilää, I. und Webb, S. beschreiben in DDT, 1997, 2, 373-381 Prinzipien der time-resolved fluormetry TRF mit Lanthanoid-Chelaten zur Drogenscreening. In der DE 102 59 677 wird eine Sonde zum Nachweis von Nukleinsäuren vorgestellt, die in einer bevorzugten Ausführungsform Seltene-Erden-Farbstoffe als Fluorophore einsetzt.

Zu einer Verwendung in der Bioanalytik durch Messen der Energieübertragung von einem Donor auf eine Akzeptor ist es erforderlich, Verbindungen zur Verfügung zu haben, die zu dieser Energieübertragung fähig sind. Eine Möglichkeit besteht im Fluoreszenz-Resonanz-Energie-Transfer (FRET) als einer besonderen Form der Energieübertragung, die auf einer Wechselwirkung zweier räumlich voneinander getrennter Dipole basiert, wovon einer (Donor) elektronisch angeregt ist. Stehen beide Dipole in Resonanz zueinander, kann die Anregungsenergie des fluoreszierenden Donors strahlungslos auf einen Akzeptor übertragen werden. Aufgrund seiner hohen Empfindlichkeit und der starken Abhängigkeit vom Abstand zwischen Donor und Akzeptor hat FRET eine vielseitige Anwendung in der Erkennung und Charakterisierung biologisch relevanter Substrate gefunden. Eine aktuelle Entwicklung betrifft die Anwendung von FRET-Systemen in homogenen Fluoreszenz-Assays (Sequenz-, Faltungsmarkungsmarkierung (Proteine), DNS). Hierbei werden die in Reaktion gebrachten Antigene und Antikörper mit einer fluorophoren Gruppe markiert, bei der das eine Fluorophor als Energie-Donor, das andere als korrespondierender Energie-Akzeptor fungiert.

Eine umfassende Anwendung des FRET-Prinzips zum direkten Nachweis von Molekül-Molekül-Wechselwirkungen in der klinischen Labordiagnostik sowie auch in der kombinatorischen Pharmasynthese setzt die Verfügbarkeit solcher Donoren und Akzeptoren voraus, die durch ein effizientes spektroskopisches Absorptions- und Emissionsverhalten im langweiligen Spektralbereich gekennzeichnet sind, wobei die Anregungswellenlänge des Donors > 350 nm betragen muß, um eine Anregung von biologischem Substrat zu vermeiden. Ein weiteres wesentliches Kriterium für ihre Anwendbarkeit in biologischen Testverfahren ist dabei die Ausbildung einer stabilen, kovalenten Biopolymer-Fluorophor-Bindung, ohne dass die Stabilität und die biologische Aktivität des markierten Moleküls dabei beeinträchtigt werden.

In Ansehen des Bedarfs an Erkenntnissen auf diesem Gebiet wird nach passenden Donor-Akzeptor-Systemen gesucht, die den Anforderungen an Genauigkeit und kurzfristiger Datenverfügbarkeit entsprechen, denn die bekannten Donor-Akzeptorsysteme haben einige Nachteile. So ist die Energieübertragung von Donor auf einen Akzeptor häufig nicht befriedigend. Darunter leidet aber die Empfindlichkeit der analytischen Verfahren insgesamt. Ferner reicht die Komplexstabilität des Donors häufig nicht aus und seine Wasserlöslichkeit ist unzureichend.

Die Ursachen der geschilderten Nachteile liegen zunächst im Aufbau, das heißt in der Struktur der Donorverbindungen. Es ist bisher nicht gelungen, Verbindung von hoher Stabilität der Donorverbindungen mit den geforderten spektroskopischen Eigenschaften von Absorption und Emission zu schaffen. Weiterhin sind die bisher verwendeten Akzeptorfarbstoffe nicht die Verbindungen der Wahl, weil sie bezüglich ihres Absorptionsmaximums in bezug auf das Emissionsspektrum des Donors nicht abgestimmt sind und es bisher nicht gelungen ist, eine ausreichende Löslichkeit der Akzeptorfarbstoffe zu gewährleisten.

Für eine gezielte Anwendung der Fluorophore liegt der vorrangige Entwicklungsanspruch bei der Synthese neuer Verbindungen in der Effizienzerhöhung der Energieübertragung vom Donor auf den entsprechenden Akzeptor und damit in einer Erhöhung der Empfindlichkeit des analytischen Verfahrens. Diesem Anspruch sollte durch Einsatz eines Lanthanoidkomplexes als Energie-Donor Rechnung getragen werden können, in dem eine Polyaminopolycarbonsäure mit speziellem, auf die Anwendung bezogenen chromophoren Rest das Lanthanoid(III)ion chelatisiert. Die Komplexierung des eigentlichen Fluorophors, dem Lanthanoid(III)ion, durch eine Polyaminopolycarbonsäure sollte eine hohe Komplexstabilität des verwendeten Donors und gute Löslichkeit im wässrigen Medium gewährleisten. Die Besonderheit dieser erstmals genutzten Lanthanoid(III)-Komplexe liegt in der Modifizierung des Ligandensystems durch Substituenten (im weiteren mit Antenne bezeichnet), die im vorgesehenen Wellenlängenbereich absorbieren und somit auf die Anregungswellenlänge abgestimmt werden können.

Die Verwendung von Lanthanoid(III)-Verbindungen als Energie-Donoren in der Fluoroszenzanalytik sollte im Vergleich zu organischen Donoren spektroskopische Vorteile in bezug auf Empfindlichkeit und Signal-Rausch-Verhältnis bieten und müßte somit eine Vielzahl neuer Anwendungsaspekte öffnen, insbesondere auch in der kombinatorischen Pharmasynthese. Grund dafür sind die für Lanthanoid(III)-Komplexe charakteristischen spektroskopischen Eigenschaften, wie die große STOKES- Verschiebung, die linienartige Emission verbunden mit hoher Intensität sowie langen Lebenszeiten der angeregten Zustände. Ein entscheidender Vorteil von Lanthanoid(III)-Verbindungen liegt des weiteren neben einer zeitaufgelösten Spektrenaufnahme in günstigen Donor-Akzeptor-Abständen, die eine bessere Signalseparation und -intensivierung gegenüber unvollständig markiertem Substrat ermöglichen und generell große Label erlauben.

Die Aufgabe der Erfindung besteht in der Darstellung von zur Energieübertragung befähigten Lanthanoid (III)-Chelatkomplexen als Donor, gekennzeichnet von einer hohen Stabilität sowie günstigen spektroskopischen Eigenschaften von Absorption und Emission im sichtbaren Spektralbereich.

Für die Testung der Lanthanoid(III)-Komplexe hinsichtlich ihrer Donoreigenschaften liegt die weitere Aufgabe in der Wahl und synthetischen Abwandlung für den Donor spektroskopisch geeigneter Akzeptorfarbstoffe und in der sich anschließenden Fluoreszenz-Untersuchung des Donor-Akzeptor-Paares in homogener Lösung. Als Akzeptorfarbstoffe werden sowohl neue als auch an sich bekannte Akzeptorfarbstoffe wie z.B. Rhodamin-, Polymethinfarbstoffe verwendet, abgestimmt auf die Lage ihres Absorptionsmaximums im Bezug auf das Emissionsspektrum des Donors, ihrer Emissionsintensität und Löslichkeit in Wasser.

Voraussetzung für eine erfolgreiche Markierung des Donors und Akzeptors ist das Vorhandensein geeigneter Haftgruppen am jeweiligen Fluorophor, um eine kovalente Bindung zum Substratmolekül zu ermöglichen. Deshalb ist im weiteren eine entsprechende Funktionalisierung des Donors bzw. Akzeptors erforderlich.

Abschließend soll mit dem funktionalisierten Donor-Akzeptor-Paar orientierend ein homogener Bioassay an einer realen und forschungsrelevanten biochemischen Problemstellung aufgebaut und der Nachweis der prinzipiellen Eignung dieses Donor-Akzeptor-Paares für die Erkennung biologischer Molekül-Molekül-Wechselwirkungen erbracht werden.

Die Aufgabe besteht deshalb darin, ein System zu schaffen, in welchem eine Energieübertragung von einer Antenne auf ein Lanthanoid(III)ion-Chelatbildner ermöglicht wird. Die praktische Anwendung ist dadurch gegeben, dass Haftgruppen entweder an der Antenne oder an dem Chelatbildner jeweils die Verbindung zu Biomolekülen herstellen und die Energieübertragung in diesem Donor-Akzeptor-System gemessen werden kann.

Dann sind Rückschlüsse auf die Eigenschaften und das Verhalten dieser Biomoleküle möglich. Möglichkeiten der Erfassung solcher Messdaten sind unter anderem das FRET-System.

Trotz intensiver weltweiter Forschungen auf diesem Gebiet und einiger erfolgversprechender Ansätze in Einzelfragen gibt es keine geschlossene Theorie, welche die Energieübertragung von der Antenne auf den Emitter erläutert und eine Arbeitshypothese aufzustellen erlaubt. Es ist nicht vorhersehbar, welche Antenne besonders geeignet sein könnte. Orientiert man sich hilfsweise am Triplettzustand des Antennenchromophors, der energetisch in einem bestimmten Bereich liegt und stellt den entsprechenden Antennenkomplex her, wird man enttäuscht, weil sich das Triplett-Niveau mit der Komplexierung der Seltenen Erden dramatisch ändert. Es ist ebenso wenig vorhersehbar, ob das von uns verwendete Chromophor 1,10- Phenanthrolin - substituiert oder nicht substituiert - in der Lage ist, die empfangene Energie auf das SE(III)-Zentrum zu übertragen.

Die Aufgabe wird erfindungsgemäß dadurch gelöst, dass in einem ersten Schritt Antennenkomplexe von Europium(III) und Terbium(III) hergestellt werden. Zu diesem Zweck werden zunächst Antennenliganden hergestellt.

Das Gerüst des angestrebten Antennenliganden besteht aus dem eigentlichen Chelatliganden, der das Metallion koordiniert und gleichzeitig die für eine Anwendung von Lanthanoid(III)-Verbindungen als Donoren notwendige hohe Stabilität garantiert. Zum anderen soll der Chelatligand über eine chromophore Gruppe, die Antenne, verfügen, die eine Absorption des Donors im sichtbaren Spektralbereich ermöglicht.

Es sind bereits fluoreszierende Lanthanoid(III)-Verbindungen bekannt, deren Stabilität in ihrer Komplexierung durch *O*-funktionalisierte Ligandensysteme begründet liegt. Erfindungsgemäß für den angestrebten Anwendungsaspekt ist die Wahl einer Polyaminopolycarbonsäure, im speziellen die Diethylentriaminpentaessigsäure (**$LH_5$**). Neben der hohen Komplexstabilität zeigen Lanthanoidchelate von Polyaminopolycarbonsäuren auch eine sehr gute Löslichkeit in wässrigen Medien. Durch die Vielzähnigkeit kann trotz Einführung einer chromophoren Gruppe eine koordinative Absättigung von Europium(III)- oder Terbium(III)ionen erreicht werden. Weiterhin können zusätzliche funktionelle Gruppen als Haftgruppen eingeführt werden, welche die Fixierung eines biologischen Analyten ermöglichen.

Die aus 4-Ethinylanilin und der chromophoren Gruppe zusammengesetzte Antenne bestimmt mit ihren photophysikalischen Eigenschaften die Anregungswellenlänge und die Effizienz der Energieübertragung auf das sowohl in unkomplexierter als auch in mit **$LH_5$** komplexierter Form im relevanten Spektralbereich nicht absorbierende Lanthanoid (III)ion. Für eine Anwendung von Lanthanoid(III)-Verbindungen als Lumineszenz-Marker in biologischen Systemen ist

dabei von besonderem Vorteil, dass dessen Anregung im sichtbaren Spektralbereich möglich ist, wodurch eine Direktanregung von zum Teil selbst zur Fluoreszenz befähigten biologischen Substraten ausgeschlossen werden kann. Deshalb werden erfindungsgemäß solche Chromophore in die zum Beispiel Diethylentriaminpentaessigsäure eingeführt, die im Spektralbereich oberhalb von 350 nm absorbieren.

Mit Acetylengruppen substituierte konjugierte 1,10-Phenanthroline stellen neuartige effiziente und im sichtbaren Spektralbereich fluoreszierende Chromophore dar. Das Interesse an diesen Verbindungen beruhte bisher ausschließlich auf ihrer Anwendung als modulierbare Fluorophore zum Aufbau von fluoreszierenden Sensoren und Schaltern auf dem Gebiet der molekularen Elektronik. Die Absorptions- und Emissionswellenlängen dieser Chromophore werden von der Art der Substituenten bestimmt.

1,10-Phenanthrolinderivate sind aufgrund ihres konjugierten $\pi$-Systems stark absorbierende Chromophore und deshalb für die spezielle Anwendung der Erfindung prinzipiell geeignet, wobei sich je nach Substitution am Phenanthrolingerüst und Funktionalität des entsprechenden Substituenten die Absorptionseigenschaften im Bezug auf die Lage des Absorptionsmaximums und dessen Intensität beeinflussen lassen. Um das gewünschte Chromophor in den Chelatliganden einführen zu können, ist eine primäre Aminofunktion am Substituenten erforderlich. Neben einer symmetrischen Substitution des 1,10-Phenanthrolins in der 3,8-Position erreicht man vor allem mit einer unsymmetrischen Substitution eine weitere Variationsmöglichkeit der spektroskopischen Eigenschaften des Chromophors.

Erfindungsgemäß wurde 2-Chlor-1,10-phenanthrolin dargestellt. Die Synthese des Antennen-Chromophors **P*B** erfolgte durch eine Kreuzkupplung von 2-Chlor-1,10-phenanthrolin **P*** mit Ethinylanilin **(B).**

**P*B** ist eine intensiv bis in den sichtbaren Spektralbereich hinein absorbierende Verbindung. Das Absorptionsspektrum von **P*B** ist in Abbildung 1 dargestellt und die gemessenen Absorptionswerte in Tabelle 1 zusammengefasst. Ein Vergleich des erstmalig dargestellten Antennen-Chromophors **P*B** mit 1,10-Phenanthrolin (**phen**) zeigt, dass die Substitution des Phenanthrolingerüstes mit dem Alkin **B** zu einer bathochromen Verschiebung und Intensivierung der längstwelligen Absorption (n $\rightarrow$ $\pi^*$-Übergang) führt.

Tab. 1: Absorptionseigenschaften der Antennen-Chromophore **$PA_2$, $PB_2$** und **P*B** im Vergleich zu 1,10-Phenanthrolin phen in MeOH.

| Antennen-Chromophor | Absorption: $\lambda_{abs}$ in nm ($\varepsilon$ in $M^{-1}$ $cm^{-1}$) |
|---|---|
| **Phen** | 230 ($5.1 \cdot 10^4$) / 264 ($3.0 \cdot 10^4$) / 280 ($1.2 \cdot 10^4$) |
| **$PA_2$** | 216 ($2.2 \cdot 10^4$) / 283 ($2.4 \cdot 10^4$) / 348 ($2.2 \cdot 10^4$) |
| **$PB_2$** | 280 ($4.2 \cdot 10^4$) / 395 ($4.2 \cdot 10^4$) |
| **P*B** | 233 ($3.8 \cdot 10^4$) / 275 ($4.7 \cdot 10^4$) / 370 ($3.4 \cdot 10^4$) |

Die Kupplung mit **B** zu **P*B** verstärkt die Rotverschiebung der Absorption und intensiviert diese gleichzeitig. Zudem verändern sich die Intensitätsverhältnisse der Absorptionsbanden zueinander.

Neben einer starken Absorption zeigt die Antenne **P*B** zugleich eine sehr intensive Emission im sichtbaren Spektralbereich. Die Emissionseigenschaften **P*B** sind aber für die zukünftige Anwendung der Antennen nicht vordergründig von Relevanz. Die Einführung der Antenne in den Chelatliganden erfolgte durch Umsetzung des Dianhydrids **LH-A** der Diethylentriaminpentaessigsäure mit dem 1,10-Phenanthrolinderivat **P*B** unter Ausbildung einer Amidbindung. Die relativ geringe Reaktivität von Carbonsäuren gegenüber primären Aminen erfordert eine Aktivierung der Carbonylkomponente. Im allgemeinen wird deshalb die Aminolyse der entsprechenden Säurechloride, -anhydride oder Ester der Carbonsäure durchgeführt. Im Fall des verwendeten Chelatliganden wurde eine Polyaminopolycarbonsäure erst in ihr Dianhydrid **LH-A** überführt. Das Dianhydrid **LH-A** ist gegenüber der freien Säure nicht nur reaktiver, sondern erhöht gleichzeitig auch die Produktselektivität, da nur zwei der fünf Carboxylgruppen zu einer Reaktion mit dem aromatischen Amin befähigt sind. Eine Reaktion des entsprechenden Säurechlorides oder des Esters wäre dagegen unselektiv.

Ein weiterer Vorteil dieser Synthesestrategie in Bezug auf die Fragestellung der Erfindung besteht darin, dass mit der Wahl des Dianhydrids nicht nur die Antenne sondern auch eine weitere aminofunktionalisierte Gruppe an den Chelatliganden gekoppelt werden kann.

**Synthese von LH-A: Diethylentriaminpentaessigsäure-dianhydrid**

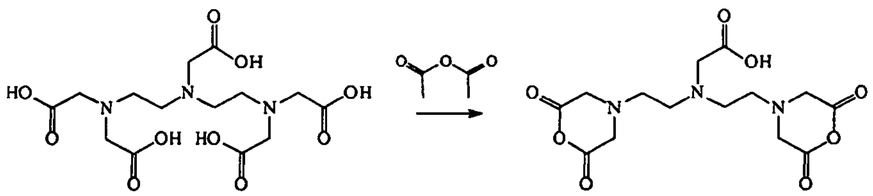

$LH_5A$

Die Reaktion ist unter Schutzgas durchzuführen.

Zu 12.9 ml Acetanhydrid und 12.3 ml Pyridin werden 13.4 g Diethylentriaminpentaessigsäure gegeben. Die Suspension wird bei 70 °C für 24 h gerührt. Das Reaktionsgemisch verfärbt sich dabei nach dunkelbraun. Nach Abkühlen wird filtriert und der Produktniederschlag mit Acetanhydrid, anschließend mehrmals mit Hexan gewaschen und im Vakuum getrocknet.
Ausbeute ca. 90 %

**Synthese von Antennenligand P*BLH$_4$**

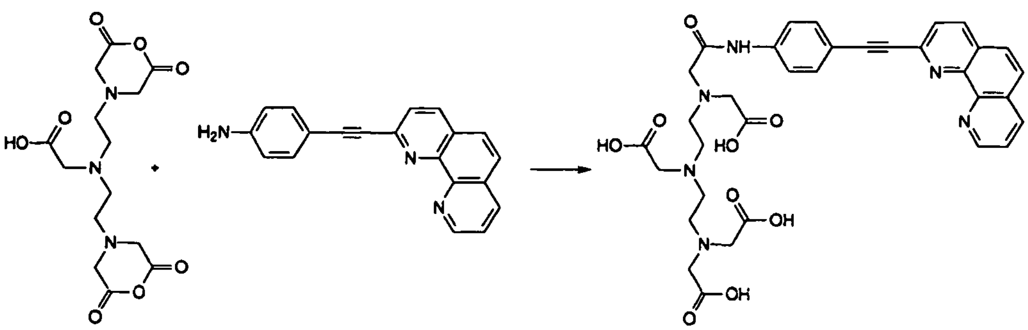

P*BLH$_4$

Die Reaktionen mit dem Dianhydrid **LH-A** und der Antenne **P*B** wurden im Verhältnis 1 : 1 durchgeführt, mit dem Ziel nur jeweils ein Antennen-Chromophor in den Chelatliganden einzuführen, unter Bildung von **P*BLH$_4$**. Die Aufarbeitung erfolgte in wässriger Lösung, wobei die zweite Anhydridfunktion geöffnet wird.

Die Einführung der Antenne **P*B** in den Chelatliganden **LH$_5$** führt selbst zu keiner wesentlichen Änderung ihrer der spektroskopischen Eigenschaften, während der ursprüngliche Chelatligand, die Diethylentriaminpentaessigsäure, nun alle angestrebten spektroskopischen Eigenschaften eines Antennenliganden aufweist. Mit Abbildung 2 wird das erhaltene Ergebnis am Beispiel der an einem Fluoreszenzspektrometer der Fa. PERKIN-ELMER (LS50B) aufgenommenen Anregungsspektren verdeutlicht.

Die Absorptionsmaxima von **P*B** werden durch die Verknüpfung mit Diethylentriaminpentaessigsäure geringfügig hypsochrom verschoben, wobei sich die Intensitätsverhältnisse der Absorptionsbanden und zum Teil ihre Bandenstruktur ändern. In jedem Fall aber führt die Einführung der Antenne **P*B** in die Diethylentriaminpentaessigsäure zu einer Intensivierung der längstwelligen Absorption im Vergleich zur freien Antenne, während die spektrale Lage der Emission nicht beeinflusst wird.

In Tabelle 2 sind die spektroskopischen Eigenschaften des Antennenliganden **P*BLH$_4$** im Vergleich zu **P*B** gegenübergestellt. Von besonderem Vorteil ist, dass die Anregung des Liganden nicht im Absorptionsmaximum der Antennengruppe erfolgen muss, da die Lage der Emissionsbanden unabhängig von der Anregungswellenlänge ist. Das ermöglicht zugleich eine beliebig langwellige Anregung der Lanthanoid(III)-Komplexe im Bereich seiner Absorption, wobei der Absorptionsbereich durch die Verwendung anderer Chromophore zusätzlich ausgedehnt werden kann.

Tab. 2: Absorptions- und Emissionsmaxima des Antennenliganden **P*BLH$_4$** und des korrespondierenden Antennen-Chromophors **P*B.**

| **Antennen-Chromophor** | | | | **Antennenligand** | | | |
|---|---|---|---|---|---|---|---|
| | **$\lambda_{abs}$ (nm)** | **$\varepsilon$ ($M^{-1}$ $cm^{-1}$)** | **$\lambda_{em}$ (nm)** | | **$\lambda_{abs}$ (nm)** | **$\varepsilon$ ($M^{-1}$ $cm^{-1}$)** | **$\lambda_{em}$ (nm)** |
| **P*B** | 370 | $3.4 \cdot 10^4$ | 433 | **P*BLH$_4$** | 311* | $3.5 \cdot 10^4$ | 433 |
| * Die Anregung von **P*BLH$_4$** erfolgt bei $\lambda_{exc}$ = 360 nm. | | | | | | | |

Für den Ausbau der erfindungsgemäßen Lösung wird der Antennenligand P*BLH$_4$ weiter verwendet.

Die stöchiometrische Umsetzung der Metallchloride $EuCl_3$ und $TbCl_3$ mit dem Antennenliganden **P*BLH$_4$** als freie Säure in einer schwach alkalischen wässrigen Lösung führt zu den Antennenkomplexen **[EuP*BL]$^-$** und **[TbP*BL]$^-$.** Die Komplexe können nach dem Einengen der Reaktionslösung durch Zugabe eines geeigneten Gegenions (z.B. Tetrabutylammonium-Ion $^nBu_4N^+$) gefällt werden. Für FRET-Untersuchungen wurden jedoch nur die Metallsalzlösungen jeweils zur Antennenligand-Lösung titriert und die Komplexbildung mittels UVNis- und Fluoreszenzspektroskopie kontrolliert.
Der Antennenligand mit Haftgruppe kann prinzipiell auch in einer "Eintopfsynthese" ohne Isolierung des Monoanhydrids oder des [P*BLH$_4$] dargestellt werden.

**Synthese von [EuP*BL]$^-$ und [TbP*BL]$^-$**

Die Darstellung der verwendeten Antennenkomplexe erfolgt durch Titration der Metallsalzlösungen zur Antennenligand-Lösung:
$EuCl_3$ bzw. $TbCl_3$ werden im Verhältnis 1 : 1 zu **BLH$_4$** (Konzentration > 0.1 mM) in einer wässrigen Lösung bei pH = 6 - 7 oder alkalischen Lösung ($NH_3/H_2O$) von pH = 10 gegeben.

**Spektroskopische Charakterisierung der Antennenkomplexe [EuP*BL]$^-$ und [TbP*BL]$^-$**

Aufgenommen wurden die Absorptions- und Emissionsspektren der Antennenkomplexe **[EuP*BL]$^-$** und **[TbP*BL]$^-$** in wässriger Lösung. Die erhaltenen Absorptionsspektren der beiden Donoren sind in Abbildung 3 dargestellt.

Die Lichtanregung der Antennenkomplexe **[EuP*BL]$^-$** und **[TbP*BL]$^-$** kann nun beliebig im Absorptionsbereich des Antennen-Chromophors **P*B** erfolgen. Von Interesse ist dabei eine möglichst langwellige Anregung, d. h. entweder im Absorptionsmaximum des $n \rightarrow \pi^*$-Überganges von **P*B** oder im langwelligen Auslauf dieser Absorption (wie bereits erwähnt bei $\lambda_{exc}$ = 360 nm). Für den Antennenkomplex **[EuP*BL]$^-$** resultiert aus der Anregung in die längstwellige Absorption von **P*B** die, für das Eu(III)-Ion charakteristische rote Emission, wie Abbildung 4 zeigt.

Die scharfen Emissionslinien im Emissionsspektrum von **[EuP*BL]$^-$** entsprechen Übergängen innerhalb der 4f-Niveaus. Die intensivsten Übergänge werden bei 595 nm und 617 nm beobachtet. Die Emission des Terbium(III)-Ions liegt dagegen im grünen Spektralbereich, mit den beiden intensivsten Emissionslinien bei 492 nm und 547 nm. Das in Abbildung 5 dargestellte Emissionsspektrum des Antennenkomplexes **[TbP*BL]$^-$** ist in Analogie zu **[EuP*BL]$^-$** auf eine Anregung des Antennenliganden in die längstwellige Absorption von P*B bei $\lambda_{exc}$ = 360 nm zurückzuführen.

Die beiden Lanthanoid(III)-Ionen zeigen in den Antennenkomplexen **[EuP*BL]$^-$** und **[TbP*BL]$^-$** eine sehr intensive Emission. Diese intensive Emission ist auf die starke Absorption des verwendeten Antennen-Chromophors **P*B** zurückzuführen. Verglichen mit den Emissionsspektren der jeweiligen Lanthanoid(III)-Komplexe der unsubstituierten Diethylentriaminpentaessigsäure führt die Komplexierung mit dem Antennenliganden **P*BLH$_4$** zu einer Intensivierung der Metallionenemission um das 5fache für **[TbP*BL]$^-$** bzw. das 30fache für **[EuP*BL]$^-$,** wobei die relativ lange Lebensdauer der angeregten Zustände erhalten bleibt.(Tabelle 3).

Tab. 3: Lebenszeiten $\tau$ der Lanthanoid(III)-Komplexe in $H_2O$ und die relativen Emissionsintensitäten der Chelate untereinander in $H_2O$.

| **Lanthanoid(III)-Komplex** | **$\tau H_2O$ (ms)** | **relative Intensitäten** |
|---|---|---|
| **EuLH$_5$** | 0.62 | 0.03 |
| **[EuP*BL]$^-$** | 0.59 | 1 |
| **TbLH$_5$** | 1.90 | 0.2 |

(fortgesetzt)

| Lanthanoid(III)-Komplex | $\tau H_2O$ (ms) | relative Intensitäten |
|---|---|---|
| **[TbP*BL]$^-$** | 1.61 | 1 |

Ein Vorteil der dargestellten Antennenkomplexe **[EuP*BL]$^-$** und **[TbP*BL]$^-$** liegt in den vergleichsweise langen Lebenszeiten ihrer elektronisch angeregten Zustände. Wie Tabelle 3 zeigt, beobachtet man für **[EuP*BL]$^-$** eine Lebenszeit der Emission von 0.62 ms und für **[EuP$^*$BL]$^-$** von 1.61 ms, Lebenszeiten organischer. Fluorophore liegen dagegen im Nanosekundenbereich.

Mit der Verwendung von Lanthanoid(III)-Verbindungen als Donoren ermöglicht dies nun die Aufnahme von Fluoreszenzspektren in einem zeitverzögerten Messregime, d. h. die Aufnahme der Spektren erfolgt nicht direkt nach der Anregung des Donors, sondern erst nach einer definierten Verzögerung. Alle anderen auftretenden und störenden Untergrundfluoreszenzen von Molekülen mit geringeren Lebenszeiten gegenüber der des Donors (in unserem Fall die Antennenkomplexe **[EuP*BL]$^-$** und **[TbP*BL]$^-$)** können somit eliminiert werden und tragen zu keiner Intensitätsverfälschung des eigentlichen Messsignals bei.

Alle Fluoreszenzmessungen der Donoren **[EuP*BL]$^-$** und **[TbP*BL]$^-$** wurden mit einer eingestellten Zeitverzögerung verfolgt. Die Standardeinstellungen waren:

für **[EuP*BL]$^-$:** Anregungswellenlänge: $\lambda_{exc}$ = 360 nm
Anregungsspalt: 15 nm Emissionsspalt: 5 nm
Emissionsfilter: 515 nm
Zeitfenster (gt): 4.50 ms
Zeitverzögerung (dt): 0.07 ms

für **[TbP*BL]$^-$** : Anregungswellenlänge: $\lambda_{exc}$ = 400 nm
Anregungsspalt: 15 nm Emissionsspalt: 10 nm
Emissionsfilter: 430 nm
Zeitfenster (gt): 4.60 ms
Zeitverzögerung (dt): 0.08 ms

Für die Messung der Fluoreszenzabklingkurven der Lanthanoid(III)-Komplexe wurden die gleichen Standardeinstellungen am Fluoreszenzspektrometer LS50B gewählt, wie bei FRET-Untersuchungen. Die Emission wurde bei einer Wellenlänge von $\lambda_{em}$ = 617.5 nm für **[EuP*BL]$^-$** und $\lambda_{em}$ = 547 nm für **[TbP*BL]$^-$** beobachtet.

Im folgenden wird die Eignung der Donorkomplexe für die erfindungsgemäße Lösung der Aufgabenstellung nachgewiesen. Dazu wird wie nachstehend beschrieben verfahren.

Untersucht wurden in den FRET-Experimenten die Änderung der Lebenszeit und Emissionsintensität des elektronisch angeregten Antennenkomplexes **[EuP*BL]$^-$** oder **[TbP*BL]$^-$** in Gegenwart verschiedener Akzeptorkonzentrationen von **ST936** bzw. **Rho**damin **B**. Die Effizienz der Energieübertragung gibt das Ausmaß der zu beobachtenden Fluoreszenzlöschung des Donors bei Anwesenheit des Akzeptors an.

Die Donor-Akzeptor-Paare werden durch Vermischen entsprechender Donor- und Akzeptor-Lösungen erhalten. FRET-Untersuchungen des Europium(III)-Komplexes **[EuP*BL]$^-$** wurden erfindungsgemäß mit dem Polymethinfarbstoff **ST936** der SENSIENT GmbH Wolfen als korrespondierendem Akzeptor durchgeführt. Als Akzeptorfarbstoff für den Donorkomplex **[TbP*BL]$^-$** wurde erfindungsgemäß der Farbstoff **Rhodamin B** verwendet. In Modellsystemen entscheidet sich die Wahl eines Donors und eines Akzeptors aufgrund ihrer spektroskopischen Eigenschaften. Entsprechende Funktionalisierungen an Donor oder Akzeptor für eine kovalente Bindungsknüpfung zu einem biologischen Substrat sind dabei nicht von Belang.

Unter den spektroskopischen Voraussetzungen eines Akzeptors für einen gewählten Donor in einem FRET-Experiment versteht man die Überlappung der Absorption des Akzeptors mit der Emission des Donors. Abbildung 6 zeigt die spektroskopischen Eigenschaften des Antennenkomplexes **[EuP*BL]** in Relation zu denen des gewählten Polymethinfarbstoffes **ST936** der SENSIENT GmbH Wolfen als korrespondierendem Akzeptor. Es ist zu erkennen, dass das Emissionsspektrum von **[EuP*BL]$^-$** und das Absorptionsspektrum des Akzeptors stark überlappen und somit den Anforderung an die Aufgabenstellung der Erfindung genügen. Des weiteren liegt das Emissionsmaximum von **ST936** bei $\lambda_{em}$ = 660 bathochrom verschoben zu den intensivsten Emissionslinien des Antennenkomplexes.

Bei einer Anregung von **[EuP*BL]$^-$** in Gegenwart des Polymethinfarbstoffes **ST936** führt dies zu einer Löschung der Emissionsintensität des Antennenkomplexes und zum Aufbau der Akzeptor-Emission bei 666 nm. Eine strahlungslose Energieübertragung vom verwendeten Antennenkomplex **[EuP*BL]$^-$** auf den Fluoreszenzfarbstoff **ST936** ist damit nachgewiesen. Beobachtet wurde im FRET-Experiment bei einer konstanten Konzentration des Donors eine Verringerung der Emissionsintensität von **[EuP*BL]⁻** und einen Aufbau der Emission von **ST936** mit steigender Akzeptorkonzentration. Mit einer erforderlichen Korrektur der Emissionsspektren können Donor- und sensibilisierte Akzeptor-Emission für jedes Donor-Akzeptor-Verhältnis ermittelt werden.

Befinden sich der Antennenkomplex **[TbP*BL]⁻** und der für diesen Donor gewählte Akzeptorfarbstoff Rhodamin B in einer Lösung, so resultiert auch für dieses Donor-Akzeptor-Paar nach einer Anregung von **[TbP*BL]⁻** im Bereich der längstwelligen Absorption des Antennen-Chromophors **P*B** eine Löschung der Emissionsintensität von **[TbP*BL]⁻** infolge der strahlungslosen Energieübertragung auf den Akzeptor und eine neue Emission von **Rhodamin B**. Damit ist seine Eignung nachgewiesen.

Nach der Herstellung und dem Eignungstest für die Donoren werden Akzeptorfarbstoffe synthetisiert.

Für den Donorkomplex **[EuP*BL]⁻** wurde mit dem Polymethinfarbstoff **ST936** der AcMaRi Chemie GmbH ein aufgrund seiner spektroskopischen Eigenschaften geeigneter Akzeptor gefunden, um in entsprechenden FRET-Untersuchungen den Europium(III)-Komplex auf seine Energie-Donoreigenschaften zu testen. Gekennzeichnet ist dieser Polymethinfarbstoff von einer starken Absorption im Emissionsbereich des Donors und einer intensiven Emission, bathochrom verschoben zur Emission von **[EuP*BLH$_4$]⁻.**

Für eine Anwendung von Nachteil ist aber die schlechte Löslichkeit des Polymethinfarbstoffs in wässriger Lösung und die fehlende Funktionalität für eine kovalente Bindung des zu markierenden Proteinanalyten. Notwendig wird deshalb die Substitution des Polymethinfarbstoffs **ST936** durch solche Gruppen, die eine Bindungsmöglichkeit z.B. an Proteine erlauben und gleichzeitig auch die Löslichkeit in Wasser verbessern.

Im Rahmen der Erfindung bestand das Ziel in der Derivatisierung des Indoleninrestes zum Aufbau neuer zur Markierung geeigneter Polymethinfarbstoffe unter Beibehaltung des Cy5-Grundkörpers von **ST936** und damit seiner Absorptions- und Emissionseigenschaften bezüglich der Lage der Maxima.

Ausgehend von den Indoleninderivaten **TIPBr⁻**, **TIEOBr⁻, TIPEBr⁻, TIPNBr⁻** und **TIBEI⁻** sind neben dem bereits bekannten Polymethinfarbstoff **ST936** weitere Farbstoffe dargestellt worden: **Cy5BE**, **Cy5'BE** und **Cy5PE**. Aus der Umsetzung des Indoleninderivates **TIEOBr⁻** mit dem Dianilid wurde **Cy5EE** als entsprechender Polymethinfarbstoff erhalten. Die Hydroxyfunktion ist dabei durch das in der Reaktionslösung befindliche Acetanhydrid verestert worden.

Es wurde erkannt, dass der Antennenligand eine Funktionalität besitzen muss, die eine kovalente Bindung des zu bestimmenden Analyten (die Immunoassay-Komponente) an den späteren Donor erlaubt. Die Bindung des Analyten über diesen funktionellen Rest reduziert dabei am Antennenliganden die Anzahl der freien Carboxylatgruppen, die das Lanthanoid(III)ion komplexieren und verändert somit die Gesamtladung des sich bildenden Antennenkomplexes. Für FIA-Systeme auf FRET-Basis sind nur negativ geladene oder Neutralkomplexe von Vorteil, da sie keine unspezifischen Bindungen mit dem biologischen, meist ebenfalls negativ geladenen Substrat, eingehen können. Zunächst wurden zur spektroskopischen Charakterisierung die Lanthanoid(III)-Antennenkomplexe **[LnPBL]⁻** hergestellt.

Weiterhin wird der Antennenligand **P*BLH$_4$** durch das Einführen von Linkergruppen erfindungsgemäß funktionalisiert. Das ist möglich, weil erkannt wurde, dass das Dianhydrid der Diethylentriaminpentaessigsäure **LH-A** mit dem in 2-Position substituierten 1,10-Phenanthrolinderivat **P*B** nur einseitig reagiert, so dass im Weiteren die zweite Anhydridfunktion ebenfalls mit einer aminofunktionalisierten Gruppe umgesetzt werden kann.

Auf diese Weise wurden zwei verschiedene Linkergruppen, Diamine unterschiedlicher Kettenlänge, an den Antennenliganden **P*BLH$_4$** gebunden. Synthetisiert wurden **P*BLH$_3$-EDA** (EDA: Ethylendiamin) und **P*BLH$_3$-SP1** (SP1: 1,8-Diamino-3,6-dioxaoctan).

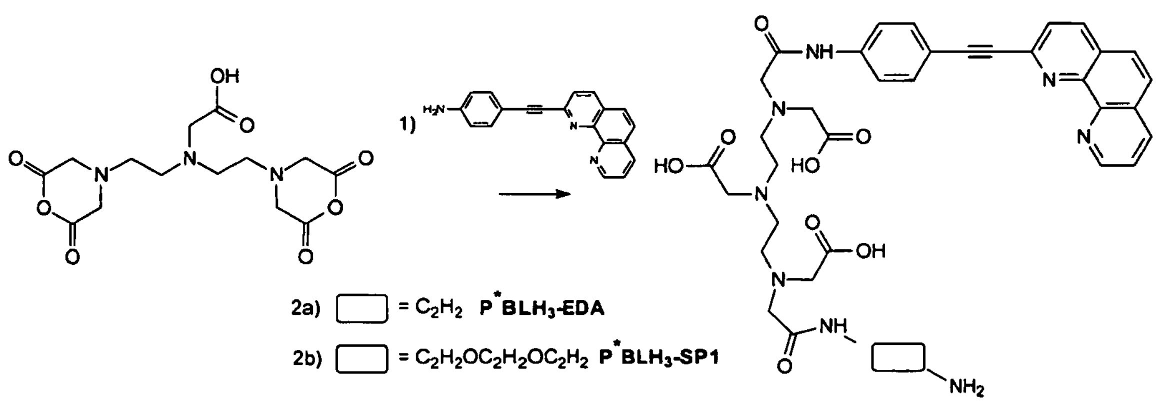


Die Reaktion ist unter Schutzgas durchzuführen.
1.5 mmol **LH-A** werden in 24 ml DMF und 2.1 ml $Et_3N$ gelöst und mit 1.5 mmol 2-(4-Aminophenyfethinyl)-1,10-phenanthrolin **(P*B),** gelöst in 5 ml DMF unter Rühren tropfenweise versetzt. Die Reaktionslösung wird nach ca. 2 h Rühren

bei Raumtemperatur zu 2 mmol Diamin in 3 ml DMF getropft, wobei ein gelber flockiger Niederschlag ausfällt. Es wird für weitere 1 - 2 h gerührt, etwas im Vakuum eingeengt und über Nacht in den Kühlschrank gestellt, wobei das Produkt vollständig ausfallen sollte. Der Produktniederschlag wird über eine Fritte von der Reaktionslösung abgetrennt, mit etwas trockenem Methanol und Ether gewaschen und anschließend im Vakuum getrocknet. Das Produkt wird als gelber Feststoff erhalten. Isoliert man das Produkt an Luft, wird der gelbe Niederschlag zähflüssig und färbt sich orange-rot. Das Produkt kann an Luft getrocknet und mit dem Mörser verrieben werden.
Ausbeute ca. 60 %

Ebenfalls funktionalisiert wurden die Polymethinfarbstoffe, denn eine Zielstellung der Erfindung bestand zum einen darin, einen Polymethinfarbstoff mit einer freien Carboxylgruppe darzustellen, durch welche der Farbstoff direkt an ein Protein gebunden werden kann, wobei der Carbonsäurerest auch eine Derivatisierung mit Gruppen anderer Reaktivität (z.B. Diamine) zum Protein zulässt. Auf der anderen Seite sollte ein Polymethinfarbstoff synthetisiert werden, dessen chemische Kopplung an einen Proteinanalyten nicht über die Bildung einer Amidbindung erfolgt.

Aus diesem Grund wurden für den ersten Aspekt die beiden symmetrischen Farbstoffe **Cy5BE** und **Cy5PE** sowie der unsymmetrische **Cy5'BE** dargestellt. Die gewünschte Carbonsäurefunktion wurde anschließend direkt aus der Hydrolyse der Esterfarbstoffe erhalten und der Farbstoff konnte als Perchlorat isoliert werden.

Mit dem Ziel der Modellierung eines Bioassays werden FRET-Untersuchungen mit definiertem Donor-Akzeptor-Abstand durchgeführt.

Der Spacer bringt im Modellsystem Donor und Akzeptor in einen räumlichen Abstand und simuliert die bindenden Wechselwirkungen von Biomolekülen (z.B. Antigen und Antikörper), Linker Linker.

Dabei darf durch die Kettenlänge des Linkers der Donor-Akzeptor-Abstand nicht so groß werden, d. h. er muß sich innerhalb des sog. FÖRSTER-Abstandes bewegen, dass kein FRET mehr beobachtet werden kann.

In diesem Modellsystem sind Donor und Akzeptor fest über die Isothiocyanatfunktion des modifizierten Farbstoffes unter Variation der Kettenlänge des Linkers in Form einer Amid-(Donor-Linker-Bindung) bzw. einer Thiocarbamatbindung verknüpft. Das angestrebte Modellsystem mit modifizierten Donor und Akzeptor ist in Abbildung 11 wiedergegeben. Als Donor wurde der Antennenkomplex **[EuP*BL]$^-$** unter Substitution des Antennenliganden mit den Diaminen Ethylendiamin **(EDA)** bzw. 1,8-Diamino-3,6-dioxaoctan **(SP1)** verwendet. Korrespondierender Akzeptor war im FRET-Experiment der Polymethinfarbstoff **Cy5ENCS.**

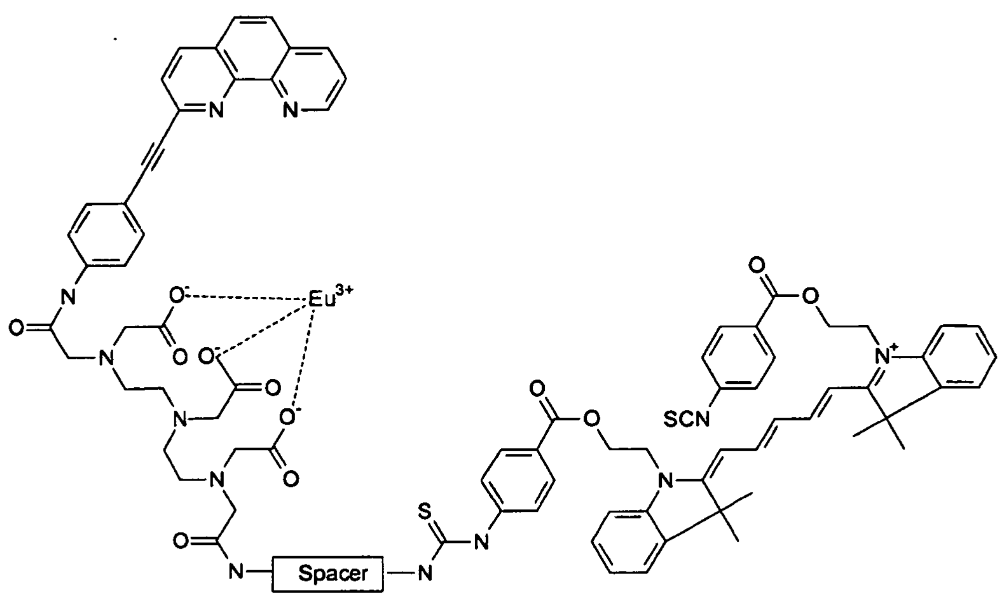

**Donor (Antennenkomplex [EuP*BL]$^-$) und Akzeptor (Polymethinfarbstoff Cy5ENCS) sind über einen Linker miteinander verbunden. Als Linker wurden Ethylendiamin (EDA) und 1,8-Diamino-3,6-dioxaoctan (SP1) verwendet.**

Die Darstellung des Donors erfolgte durch Titration einer wässrigen $EuCl_3$-Lösung ($\geq 1\cdot10^{-3}$ M) zu einer äquimolaren wässrigen Lösung von Antennenligand **P*BLH$_3$-EDA** bzw. **P*BLH$_3$-SP1.** Die Bildung des Antennenkomplexes

**[EuP*BL-EDA]** bzw. **[EuP*BL-SP1]** wurde fluoreszenzspektroskopisch verfolgt. Nach Anregung der wässrigen Lösung bei $\lambda_{exc}$ = 360 nm wurde mit Beobachtung der intensiven roten Emission des Europium(III)-Ions die Komplexbildung von **[EuP*BL-EDA]** bzw. **[EuP*BL-SP1]** bestätigt.

In einem FRET-Experiment wurde zur Lösung des mit terminaler Aminofunktion substituierten Antennenkomplexes **([EuP*BL-EDA]** oder **[EuP*BL-SP1])** konstanter Konzentration der Akzeptorfarbstoff **Cy5ENCS** schrittweise bis zu einem Überschuss an Polymethinfarbstoff zugegeben. Dabei wird die Emission des Donors **[EuP*BL-EDA]** (gleiches gilt für **[EuP*BL-SP1])** mit steigender Akzeptorkonzentration gelöscht und ein Aufbau der Emission des Akzeptors beobachtet. In Abbildung 7 sind die aus dem FRET-Experiment erhaltenen Emissionsspektren, nach erfolgter Korrektur, für das Donor-Akzeptor-Paar **{[EuP*BL]-EDA-Cy5ENCS}** graphisch dargestellt. Die Korrektur entspricht einer Normierung aller Emissionsspektren auf das Maximum des energiereichsten Überganges des Antennenkomplexes bei Abwesenheit des Akzeptors.

Unter gleichen Messbedingungen wurden die FRET-Untersuchungen des modifizierten Antennenkomplexes in Gegenwart des Linkers SP1 mit {[EuP*$BLH_4$]-SP1-Cy5ENCS} durchgeführt. Wie Abbildung 13 zeigt, wird auch hier eine Löschung der Donor-Emission bei gleichzeitiger Sensibilisierung der Emission des Polymethinfarbstoffes beobachtet.

Damit wird durch diese Modelluntersuchungen die prinzipielle Anwendbarkeit der neuen FRET-Systeme für entsprechende Immunoassays nachgewiesen.

**FRET-Bioassay mit modifiziertem Donor-Akzeptor-Paar**

Zum Nachweis und/oder der Quantifizierung von biologisch aktiven Molekülen mit einem FRET-Bioassay sollte das Messsystem, dass eines der beschriebenen Donor/Akzeptor-Paare verwendet, möglichst universell einsetzbar sein. Deshalb wurde untersucht ob sich die Bindung von Protein A an Immunglobulin G (IgG) nach Markierung der beiden Partner mit dem Antennenliganden **P*$BLH_3$-SP1** als Donor und dem Farbstoff **Cy5BA** als Akzeptor durch eine FRET-Messung nachweisen lässt.

Das verwendete System Protein A - IgG hat den Vorteil, dass die Bindung des Protein A an das IgG am Fc-Fragment erfolgt, wodurch die Wechselwirkung des IgG mit dem entsprechenden Antigen, das an das Fab-Fragment bindet, nicht beeinflusst wird.

Für die Ligandierung der beiden verwendeten Proteine mit dem Donor bzw. dem Akzeptor wurde die beiden Reaktanten im schwach sauren Bereich mit einem Überschuss an N-Ethyl-N'-(3-dimethylaminopropyl)-carbodiimid (**EDC**) bei 20°C behandelt und die gebildeten Säureamide des Antennenliganden bzw. des Farbstoffs mit dem Protein durch Gelfiltration vom nicht umgesetzten Liganden und anderen niedermolekularen Bestandteilen des Reaktionsansatzes getrennt.

Kopplung von P*$BLH_3$-SP1 an IgG

0.066 $\mu$Mol IgG wurden mit 3.3 $\mu$Mol des Antennenligandens P*$BLH_3$-SP1 und 330 $\mu$Mol EDC in 100 mM Imidazol/HCl-Puffer pH 5.0 gemischt und bei Raumtemperatur über Nacht inkubiert. Nach 1 Stunde Inkubation wurde der pH-Wert mit 1 N NaOH wieder auf pH 4.5-5.0 gebracht. Am nächsten Morgen wurde ausgefallenes Material, das im wesentlichen denaturiertes Protein repräsentiert, durch Zentrifugation bei 5000 x g entfernt und das IgG-Antennenligand-Konjugat durch Gelfiltration über eine NAP-5-Säule (Amersham Biotech) von nicht umgesetztem Antennenligand und anderen niedermolekularen Substanzen abgetrennt.

Die Ausbeute betrug etwa 60 % bezogen auf das eingesetzte IgG. Ein IgG-Molekül enthielt etwa 30 gebundene Moleküle des Antennenligandens. Dieser Substitutionsgrad lässt sich durch Variation des molaren Protein-Antennenliganden-Verhältnisses, beliebig einstellen.

Die Synthese des Addukts aus Protein A und dem Farbstoff Cy5BA erfolgte in analoger Weise, jedoch wurde der Farbstoff wegen seiner schlechten Wasserlöslichkeit als alkoholische Lösung eingesetzt. Die Aufarbeitung erfolgte wie oben beschrieben durch Abtrennung von ausgefallenem Protein durch Zentrifugation und gelchromatographische Abtrennung von nicht umgesetzten Farbstoff.

Die Ausbeute betrug etwa 10 % (bezogen auf Protein A), der Substitutionsgrad lag bei ca. 8 Mol Farbstoff pro Mol Protein A. Die schlechte Ausbeute ist durch eine starke Denaturierung des Protein durch den als Lösungsmittel für den Farbstoff verwendeten Alkohol verursacht.

Die Befähigung zum FRET der gebundenen Donoren und Akzeptoren wurde anhand von Lebenszeituntersuchungen mit zwei verschieden Konzentrationen der markierten Komponenten Protein A **(PA)** und Immunoglobulin G **(IgG)** im Verhältnis PA:IgG von 1:1 getestet. Die aus den Lebenszeitmessungen ermittelte Energieübertragungseffizienz **E** muss hierbei unabhängig von der Konzentration der Komponenten sein. Aus dem erhaltenen markiertem Material wurden Lösungen in Imidazol/HCl-Puffer von $8{,}5 \cdot 10^{-7}$ M und $2{,}5 \cdot 10^{-7}$ M hergestellt. Der Donorkomplex wurde durch Zugabe der dem Markierungsanteil entsprechenden Menge $EuCl_3$-Lösung zum Liganden hergestellt.

Die Abbildungen 8 und 9 zeigen die mit den angegebenen Konzentrationen unter den entsprechenden Anregungsbedingungen erhaltenen Spektren. Es wurde jeweils erst die reine Donorlösung untersucht und $\tau_0$ ermittelt und

die Untersuchungen nach Zugabe des markierten Proteins A zur Bestimmung von τ wiederholt. Die Lebenszeit wurde vierfach gemessen und der Mittelwert verwendet. Die Übertragungseffizienz in % ergibt sich zu

$$E = 1 - \frac{\tau}{\tau_0}.$$

Die Messwerte sind in Tabelle 4 zusammengefasst. Die Übertragungseffizienz ist demnach im Rahmen der methodenbedingten Messfehler unabhängig und die Funktionstüchtigkeit des vorliegenden Systems bewiesen. Der relativ kleine Betrag der Effizienz hat seine Ursache in dem im Vergleich zum Akzeptor höheren Markierungsgrad des Donors (30:8). Hier wäre ein Verhältnis von 1:1 oder 1:2 (IgG: PA) günstiger.

Tab. 4

| Konzentration in M | | Gemessene Lebenszeiten | | | | Mittelwert τ | **E** in % |
|---|---|---|---|---|---|---|---|
| $8{,}2*10^{-7}$ | **IgG** | 0,4780 | 0,4784 | 0,4758 | 0,4774 | 0,4774 | 19 |
| | IgG+PA | 0,3865 | 0,3876 | 0,3870 | 0,3863 | 0,3869 | |
| $2{,}5*10^{-7}$ | **IgG** | 0,5177 | 0,5154 | 0,5170 | 0,5040 | 0,5135 | 17,3 |
| | IgG+PA | 0,4184 | 0,4367 | 0,4050 | 0,4395 | 0,4279 | |

Vorschlag für die Zusammenstellung eines Baukastensystems für einen FRET-Immuno-assay

Für die Benutzung des FRET-Immunoassays in der biologischen bzw. medizinischen Analysepraxis sollten Baukastensysteme entwickelt werden, die den Nutzern eine möglichst einfache Handhabung des Analysensystems und eine hohe Flexibilität bezüglich des verwendeten primären Antikörpers und damit der Wahl des untersuchenden Analyten ermöglichen.

In Abb. 7 ist das Messprinzip eines FRET-Immnuoassay schematisch dargestellt. In einer 1. Reaktion wird das Untersuchungsmaterial mit dem Analyten mit einem Antikörper (primärer Antikörper), der mit dem Donor gekoppelt ist, inkubiert. Im 2. Reaktionsschritt erfolgt der Nachweis des gebundenen primären Antikörpers mit einem zweiten mit dem Akzeptorfarbstoff markierten Antikörper oder einem anderen Protein (z. B. Protein A oder Protein G), das spezifisch Immunglobuline bindet. Die durch diese Protein-Protein-Interaktion ausgelöste FRET-Reaktion ist dann die Messgröße des Assays. Ein solches FRET-Immunoassay muß wie eine klassische ELISA-Reaktion an einer festen Phase erfolgen, da sonst die Gefahr besteht, das der sekundäre Antikörper mit nicht umgesetzten primären Antikörper eine FRET-Reaktion ergibt.

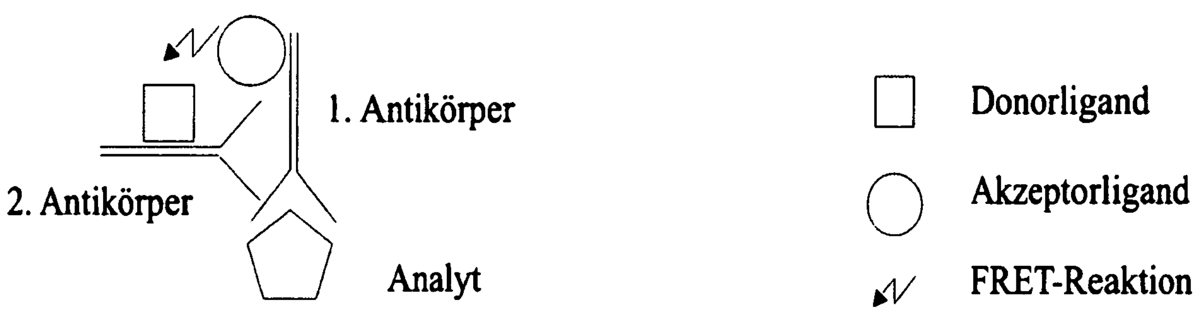


Schematische Darstellung eines FRET-Immunoassays

Ein Kit für einen FRET-Immunoassay besteht aus dem Antennenliganden und den Reagenzien (EDC, Puffer, Gelfiltrationssäule) für seine Kopplung an den primären, vom Nutzer zu stellenden Antikörper so wie dem mit dem Akzeptorfarbstoff markierten sekundären Antikörper (oder einem anderen markierten Protein, dass spezifisch Immunglobuline bindet). Werden dem Kit mehrere sekundäre Antikörper beigefügt, die mit Akzeptorfarbstoffen markiert sind, die sich so in ihren spektralen Eigenschaften unterscheiden, dass die Detektion der FRET-Reaktion bei unterschiedlichen

Wellenlängen durchgeführt werden kann, ist mit einem solchen Messsytem auch die gleichzeitige Detektion unterschiedlicher Analyten möglich (Multiplexing).

Im Fall des Donors hat sich ein System bestehend aus aminofunktionalisierter Antenne **P*B,** dem Säureanhydrid **LH-A** und dem Spacer Ethylendiamin **EDA** als effektiv erwiesen. Die drei Komponenten werden in DMF/TEA zum Antennenliganden verknüpft. Eine Variation des Spacers ist prinzipiell möglich. Die erhaltenen Produkte sind nach chromatographischer Reinigung anwendbar und werden dem Anwender als Feststoff zur Verfügung gestellt. Antikörper und Antennenligand werden mit einem nach den üblichen Verfahren unter Aktivierung der Carbonylkomponente mit N-Ethyl-N'-(3-Dimethylaminopropyl)-carbodiimid **(EDC)** oder N, N'-Carbonyldiimid-azol **(CDI)** über Ausbildung einer Amidbindung miteinander verknüpft. Zur Erweiterung des Anwendungsbereiches ist auch eine Umwandlung in eine Isothiocyanatofunktion am terminalen Aminstickstoffes mit dem Spacer versehenen Antennenliganden möglich.

Die eigentlichen Energie-Donoren, die Lanthanoid(III)-Komplexe, werden im Assay *in situ* durch Zugabe der entsprechenden Menge einer Lösung des Metallchlorids dargestellt. Die Wahl des Lanthanoid(III)ions bestimmt durch seine spezifische Emission die Wahl des Akzeptorfarbstoffs. Für das als Beispiel verwendete Europium(III)ion wurden die Polymethinfarbstoffe **Cy5R** ausgewählt, deren Absorption im Emissionsbereich des Donors (Europium(III)-Komplex) liegt. Hydroxy- und mit Säuregruppen funktionalisierte Cy5-Farbstoffe sind in hohen Ausbeuten zugänglich. Die Säurefunktion erlaubt eine direkte Knüpfung an den sekundären Antikörper über eine Amidbindung nach den bereits im Fall des Donors genannten Methoden. Mit dem Carboxylgruppen eines Proteins kann der hydroxyfunktionalisierte Farbstoff auch über eine Esterbindung verknüpft werden. Als Funktionsbeispiele werden Verbindungen aus der Klasse der Polymethinfarbstoffe angegeben sowie polymethinähnliche Farbstoffe, die erfindungsgemäß hergestellt und eingesetzt werden.

Im einfachsten Fall enthält das kommerzielle Baukastensystem erfindungsgemäß demnach mindestens folgende Komponente (Tabelle 5):

- den Antennenliganden mit dem Spacer **EDA** als Feststoff (Komponente 1)
- **CDI** oder **EDC** und Triethylaminacetatpuffer zur Markierung.
- Europium(III)chlorid als $10^{-3}$ mol/l Lösung in Wasser (Komponente 2).
- Den sekundären Antikörper markiert mit einem Cy5-Farbstoff (Komponente 3).

| **Komponente 1** | **Komponente 2** | **Komponente 3** | **Anregungswellenlänge** $\lambda_{exc}$ [nm] | **Detektionswellenlänge** $\lambda_{em}$ [nm] |
|---|---|---|---|---|
| **P*BLH-EDA** | $Eu^{3+}$ | **Cy5BA** | 360 | 665 |
| **P*BLH-EDA** | $Tb^{3+}$ | **Cy3BA** | 360 | 550 |

Der oben beschriebene FRET-Immunoassay wird bevorzugt an einer festen Oberfläche (z.B. Mikrotiterplatte, Objekträger bei histochemischen Anwendungen) durchgeführt, damit nicht an den Analyten gebundener primärer Antikörper, der bei der anschließenden FRET-Reaktion falsch positive Signale liefern würde, vor der FRET-Reaktion durch einen Waschschritt entfernt werden kann.

Für einige Anwendungen ist auch ein homogener FRET-Immunoassay denkbar. Ein Beispiel hierfür ist z. B. das Screening von Hybridomzellklonen auf Antikörperproduktion. Ein solches System enthält neben dem primären Antikörper, der die monoklonalen Antikörper erkennt (z.B. Anti-Maus-IgG) als zweite Komponente eine Protein wie z. B. den Komplementfaktor C1 q, so dass eine Unterscheidung von Antigen-Antikörper-Komplexen von freien Immunglobulinen möglich ist.

Das Hauptproblem homogener Anwendungen besteht in der Möglichkeit, dass trotz Vorliegen einer Protein-Protein-Wechselwirkung der Abstand zwischen Donor und Akzeptor so groß ist, dass kein FRET beobachtet werden kann und somit ein falsches Ergebnis erhalten wird. Diese Gefahr einer Fehlinterpretation kann umgangen werden, wenn das Experiment in Form eines üblichen kompetitiven Assays durchgeführt wird. Hierzu ist es erforderlich, dass von einem AK/AG-Paar mit einer sehr schwachen Wechselwirkung ausgegangen wird bei dem FRET nachweisbar ist. Nach Zugabe eines nicht markierten Antikörpers kann an der Abnahme des Emissionsintensität des Akzeptors in Abhängigkeit von der Konzentration dieses Antikörpers die Bildung eines wirksameren Komplexes erkannt werden.

In der Zusammenschau der Erfindung zeigt sich, dass das Kernstück der Erfindung ein Ethinylanilin entsprechend der Formel

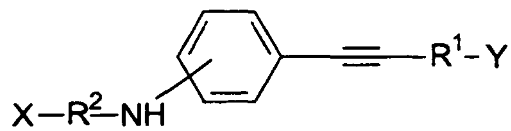

ist, in der

**R$^1$** eine Antennenfunktion ist, ausgesucht aus den folgenden: 1,10-Phenanthrolin (1) oder Fluoren (2) oder Acetophenon (3) oder Benzophenon (4) oder Fluorenon (5) oder Xanton (6) oder Azaxanton (7) oder Anthrachinon (8) oder Acridon (9) oder Chinolin (10) oder Cumarin (11)

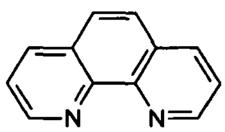
1

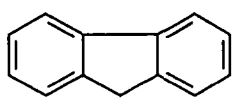
2

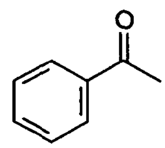
3

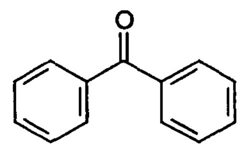
4

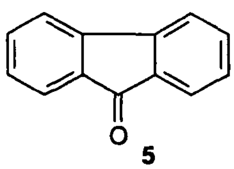
5

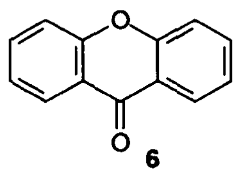
6

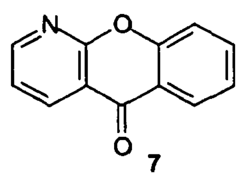
7

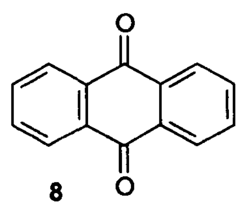
8

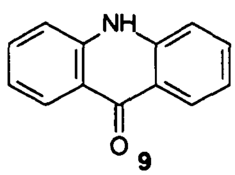
9

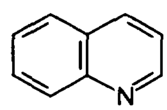
10

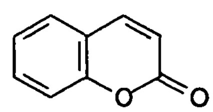
11

**R$^2$** ein Chelatbildner ist, der ein Lanthanoid(III)ion koordiniert enthält, wobei der Chelatbildner einer der folgenden ist: DTPA (n=1), TTHA (n=2)

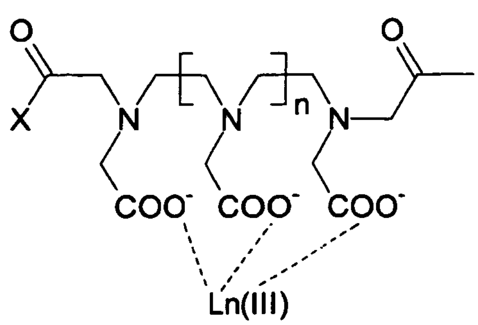

oder das TTHA-Isomer NTTHA

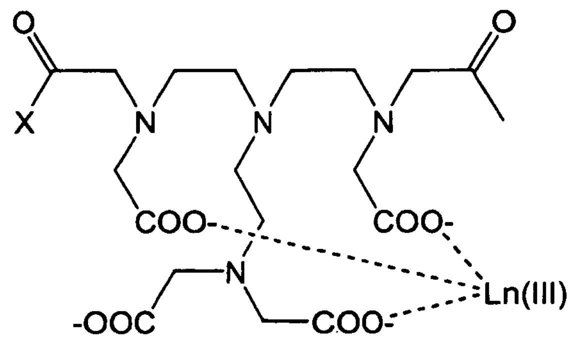

wobei die TTHA-Derivate ein zweites Ethinylanilin mit Antennenfunktion enthal ten können, so dass immer drei Carboxylatreste zur Komplexbildung zur Verfügung stehen,

**X** -OH oder eine Haftgruppe zum Biomolekül ist, die über eine Amidbindung an einen Carboxylatrest des Chelatbildners gebunden ist, wobei als Haftgruppen in Frage kommen: $-NH(CH_2)_2NH_2$, $-NH(CH_2)_2PhNH_2$, $-NH(CH_2)_2PhNCS$, $-NH(CH_2)_nNH(C_3N_3Cl_2)$ mit n = 2 bis 6 sowie $-NH(CH_2)_2O(CH_2)_2NH_2$ (SP1)

**Y** -H oder eine Haftgruppe zum Biomolekül, die an die Antennenfunktion gekoppelt ist, wobei die Haftgruppen aus den folgenden ausgesucht sind:

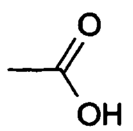

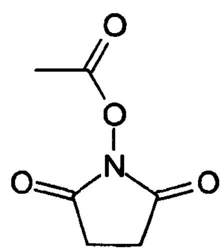

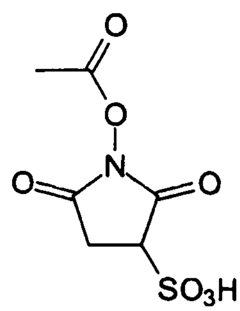

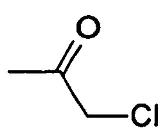

—NCS

$—NH_2$

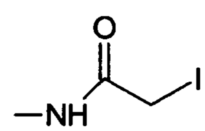

e) die Lanthanoid(III)ionen die folgenden sind: $Eu^{3+}$, $Tb^{3+}$, $Dy^{3+}$, $Sm^{3+}$.

Das erfindungswesentliche 2-(4'-Aminophenylethinyl)-1,10-phenanthrolin P*B

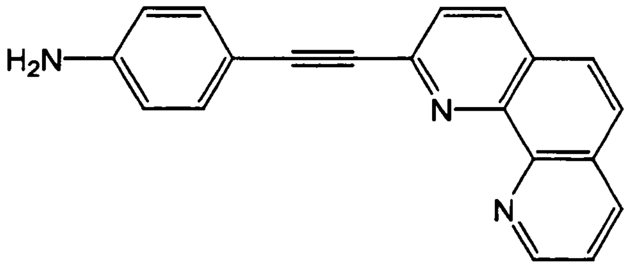

$C_{20}H_{13}N_3$

wird durch Umsetzen von 2-Chlor-1,10-phenanthrolin mit p-Ethinylanilin hergestellt.

P*B wird erfolgreich als Antennenchromophor

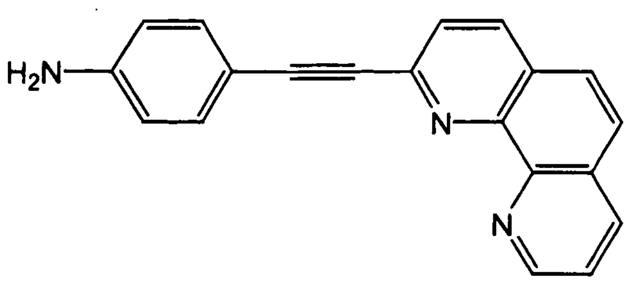

eingesetzt.

Chelatligand ist P*BLH$_4$

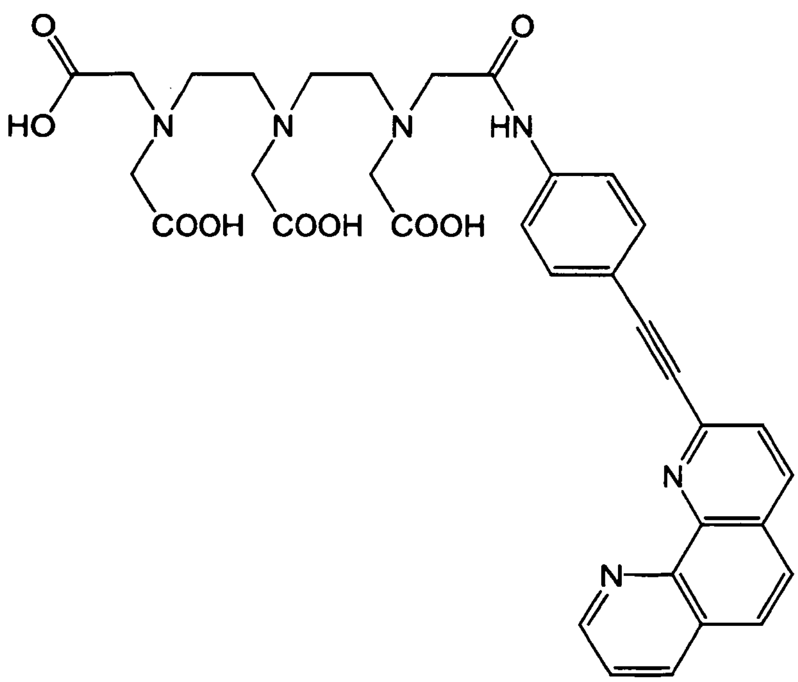

$C_{34}H_{34}N_6O_9$

(wobei LH$_4$ für Diethylentriaminpentaessigsäure steht und LH$_3$, LH$_2$, LH, L für weitere Dissoziationsstufen der Diethylentriaminpenta-essigsäure) der als Antennenligand dient:

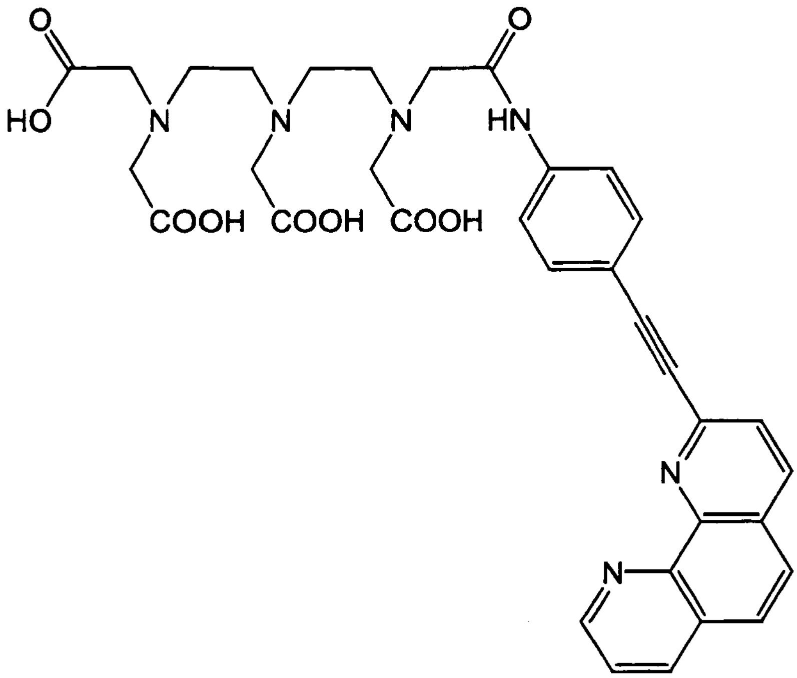

Der Komplex [EuP*BL)]$^-$ und die Verbindung $^{n-}Bu_4N$[EuP*BL]

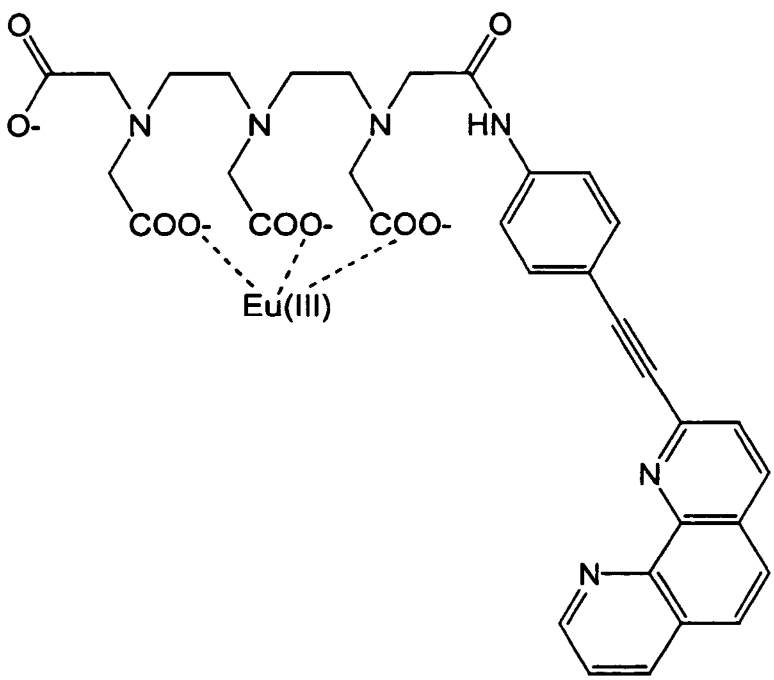

[EuP*BL)]$^-$

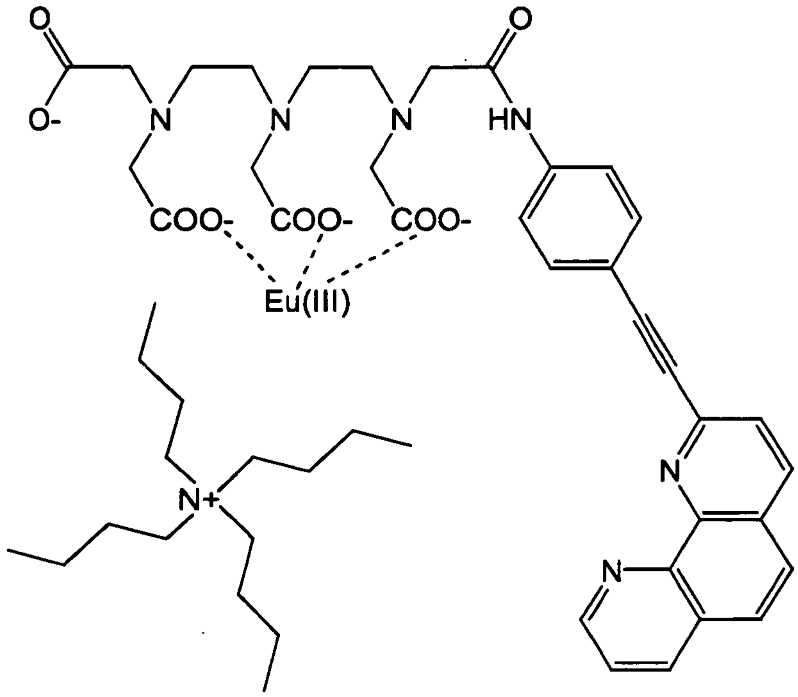

$^{n-}Bu_4N$[EuP*BL] wurden erstmals hergestellt und sorgfältig vermessen, ebenso der Komplex [TbP*BL]$^-$ und die Verbindung $^{n-}Bu_4N$ [TbP*BL]

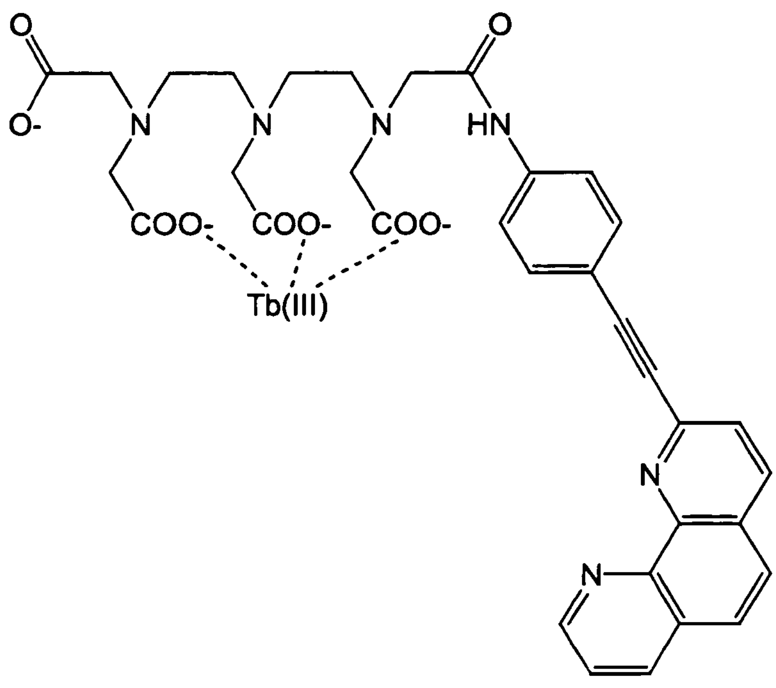

[TbP*BL]⁻

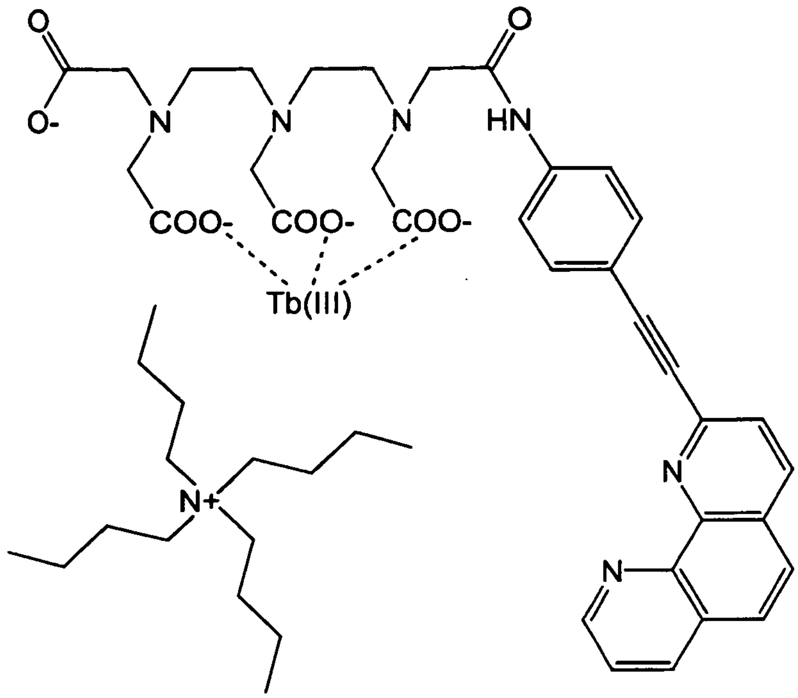

$^{n-}Bu_4N$ [TbP*BL ] .

P*$BLH_3$-EDA und P*$BLH_3$SP1

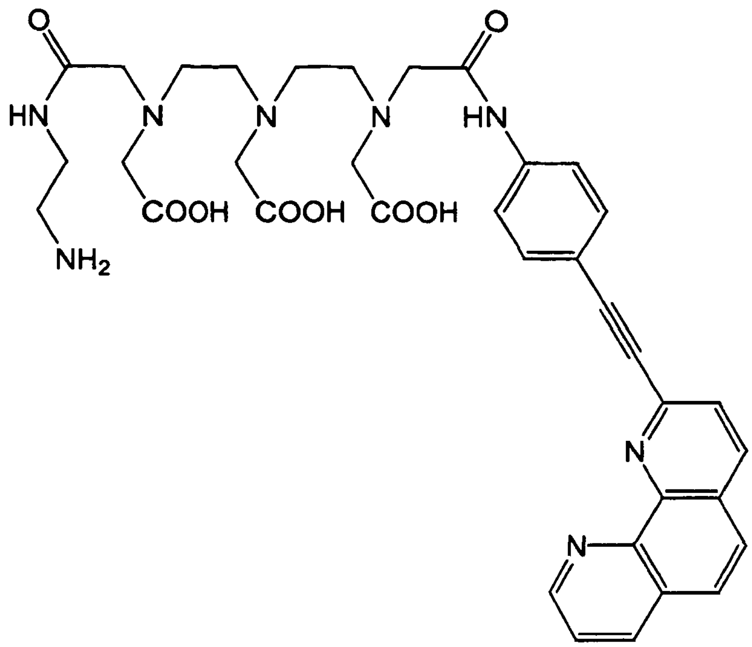

$C_{36}H_{40}N_8O_8$

P*BLH$_3$-EDA

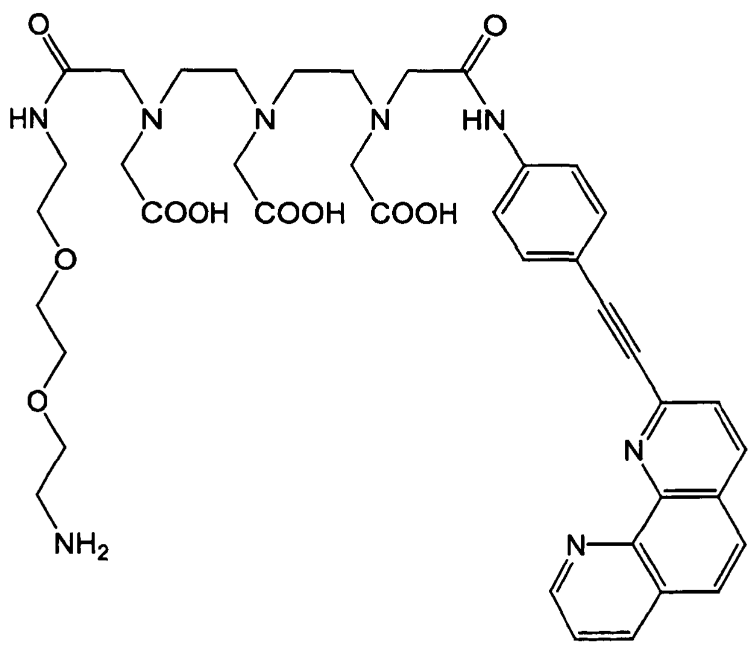

$C_{40}H_{48}N_8$

P*BLH$_3$-SP1

koordinieren mit Eu(III) zu den Antennenkomplexen EuP*BL-EDA und dem Antennenkomplex EuP*BL-SP1

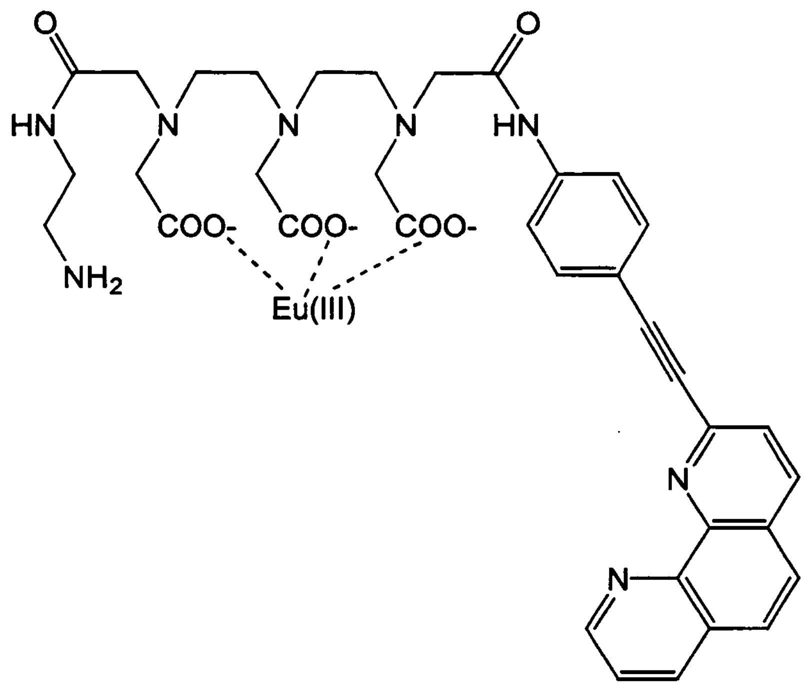

EuP*BL-EDA

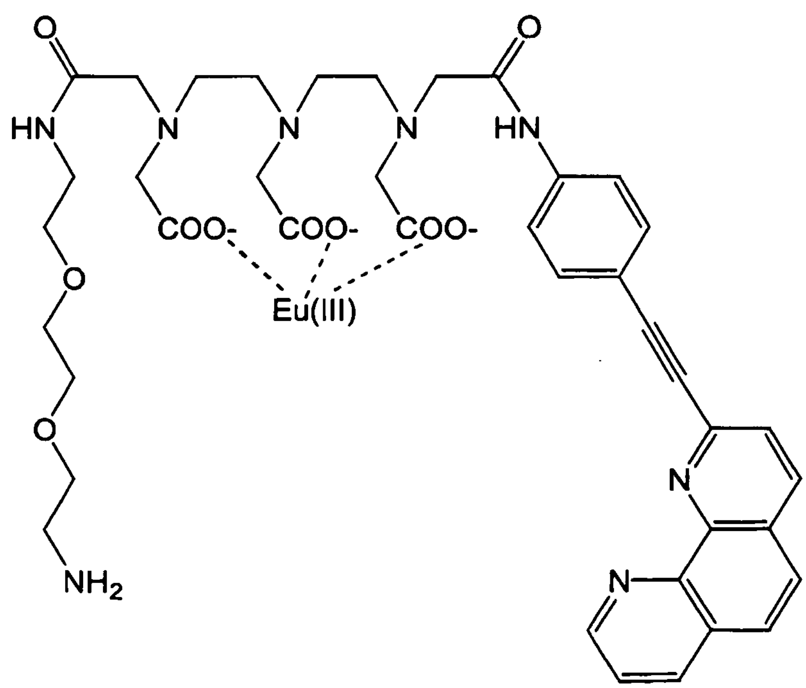

EuP*BL-SP1

Die gewonnenen Erkenntnisse haben zur Erarbeitung eines fluorimetrischen Analysenverfahrens auf der Basis

von TRF und FRET zum qualitativen Nachweis und zur quantitativen Bestimmung von Biomolekülen geführt, das dadurch charakterisiert ist, dass ein Ethinylanilin wie eingangs der Zusammenschau formelmäßig dargestellt und charakterisiert, durch eine Linkerreaktion kovalent an ein Biomolekül gebunden wird, wobei als zu markierende Biomoleküle Verbindungen aus den Substanzklassen der Peptide, Proteine, Oligonucleotide, Nucleinsäuren wie DNA und RNA, Oligo- und Polysaccharide, Glycoproteine, Phospholipide, niedermolekulare Substrate von Enzymen und niedermolekulare Liganden von Proteinen eingesetzt werden und der Nachweis des Analyten durch eine der folgenden Methoden erfolgt:

a. direkte Messung der Fluoreszenz des Lanthanoid(III)-Ions nach der TRF-Methode oder
b. nach Zugabe eines organischen Farbstoffs aus der Klasse der Polymethinfarbstoffe, gebunden an ein Biomolekül wie vorstehend bezeichnet, zur Messreaktion die von dem Donormolekül auf den organischen Farbstoff, nämlich das Akzeptormolekül übertragene Energie durch Messung der Fluoreszenzemission des Akzeptormoleküls bestimmt wird.

Deshalb kann Ethinylanilin erfolgreich in einem FRET-Bioassay veerwendet werden, der aus

- einem Antennenliganden mit dem Linker Ethylendiamin EDA als Feststoff
- N,N'-Carbonyldümidazol CDI oder N-Ethyl-N'-(3-diaminopropyl)-carbodiimid CDD und Triethanolamin/HCl-Puffer zur Markierung
- Eu(III)chlorid als $10^{-3}$ mol/l Lösung in Wasser
- Cy5 Farbstoff mit Säurefunktion als Feststoff, gegebenenfalls gebunden an einen sekundären Antikörper oder Protein A oder Protein G,

besteht, wobei

- als Antennenligand **P*BLH$_4$** verwendet wird

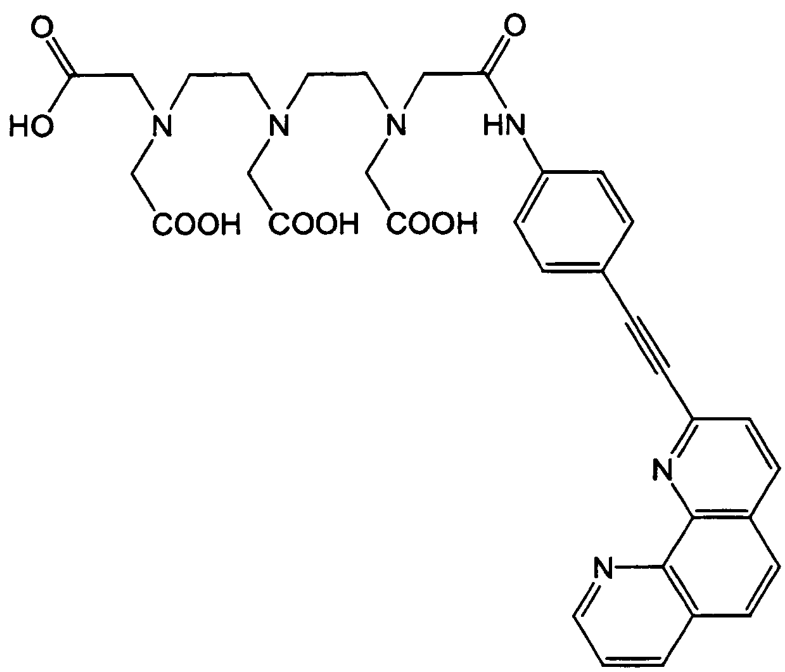

**P*BLH$_4$**

$C_{34}H_{34}N_6O_9$

wobei $LH_4$ für Diethylentriaminpentaessigsäure steht und $LH_3$, $LH_2$, LH, L für weitere Dissoziationsstufen der Diethylentriaminpenta-essigsäure stehen,

- der Cy5-Farbstoff mit Säurefunktion Cy5P oder Cy5A oder Cy5E oder Cy5eNCS ist

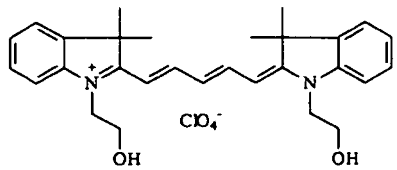

$C_{29}H_{35}N_2O_6Cl$

Polymethinfarbstoff **Cy5E**

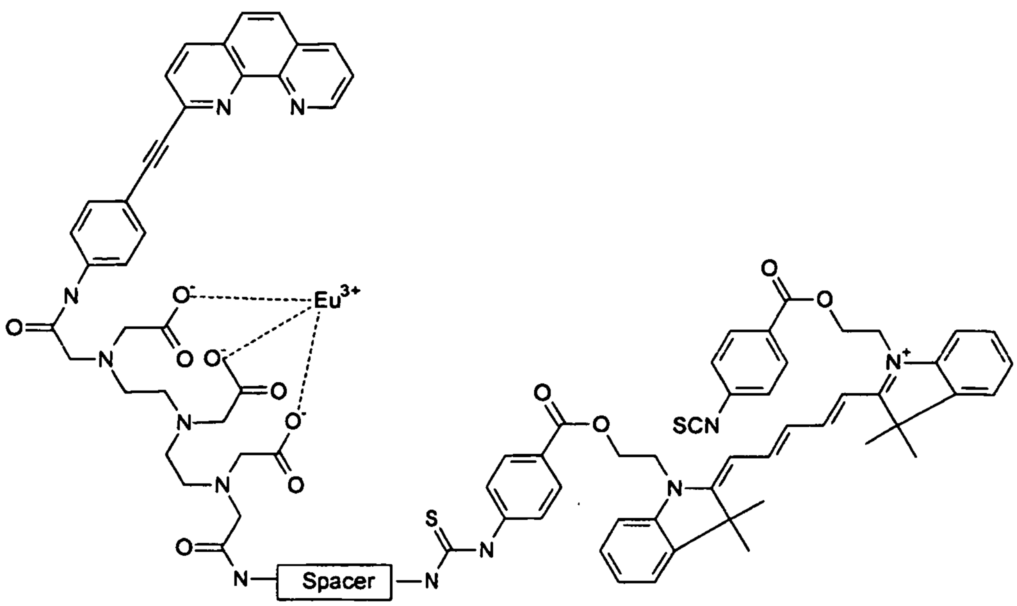

Akzeptor (Polymethinfarbstoff **Cy5ENCS**)

- und die Anregungswellenlänge $\lambda_{exc}$ 360 nm und die Detektionswellenlänge $\lambda_{em}$ 665 nm beträgt.

Nachfolgend werden Substanzen aufgeführt, die beispielhaft zur Erläuterung der Erfindung dienen.
Katalog der synthetisierten Substanzen

3,8-Dibromphenanthrolin (**P**)

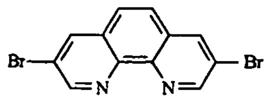

$C_{12}H_6N_2Br_2$

mp: 276 °C
$^1$H-NMR (ppm, $CDCl_3$):
9.18 (d, 2H phen-2,9), 8.41 (d, 2H phen-4,7), 7.76 (s, 2H phen-5,6)
IR ($cm^{-1}$, KBr):
3025 m, 1617 m, 1586 s, 1574 m, 1478 m, 1414 s, 1372 m, 1208 s, 1103 s, 1035 m, 906 s, 894 s, 784 s, 722 s, 509 w
EI-MS (m/z $M^+$): 337.9 ber.: 338.00

1-Methyl-1,10-phenanthrolin-1-iumiodid **(P*(a))**

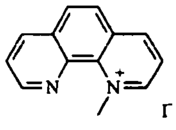

$C_{13}H_{11}N_2I$

mp: 210-213 °C
$^1$H-NMR (ppm, $D_2O$):
9.07 (s, 1 H phen-9), 8.89 (d, 2H phen-4,7), 8.12 (d, 2H phen-3,8), 7.75 (s, 2H phen-5,6), 7.63 (s, 1 H phen-2), 4.98 (s, 3H $CH_3$)

1-Methyl-1,10-phenanthrol-2-on **(P*(b))**

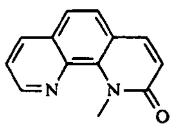

$C_{13}H_{10}N_2O$

mp: 123-124 °C
$^1$H-NMR (ppm, $CDCl_3$):
8.88 (d, 1 H phen-9), 8.12 (d, 1 H phen-7), 7.73 (d, 1 H, phen-4), 7.50 (s, 2H phen-5,6), 7.45 (m, 1 H phen-8), 6.92 (d, 1 H phen-3),4.43 (s, 3H $CH_3$)

2-Chlorphenanthrolin **(P*)**

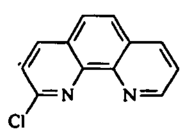

$C_{12}H_7N_2Cl$

mp: 129 - 130 °C
$^1$H-NMR (ppm, MeOH-$d_4$):
9.01 (d, 1 H phen-9), 8.37 (m, 2H phen-4,7), 7.86 (d, 2H phen-5,6), 7.71 (m, 2H phen-3,8) EI-MS (m/z $M^+$): 214.0 ber.: 214.03

3-(2-Hydroxy-2-Methylbut-3-inyl)anilin **(A(a2))**

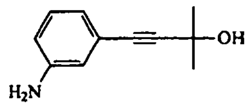

$C_{11}H_{13}NO$

mp: 114 - 115 °C
$^1$H-NMR (ppm, $CDCl_3$):
7.08 (t, 1 H Ph), 6.82 (d, 1 H Ph), 6.74 (s, 1 H Ph), 6.63 (dd, 1 H Ph), 3.03 (br s, 2H $NH_2$), 1.60 (s, 6H, $CH_3$), 1.25 (s, 1 H OH)
$^{13}$C-NMR (ppm, $CDCl_3$):
146.9 (C-$NH_2$), 129.9 (Ph), 124.1 (Ph), 122.8 (Ph), 118.6 (Ph), 115.9 (Ph), 93.9 (C≡C), 83.0 (C≡C), 66.3 ($C(CH_3)_2$), 32.2 ($CH_3$)
IR ($cm^{-1}$, KBr):
3382 s, 3204 s, 2980 s, 2932 m, 2220 w, 1600 s, 1582 s, 1485 s, 1446 s, 1372 m, 1360 m, 1295 m, 1228 s, 1160 s, 1131 s, 973 m, 943 s, 891 s, 872 s, 794 s, 691 s, 662 m, 571 w, 553 w, 534 w, 481 m, 464 w
EI-MS (m/z $M^+$): 175.1 ber.: 175.301

3-Ethinylanilin **(A)**

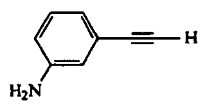

$C_8H_7N$

bp: 68 °C bei p = $3.9 \cdot 10^{-1}$ Torr
$^1$H-NMR (ppm, $C_6D_6$):
6.97 (d, 1 H Ph), 6.83 (t, 1 H Ph), 6.58 (s, 1 H Ph), 6.15 (dd, 1 H Ph), 2.73 (s, 1 H ≡CH), 2.68 (br s, 2H $NH_2$)
$^{13}$C-NMR (ppm, $C_6D_6$):
147.7 (C-$NH_2$), 130.1 (Ph), 124.1 (Ph), 122.9 (Ph), 118.9 (Ph), 116.2 (Ph), 85.2 (C≡C), 77.8 (C≡C)
IR ($cm^{-1}$, KBr):
3443 s, 3362 s, 3287 s, 3047 m, 2107 m, 1622 s, 1530 m, 1488 s, 1446 s, 1316 s, 1285 s, 1156 s, 995 s, 932 s, 867 s, 788 s, 689 s, 533 s, 460 s

4-(2-Hydroxy-2-Methylbut-3-inyl)anilin (**B(a2)**)

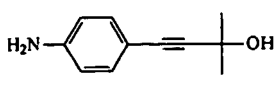

$C_{11}H_{13}NO$

mp: 79 °C
$^1$H-NMR (ppm, $CDCl_3$):
7.22 (d, 2H Ph), 6.59 (d, 2H Ph), 3.77 (s, 2H $NH_2$), 1.59 (s, 6H $CH_3$)
$^{13}$C-NMR (ppm, $CDCl_3$):
147.2 (C-$NH_2$), 133.7 (Ph), 115.3 (Ph), 112.8 (Ph), 92.2 (C≡C), 83.3 (C≡C), 66.4 ($C(CH_3)_2$), 32.3 ($CH_3$)

4-Nitrophenylacetylen **(B(b3))**

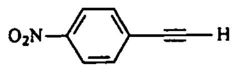

$C_8H_5NO_2$

mp: 150 °C
$^1$H-NMR (ppm, MeOH-$d_4$):
8.23 (d, 1 H Ph), 7.69 (d, 1 H Ph), 3.93 (s, 1 H ≡CH)

4-Ethinylanilin **(A)**

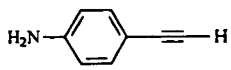

$C_8H_7N$

mp: 99 -100 °C
$^1$H-NMR (ppm, $C_6D_6$):
7.32 (d, 2H Ph), 6.03 (d, 2H Ph), 2.76 (s, 1 H ≡CH), 2.71 (br s, 2H $NH_2$)
IR ($cm^{-1}$, KBr):
3486 s, 3388 s, 3305 w, 3260 s, 3036 w, 2098 s, 1618 s, 1512 s, 1305 s, 1178 s, 829 s, 672 m, 605 m, 532 s

3,8-Bis(3'-Aminophenylethinyl)-1,10-phenanthrolin **($PA_2$)**

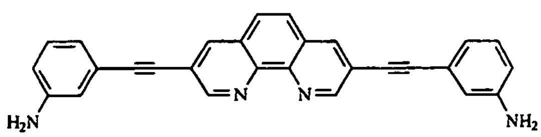

$C_{28}H_{18}N_4$

mp: 252 °C
$^1$H-NMR (ppm, $CDCl_3$):
9.18 (d, 2H phen-2,9), 8.71 (d, 2H phen-4,7), 8.05 (s, 2H phen-5,6), 7.11 (t, 2H Ph), 6.84 (s, 2H Ph), 6.79(d, 2H Ph), 6.67 (d, 2H Ph)
$^{13}$C-NMR (ppm, DMSO-$d_6$):
151.5 (phen-C2,9), 148.9 (C-$NH_2$), 140.0 (phen-C4,7), 138.2 (Ph), 129.4 (phen-C5,6), 127.2 (Ph), 121.7 (phen-C3,8), 118.9 (Ph), 116.2 (Ph), 115.2 (Ph), 94.7 (C≡C), 85.2 (C≡C)
EI-MS (m/z $M^+$): 410.0 ber.: 410.153
UV/Vis-Spektren (MeOH):
$\lambda_{abs}$ ($\varepsilon$ in $M^{-1}$ $cm^{-1}$): 216 ($2.2 \cdot 10^4$); 283 ($2.4 \cdot 10^4$); 348 ($2.2 \cdot 10^4$)

3,8-Bis(4'-Aminophenylethinyl)-1,10-phenanthrolin **($PB_2$)**

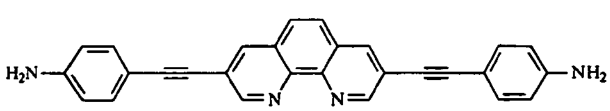

$C_{28}H_{18}N_4$

mp: 249 - 250 °C
$^1$H-NMR (ppm, DMSO-$d_6$):

9.09 (s, 2H phen-2,9), 8.47 (s, 2H phen-4,7), 7.92 (d, 2H phen-5,6), 7.35 (d, 2H Ph), 6. 70 (d, 2H Ph), 4.56 (br s, 4H $NH_2$)
$^{13}$C-NMR (ppm, DMSO-$d_6$):
151.3 (phen-C2,9), 150.1 (C-$NH_2$), 137.0 (phen-C4,7), 133.1 (Ph), 127.9 (phen-C5,6), 119.7 (phen-C3,8), 113.6 (Ph), 107.2 (Ph), 96.1 (C≡C), 84.0 (C≡C)
IR ($cm^{-1}$, KBr):
3438 s, 3211 w, 3033 w, 2200 s, 1619s, 1598 s, 1515 s, 1423 m, 1286 m, 1177 m, 1138 m, 909 w, 830 m, 728 m, 532 w
EI-MS (m/z $M^+$): 410.1 ber.: 410.153
UV/Vis-Spektren (MeOH):
$\lambda_{abs}$ (ε in $M^{-1}$ $cm^{-1}$): 280 ($4.2 \cdot 10^4$); 395 ($4.2 \cdot 10^4$)

2-(4'-Aminophenylethinyl)-1,10-phenanthrolin **(P*B)**

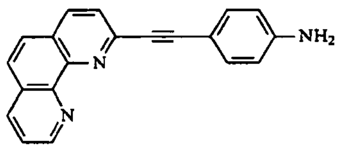

$C_{20}H_{13}N_3$

mp: 240 - 242 ˚C
$^1$H-NMR (ppm, $CDCl_3$):
9.23 (s, 1 H phen-9), 8.25 (d, 1 H phen-4), 8.19 (d, 1 H phen-7), 7.83 (d, 1 H phen-3), 7.77 (s, 2H phen-5,6), 7.64 (d, 1 H phen-8), 7.49 (d, 2H Ph), 6.66 (d, 2H Ph), 2.00 (br s, 2H $NH_2$)
$^{13}$C-NMR (ppm, $CDCl_3$):
151.1 (phen-C9), 148.2 (C-$NH_2$), 145.1 (phen-C2), 136.6 (phen-C4), 136.5 (phen-C7), 134.5 (Ph), 127.3 (phen-C5), 127.1 (phen-C6), 126.9 (phen-C3), 123.8 (phen-C8), 115.3 (Ph), 111.9 (Ph), 116.4 (Ph), 92.6 (C≡C), 89.3 (C≡C)
IR ($cm^{-1}$, KBr):
3439 m, 3295 s, 3199 m, 3035 m, 2194 s, 1618 s, 1603 s, 1582 s, 1549 m, 1518 s, 1482 s, 1445 s, 1386 m, 1307 m, 1154 s, 1082 m, 853 s, 829 s, 741 m, 630 m, 533 m, 473 w
EI-MS (m/z $M^+$): 295.2 ber.: 295.11
UV/Vis-Spektren (MeOH):
$\lambda_{abs}$ (ε in $M^{-1}$ $cm^{-1}$): 233 ($3.8 \cdot 10^4$); 275 ($4.7 \cdot 10^4$); 370 ($3.4 \cdot 10^4$)

Diethylentriaminpentaessigsäure-dianhydrid (**LH-A**)

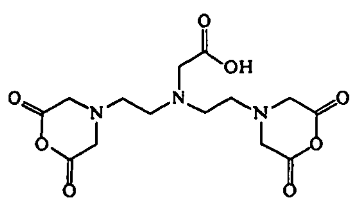

$C_{14}H_{19}N_3O_8$

mp: 176-179 ˚C
$^1$H-NMR (ppm, $CDCl_3$):
3.61 (s, 2H $CH_2$), 3.44 (s, 8H $CH_2$), 3.02 (t, 4H en), 2.89 (t, 4H en)
IR ($cm^{-1}$, KBr):
3433 m, 2979 m, 2858 w, 1820 s, 1774 s, 1641 s, 1473 m, 1459 m, 1441 m, 1306 m, 1257m, 1219 m, 1109 s, 958 m, 944 m, 887 w, 606 m, 566 m, 416 m

Antennenligand **PA2LH3**

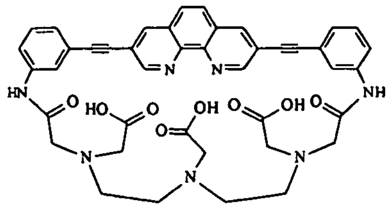

$C_{42}H_{37}N_7O_8$

mp: 195 ˚C Zersetzung
$^1$H-NMR (ppm, DMSO-$d_6$):
10.33 (br s, OH), 9.23 (d, 2H phen-2,9), 8.78 (d, 2H phen-4,7), 8.12 (s, 2H phen-5,6), 8.09 (s, Ph), 7.76 (m, Ph), 7.37 (m, Ph), 7.11 (d, 2H Ph), 3.58 (s, $CH_2$), 3.50 (s, $CH_2$), 3.47 (s, $CH_2$), 3.10 (t, en), 3.02 (t, en)
IR ($cm^{-1}$, KBr):
3438 s, 2961 m, 2921 m, 2854 m, 2208 m, 1715 s, 1682 s, 1633 s, 1582 m, 1531 m, 1484 m, 1422 s, 1224 m, 1089 w, 909 m, 792 m, 727 m, 686 m, 527 w
ESI-MS (m/z $[M-H]^-$): 766.26256 ber.: 766.26236
UV/Vis-Spektren ($H_2O/NH_3$ pH = 10):
$\lambda_{abs}$ in nm (ε in $M^{-1}$ $cm^{-1}$): 205 ($2.7 \cdot 10^4$); 246 ($2.6 \cdot 10^4$); 338 ($1.8 \cdot 10^4$)

Antennenligand **(PB2)2L2H6**

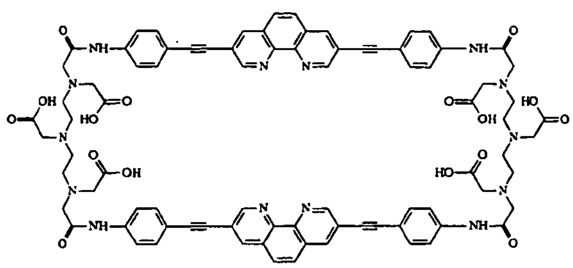

$C_{84}H_{74}N_{14}O_{16}$

mp: > 200˚C Zersetzung
$^1$H-NMR (ppm, DMSO-$d_6$):
10.5 (br s, OH), 8.75 (s, 2H phen-2,9), 8.02 (s, 2H phen-4,7), 7.46 (d, 2H phen-5,6), 7.01 (d, 2H Ph), 6. 35 (d, 2H Ph)
IR ($cm^{-1}$, KBr):
3438 s, 2961 m, 2921 m, 2854 m, 2208 w, 1715 s, 1682 s, 1633 s, 1582 m, 1531 m, 1484 m, 1422 m, 1224 m, 1089 w, 909 m, 792 m, 727 m, 686 m
ESI-MS (m/z $[2M-2H]^{2-}$, MeOH, $H_2O$, $NH_3$): 766.26348 ber.: 766.26308
UV/Vis-Spektren ($H_2O/NH_3$ pH = 10):
$\lambda_{abs}$ in nm (ε in $M^{-1}$ $cm^{-1}$): 282 (3.9.$10^4$); 353 ($3.6 \cdot 10^4$)

Antennenligand **P*BLH4**

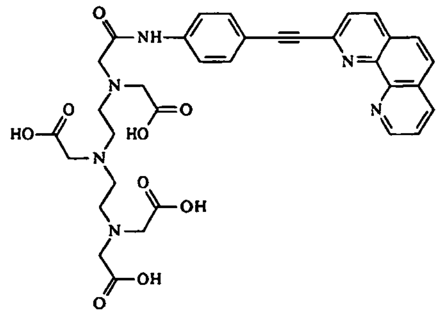

$C_{34}H_{34}N_6O_9$

mp: ab 181 ˚C
$^1$H-NMR (ppm, DMSO-$d_6$):
10.41 (br s OH), 9.11 (dd), 8.50 (m), 8.02 (s), 7.89 (m), 7.67 (d), 7.64 (d), 3.54 (s, $CH_2$), 3.50 (s, $CH_2$), 3.45 (s, $CH_2$), 3.43 ($CH_2$), 3.09 - 2.88 (m, en)
FAB-MS (m/z $[M+H]^+$, 3-NBA): 671.2 ber.: 671.2
UV/Vis-Spektren ($H_2O/NH_3$ pH = 10):
$\lambda_{abs}$ in nm ($\varepsilon$ in $M^{-1}$ $cm^{-1}$): 232 ($3.6\cdot10^4$); 311 ($3.4\cdot10^4$); 360 ($2.0\cdot10^4$)

Antennenligand **P*BLH3-EDA**

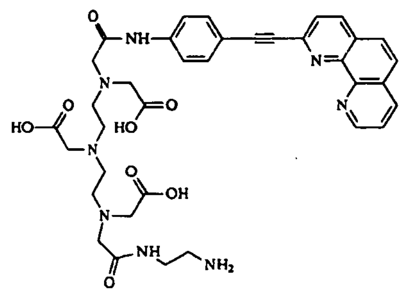

$C_{36}H_{40}N_8O_8$

mp: 190 ˚C
$^1$H-NMR (ppm, $D_2O$):
Zuordnung der Signale erfolgte nicht.
ESI-MS (m/z $[M+H]^+$, $MeOH/H_2O$ + $NH_4COOH$): 713.30462 ber.: 713.30474
UV/Vis-Spektren ($H_2O$):
$\lambda_{abs}$ in nm ($\varepsilon$ in $M^{-1}$ $cm^{-1}$): 232 ($2.6.10^4$); 272 ($1.8\text{-}10^4$); 310 ($2.2\cdot10^4$); 360 ($1.4\cdot10^4$)

Antennenligand **P*BLH3-SP1**

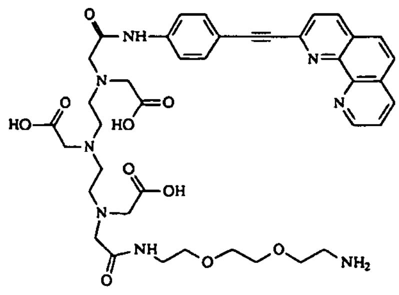

$C_{40}H_{48}N_8O_{10}$

mp: 135 ˚C
$^1$H-NMR (ppm, $D_2O$):
Zuordnung der Signale erfolgte nicht.
ESI-MS (m/z $[M+H]^+$, MeOH/$H_2O$): 801.35753 ber.: 801.35662
UV/Vis-Spektren ($H_2O$):
$\lambda_{abs}$ in nm ($\varepsilon$ in $M^{-1}$ $cm^{-1}$): 232 ($2.6\cdot10^4$); 274 ($1.8\cdot10^4$);311 ($2.2\cdot10^4$); 359 ($1.4\cdot10^4$)

Indoleninderivat **TIPBr⁻**

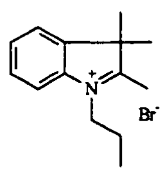

$C_{14}H_{20}NBr$

mp: 158 -159 ˚C
ESI-MS ($M^+$, MeOH): 202.0 ber.: 202.159
$^1$H-NMR (ppm, MeOH-$d_4$):
7.89 (m), 7.78 (m), 7.66 (m), 4.51 (t), 2.86 (m), 2.03 (sextett), 1.62 (s), 1.11 (t)

Indoleninderivat **TIEOBr⁻**

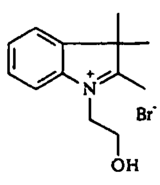

$C_{13}H_{18}NOBr$

mp: 195 ˚C
$^1$H-NMR (ppm, MeOH-$d_4$):
7.89 (m), 7.78 (m), 7.66 (m) 4.69 (t), 4.06 (q), 3.14 (s), 2.24 (s), 1.63 (s)

Indoleninderivat **TIPEBr⁻**

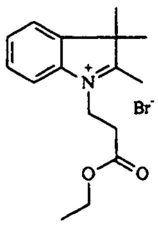

$C_{16}H_{22}NO_2Br$

mp: 130 °C
ESI-MS ($M^+$): 260.2 ber.: 260.165
$^1$H-NMR (ppm, $CDCl_3$):
7.98 (d), 7.58 (m), 4.79 (t), 4.05 (q), 3.14 (s), 2.68 (t), 2.24 (m), 1.62 (s), 1.19 (t)
$^{13}$C-NMR (ppm, $CDCl_3$):
196.8, 173.2, 142.1, 141.7, 130.7, 130.2, 123.8, 116.5, 61.6, 55.3, 49.4, 23.7, 23.5, 17.4, 14.7

Indoleninderivat **TIBEI**$^-$

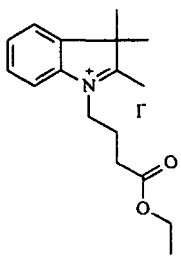

$C_{17}H_{24}NO_2I$

mp: 148°C
ESI-MS ($M^+$): 274.1 ber.: 274.180
$^1$H-NMR (ppm, MeOH-$d_4$):
8.04 (m), 7.80 (m), 7.65 (m), 4.62 (t), 4.09 (q), 3.14 (s), 2.68 (t), 2.26 (m), 1.65 (s), 1.22 (t)

Polymethinfarbstoff **ST936**

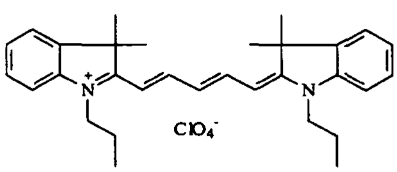

$C_{31}H_{39}N_2O_4Cl$

ESI-MS ($M^+$): 439.3 ber.: 439.311 1
$^1$H-NMR (ppm, $CDCl_3$):
8.05 (t), 7.37 (d), 7.35 (d), 7.22 (t), 7.07 (d), 6.64 (t), 6.39 (d), 4.51 (s), 3.99 (t), 3.75 (s), 3.47 (q), 1.74 (s), 1.20 (t), 1.04 (t)
UV/Vis-Spektren (MeOH):
$\lambda_{abs}$ in nm ($\varepsilon$ in $M^{-1}$ $cm^{-1}$): 642 ($1.73 \cdot 10^5$) $\lambda_{em}$ in nm: 668

Polymethinfarbstoff **Cy5EE**

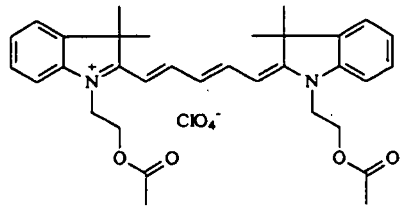

$C_{35}H_{43}N_2O_8Cl$

ESI-MS ($M^+$): 527.2 ber.:
$^1$H-NMR (ppm, MeOH-$d_4$):
8.31 (t), 7.49 (d), 7.43 (t), 7.34 (d), 7.28 (t), 6.64 (t), 6.39 (d), 4.56 (s), 4.51 (m), 4.42 (m), 3.17 (q), 1.86 (s), 1.73 (s), 1.31 (t)
$^1$H-NMR (ppm, DMSO-$d_6$):
9.95 (s), 8.39 (t), 7.62 (d), 7.41 (s), 7.25 (d), 6.56 (t), 6.41 (d), 5.32 (s), 4.42 (s), 3.34 (s), 3.06 (dd), 1.82 (s), 1.67 (s), 1.23 (s), 1.19 (t)
$^{13}$C-NMR (ppm, DMSO-$d_6$):
173.6, 170.1, 154.5, 142.1, 140.9, 128.3, 125.5, 124.7, 122.4, 111.1, 103.6, 60.3, 45.4, 43.2, 27.5, 20.5, 8.5
UV/Vis-Spektren (MeOH):
$\lambda_{abs}$ in nm ($\varepsilon$ in $M^{-1}$ $cm^{-1}$): ($3.8 \cdot 10^4$) $\lambda_{em}$ in nm: 668

Polymethinfarbstoff **Cy5BE**

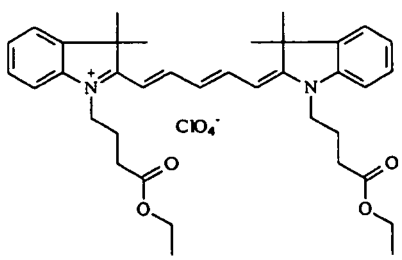

$C_{37}H_{47}N_2O_8Cl$

ESI-MS ($M^+$, MeOH): 583.35247 ber.: 583.35303
$^1$H-NMR (ppm, MeOH-$d_4$):
8.29 (t), 7.50 (d), 7.43 (t), 7.36 (d), 7.27 (t), 6.63 (t), 6.39 (d), 4.14 (m), 2.85 (t), 2.55 (t), 2.08 (m), 2.03 (s), 1.73 (s), 1.26 (t)
$^{13}$C-NMR (ppm, MeOH-$d_4$):
174.9, 174.5, 155.7, 143.5, 142.6, 129.8, 126.9, 126.3, 123.5, 111.9, 104.4, 63.9, 61.8, 54.4, 44.2, 31.2, 27.9, 23.5, 20.4, 14.5
UV/Vis-Spektren (MeOH):
$\lambda_{abs}$ in nm ($\varepsilon$ in $M^{-1}$ $cm^{-1}$): 642 ($1.3 \cdot 10^5$) $\lambda_{em}$ in nm: 668

Polymethinfarbstoff **Cy5'BE**

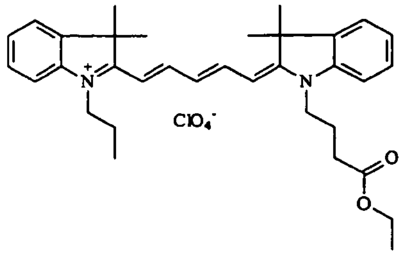

$C_{34}H_{43}N_2O_6Cl$

$^1$H-NMR (ppm, MeOH-$d_4$):
Zuordnung der Signale erfolgte nicht.
UV/Vis-Spektren (MeOH):
$\lambda_{abs}$ in nm (ε in $M^{-1}$ $cm^{-1}$): 643 ($7.1 \cdot 10^4$) $\lambda_{em}$ in nm: 664

Polymethinfarbstoff **Cy5BA**

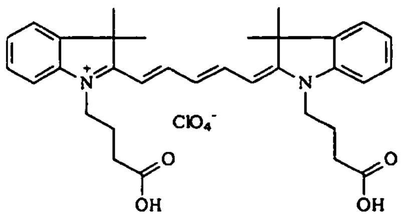

$C_{33}H_{39}N_2O_8Cl$

ESI-MS ($M^+$, MeOH): 527.2 ber.: 527.2909
$^1$H-NMR (ppm, MeOH-$d_4$):
Zuordnung der Signale erfolgte nicht.
UV/Vis-Spektren (MeOH): als inneres Salz
$\lambda_{abs}$ in nm (ε in $M^{-1}$ $cm^{-1}$): 642 ($7.9.10^4$)

Polymethinfarbstoff **Cy5'BA**

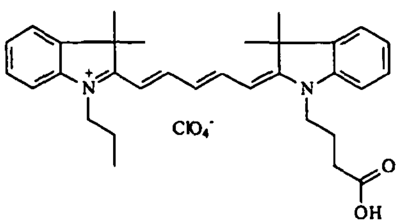

$C_{32}H_{39}N_2O_6Cl$

$^1$H-NMR (ppm, DMSO-$d_6$):
Zuordnung der Signale erfolgte nicht.
UV/Vis-Spektren (MeOH):
$\lambda_{abs}$ in nm (ε in $M^{-1}$ $cm^{-1}$): 643 ($1.42 \cdot 10^5$) $\lambda_{em}$ in nm: 668

Polymethinfarbstoff **Cy5E**

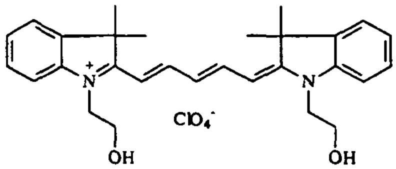

$C_{29}H_{35}N_2O_6Cl$

ESI-MS ($M^+$, MeOH): 443.2689 ber.: 443.2693
$^1$H-NMR (ppm, DMSO-$d_6$):
8.31 (t), 7.60 (d), 7.38 (s), 7.23 (m), 6.48 (t), 6.33 (d), 5.02 (t), 4.18 (s), 3.76 (d), 3.32 (s), 1.69 (s)
UV/Vis-Spektren (MeOH):
$\lambda_{abs}$ in nm ($\varepsilon$ in $M^{-1}$ $cm^{-1}$): 642 ($2.5 \cdot 10^5$) $\lambda_{em}$ in nm: 668

**Synthese des Polymethinfarbstoffs Cy5R**

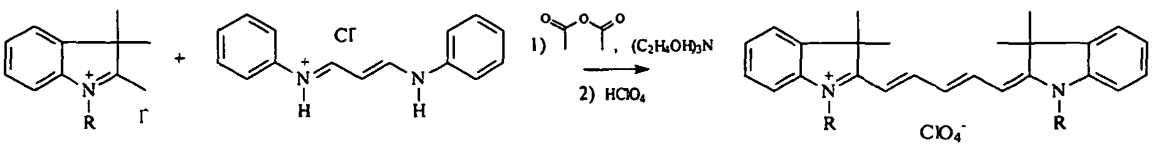


Eine Lösung von 20 mmol des alkylierten Indolenins und 10 mmol Dianilid in 50 ml Acetanhydrid wird auf 70˚C gebracht und langsam Triethanolamin zugetropft. Der Farbstoff wird mit Perchlorsäure gefällt und abgesaugt. Zum Umkristallisieren wird in wird mit Perchlorsäure gefällt und abgesaugt. Zum Umkristallisieren wird in wenig Methanol gelöst und die Lösung in das 10fache Volumen Diethylether gegeben und bei - 40˚C der Kristallisation überlassen (Ausbeute ca. 80%).

Indoleninderivat **TIPBr-**

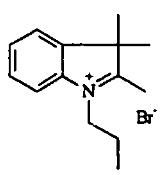

$C_{14}H_{20}NBr$

mp: 158 -159 ˚C
ESI-MS ($M^+$, MeOH): 202.0 ber.: 202.159
$^1$H-NMR (ppm, MeOH-$d_4$):
7.89 (m), 7.78 (m), 7.66 (m), 4.51 (t), 2.86 (m), 2.03 (sextett), 1.62 (s), 1.11 (t) Abbildungen

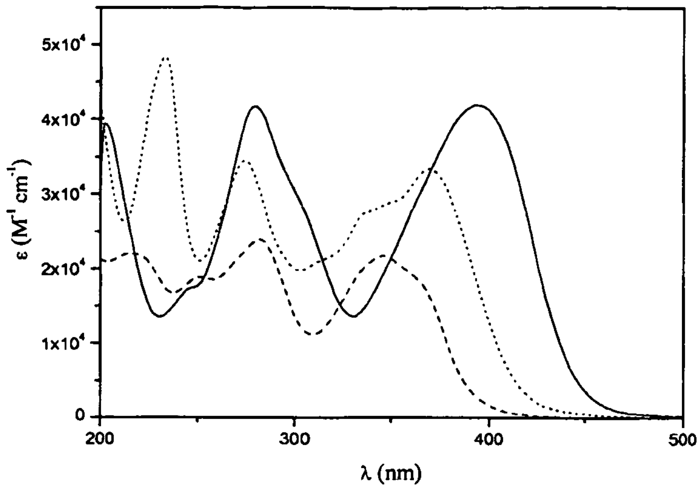


Abb. 1: Absorptionsspektren der Chromophore **$PA_2$** (----), **$PB_2$** (—) und **P*B** (····) in MeOH.

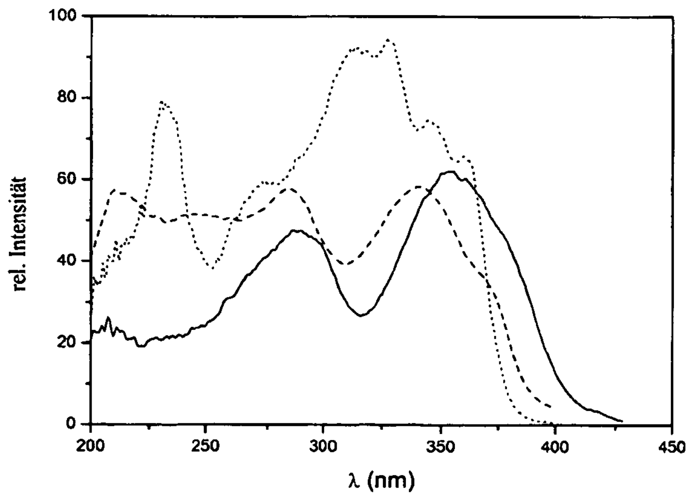


Abb. 2: Anregungsspektren der Antennenliganden **$PA_2LH_4$** (----), **$(PB_2)_2L_2H_6$** (—) und **P*$BLH_4$** (····) in $NH_3/H_2O$ bei pH = 10.

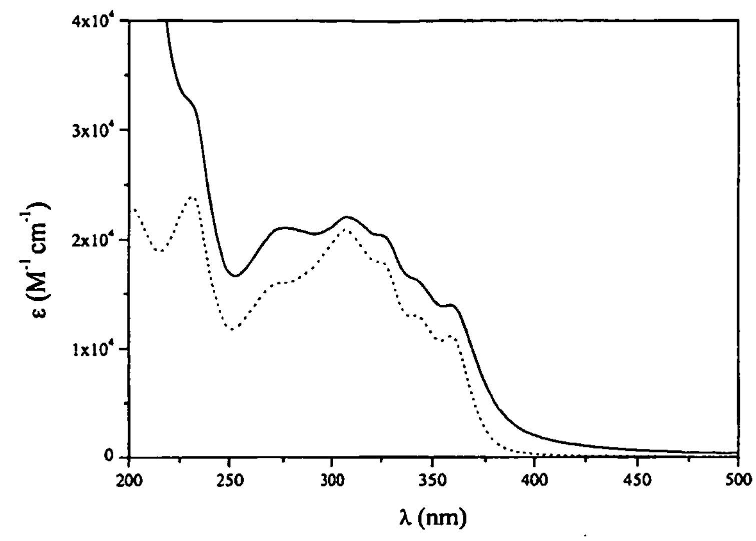


Abb. 3: UV/Vis-Spektren von **[EuP*BL]⁻** (—) und **[TbP*BL]⁻** (····) in $H_2O$.

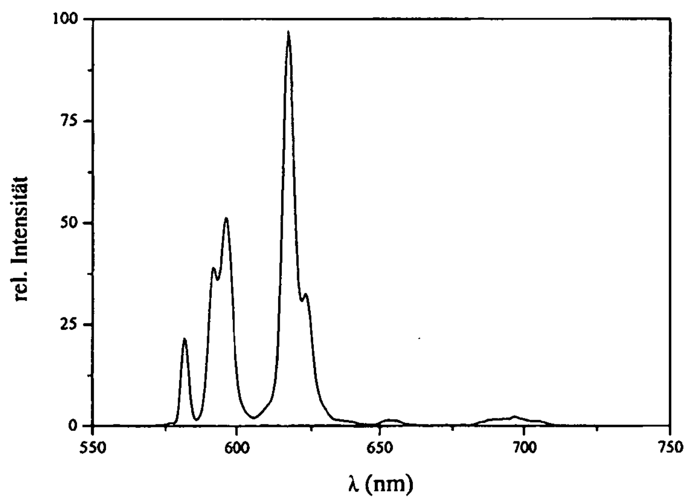


Abb. 4: Normalisiertes Emissionsspektrum von **[EuP*BL]⁻** in $H_2O$; (Anregungsbedingungen: $\lambda_{exc}$ = 360 nm, Spalt: 10/2.5 nm, dt = 0.07 ms, gt = 4.5 ms).

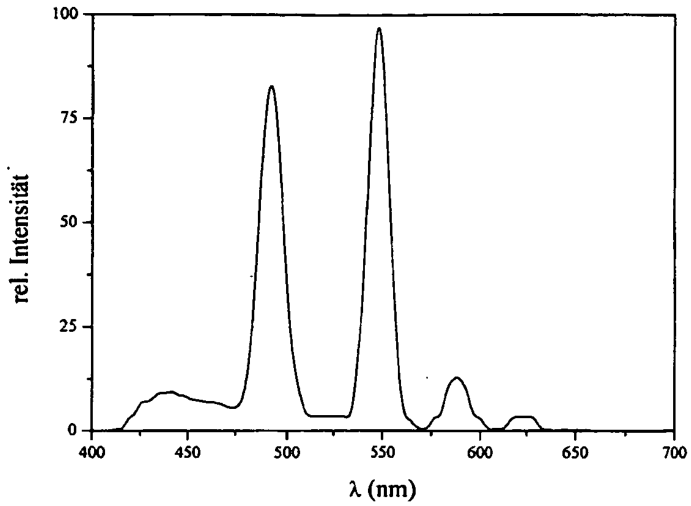


Abb. 5: Normalisiertes Emissionsspektrum von **[TbP*BL]**⁻ in $H_2O$; (Anregungsbedingungen: $\lambda_{exc}$ = 360 nm, Spalt: 10/5 nm, dt = 0.08 ms, gt = 4.6 ms).

Alle Fluoreszenzmessungen der Donoren **[EuP*BL]**$^-$ und **[TbP*BL]**$^-$ wurden mit einer eingestellten Zeitverzögerung verfolgt. Die Standardeinstellungen waren:

für **[EuP*BL]$^-$:** Anregungswellenlänge: $\lambda_{exc}$ = 360 nm
Anregungsspalt: 15 nm Emissionsspalt: 5 nm
Emissionsfilter: 515 nm
Zeitfenster (gt): 4.50 ms
Zeitverzögerung (dt): 0.07 ms

für **[TbP*BL]**$^-$ : Anregungswellenlänge: $\lambda_{exc}$ = 400 nm
Anregungsspalt: 15 nm Emissionsspalt: 10 nm
Emissionsfilter: 430 nm
Zeitfenster (gt): 4.60 ms
Zeitverzögerung (dt): 0.08 ms

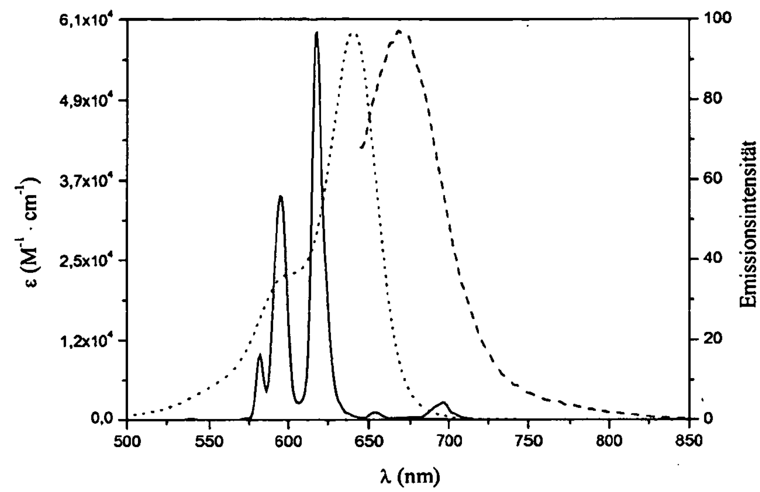


Abb. 6: Spektroskopische Eigenschaften von **[EuP*BL]⁻** und **ST936** in $NH_3/H_2O$ bei pH = 10 mit Absorption des Akzeptors (···) bzw. Donor- (—) und Akzeptor-Emission (----).

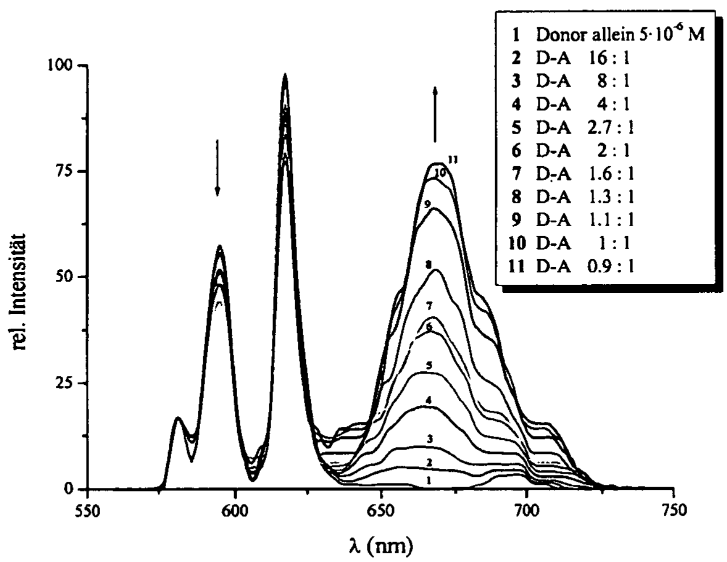


Abb. 7[P] FRET-Experiment von **[EuP*BLH₃ ]-EDACy5ENCS** in $H_2O$; korrigierte Emissionsspektren in Abhängigkeit steigender Akzeptorkonzentration. ($c_{DONOR} = 5 \cdot 10^{-6}$ M, $c_{AKZEPTOR} = 1.6 \cdot 10^{-7}$ M bis $5.6 \cdot 10^{-6}$ M)

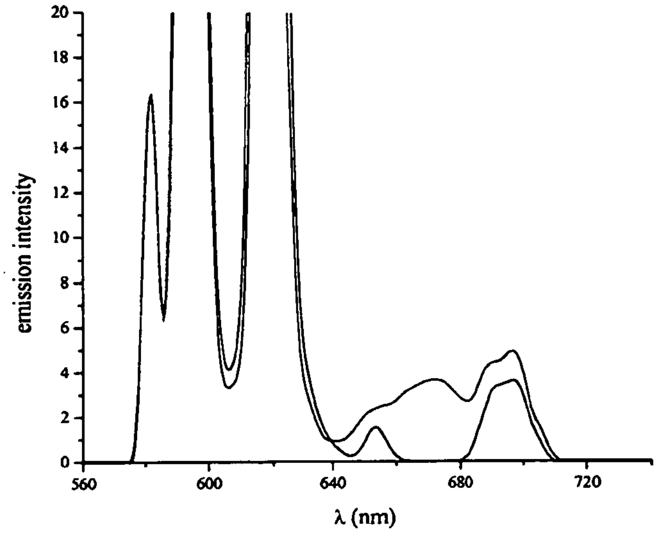


Abb. 8 Fluoreszenzspektren mit C(IgG)=C(PA)= $2{,}5*10^{-7}$ M (punktierte Linie = IgG, schwarze Linie = IgG+PA)

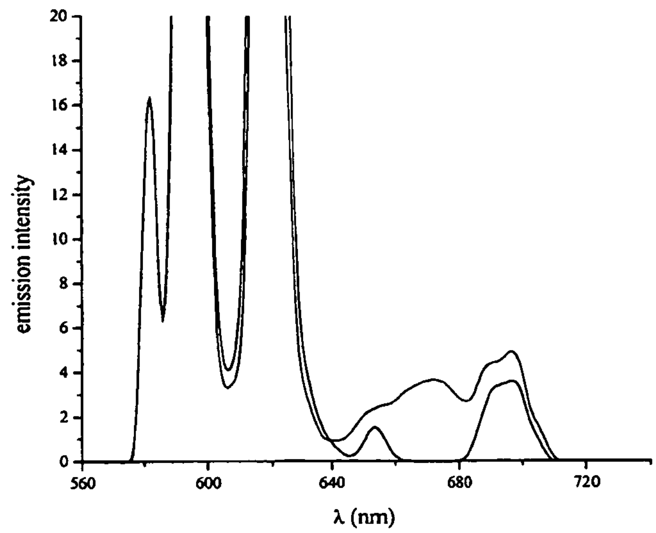


Abb. 9 Fluoreszenzspektren mit C(IgG)=C(PA)= $8{,}2*10^{-7}$ M (punktierte Linie = IgG, schwarze Linie = IgG+PA)

Abkürzungsverzeichnis

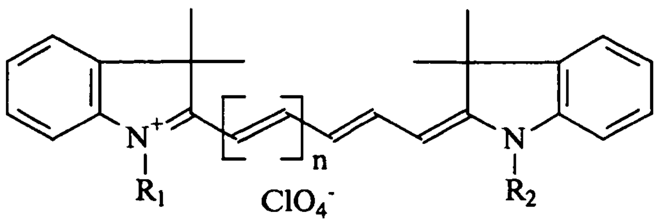

| Kürzel | n | $R_1$ | $R_2$ |
|---|---|---|---|
| **Cy3P** | 0 | $-C_3H_7$ | $-C_3H_7$ |
| **Cy5P** | 1 | $-C_3H_7$ | $-C_3H_7$ |
| **Cy3BA** | 0 | $-C_3H_6COOH$ | $-C_3H_6COOH$ |
| **Cy5BA** | 1 | $-C_3H_6COOH$ | $-C_3H_6COOH$ |
| **Cy3E** | 0 | $-C_2H_4OH$ | $-C_2H_4OH$ |
| **Cy5E** | 1 | $-C_2H_4OH$ | $-C_2H_4OH$ |
| **Cy3ENCS** | 0 | $-C_2H_4OOCPhNCS$ | $-C_2H_4OOCPhNCS$ |
| **Cy5ENCS** | 1 | $-C_2H_4OOCPhNCS$ | $-C_2H_4OOCPhNCS$ |
| **Cy3'BA** | 0 | $-C_3H_7$ | $-C_3H_6COOH$ |
| **Cy5'BA** | 1 | $-C_3H_7$ | $-C_3H_6COOH$ |
| **Cy3'E** | 0 | $-C_3H_7$ | $-C_2H_4OH$ |
| **Cy5'E** | 1 | $-C_3H_7$ | $-C_2H_4OH$ |
| **Cy3'ENCS** | 0 | $-C_3H_7$ | $-C_2H_4OOCPhNCS$ |
| **Cy5'ENCS** | 1 | $-C_3H_7$ | $-C_2H_4OOCPhNCS$ |

| | |
|---|---|
| CDI | N, N'Carbonyldiimidazol |
| CDD | N-Ethyl-N'-(3-dimethylaminopropyl)-carbodiimid |
| EDA | Ethylendiamin |
| FIA | Fluoreszenz-Immuno-Assay |
| phen | Phenanthrolin |
| DMF-TEA | Dimethylformamid-Triethanolamin |

**Patentansprüche**

**1.** Ein Ethinylanilin entsprechend der Formel

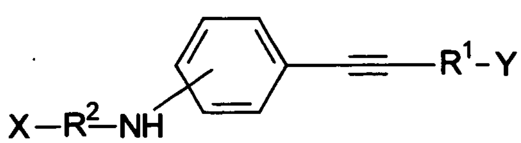

in der

$R^1$ eine Antennenfunktion ist, ausgesucht aus den folgenden: 1,10-Phenanthrolin (1) oder Fluoren (2) oder Acetophenon (3) oder Benzophenon (4) oder Fluorenon (5) oder Xanton (6) oder Azaxanton (7) oder Anthrachinon (8) oder Acridon (9) oder Chinolin (10) oder Cumarin (11)

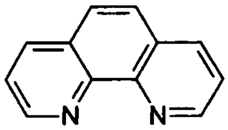
**1**
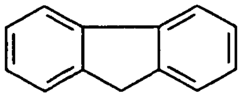
**2**
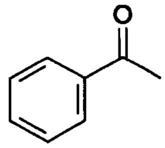
**3**
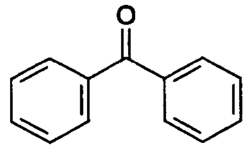
**4**

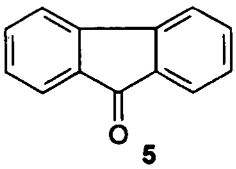
**5**
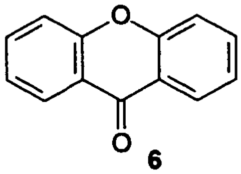
**6**
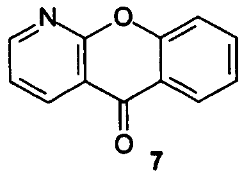
**7**
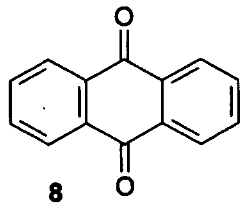
**8**

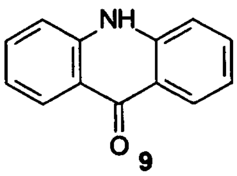
**9**
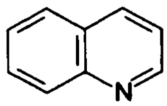
**10**
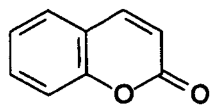
**11**

**$R^2$** ein Chelatbildner ist, der ein Lanthanoid(III)ion koordiniert enthält, wobei der Chelatbildner einer der folgenden ist:
DTPA (n=1), TTHA (n=2)

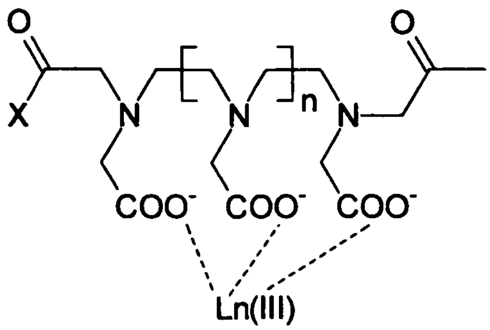

oder das TTHA-Isomer NTTHA

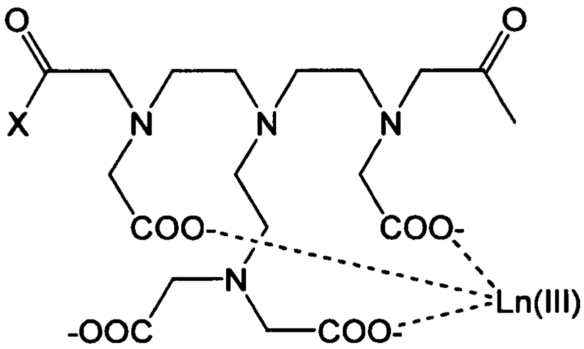

wobei die TTHA-Derivate ein zweites Ethinylanilin mit Antennenfunktion enthalten können, so dass immer drei Carboxylatreste zur Komplexbildung zur Verfügung stehen,

**X** -OH oder eine Haftgruppe zum Biomolekül ist, die über eine Amidbindung an einen Carboxylatrest des Chelatbildners gebunden ist, wobei als Haftgruppen in Frage kommen:

$-NH(CH_2)_2NH_2$, $-NH(CH_2)_2PhNH_2$, $-NH(CH_2)_2PhNCS$, $-NH(CH_2)_nNH(C_3N_3Cl_2)$ mit n = 2 bis 6 sowie $-NH(CH_2)_2O(CH_2)_2NH_2$ (SP1)

**Y** -H oder eine Haftgruppe zum Biomolekül, die an die Antennenfunktion gekoppelt ist, wobei die Haftgruppen aus den folgenden ausgesucht sind:

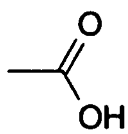

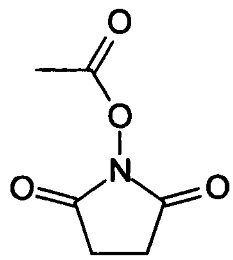

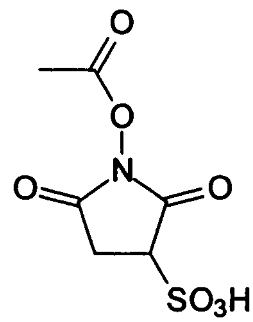

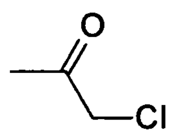

—NCS

—$NH_2$

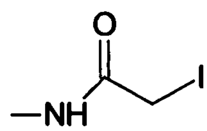

e) die Lanthanoid(III)ionen die folgenden sind: $Eu^{3+}$ , $Tb^{3+}$, $Dy^{3+}$, $Sm^{3+}$.

**2.** 2-(4'-Aminophenylethinyl)-1,10-phenanthrolin P*B

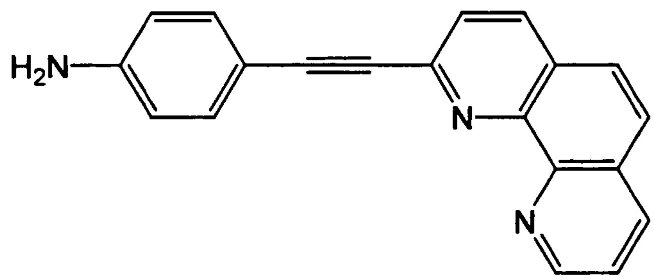

$C_{20}H_{13}N_3$

**3.** Verfahren zur Herstellung von 2-(4'-Aminophenylethinyl)-1,10-phen P*B durch Umsetzen von 2-Chlor-1,10-phenanthrolin mit p-Ethinylanilin

**4.** Verwendung von P*B als Antennenchromophor

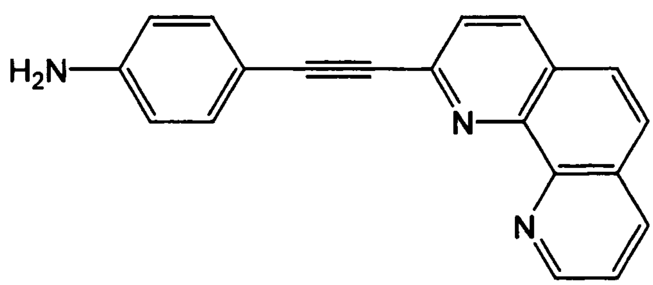

**5.** Chelatligand $P^*BLH_4$

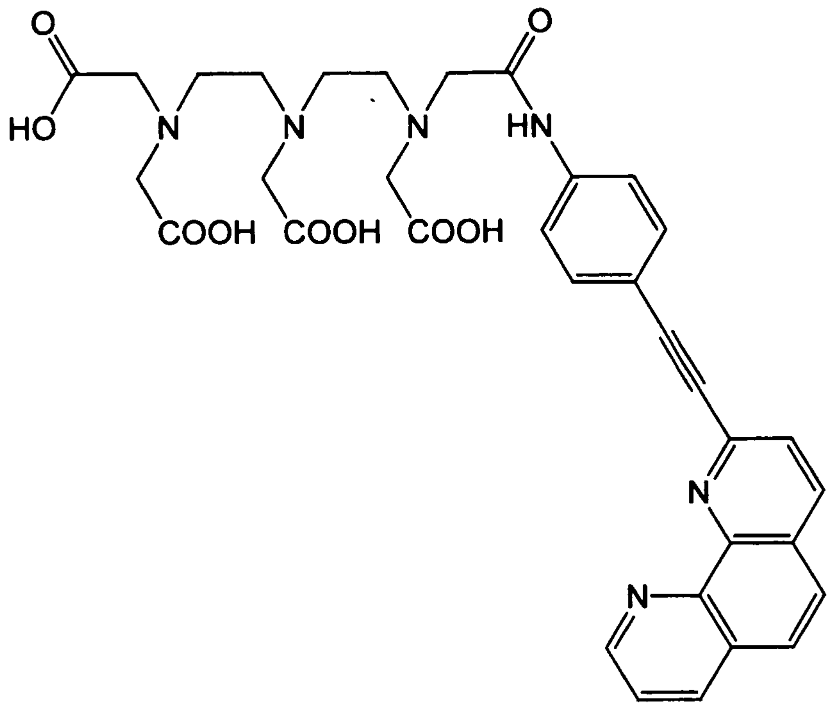

$C_{34}H_{34}N_6O_9$

wobei $LH_4$ für Diethylentriaminpentaessigsäure steht und $LH_3$, $LH_2$, LH, L für weitere Dissoziationsstufen der Diethylentriaminpenta-essigsäure stehen.

**6.** Verwendung des Chelatliganden $P^*BLH_4$ als Antennenligand

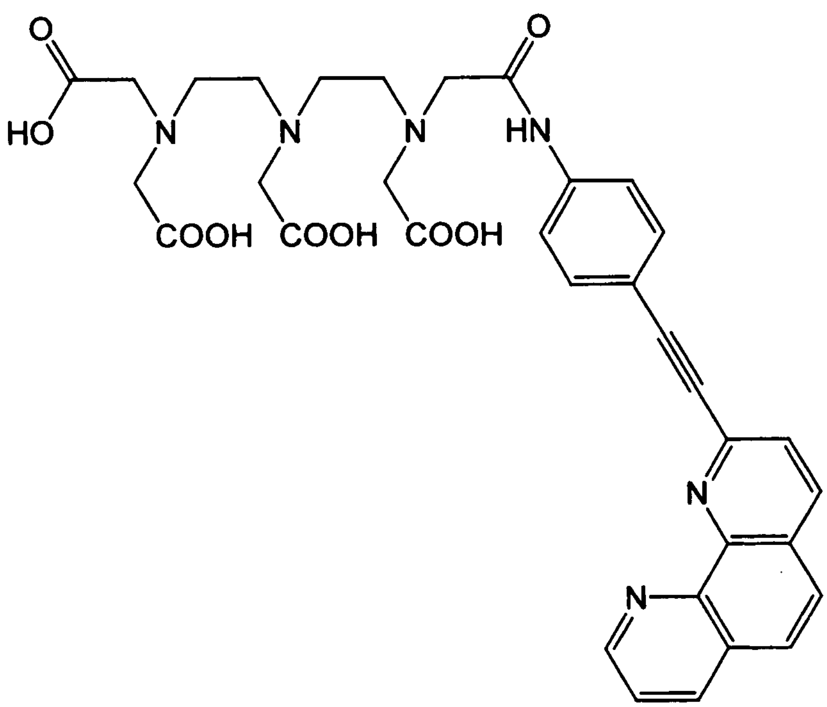

**7.** Komplex [EuP*BL)]$^-$ und Komplex $^{n-}Bu_4N$[EuP*BL]

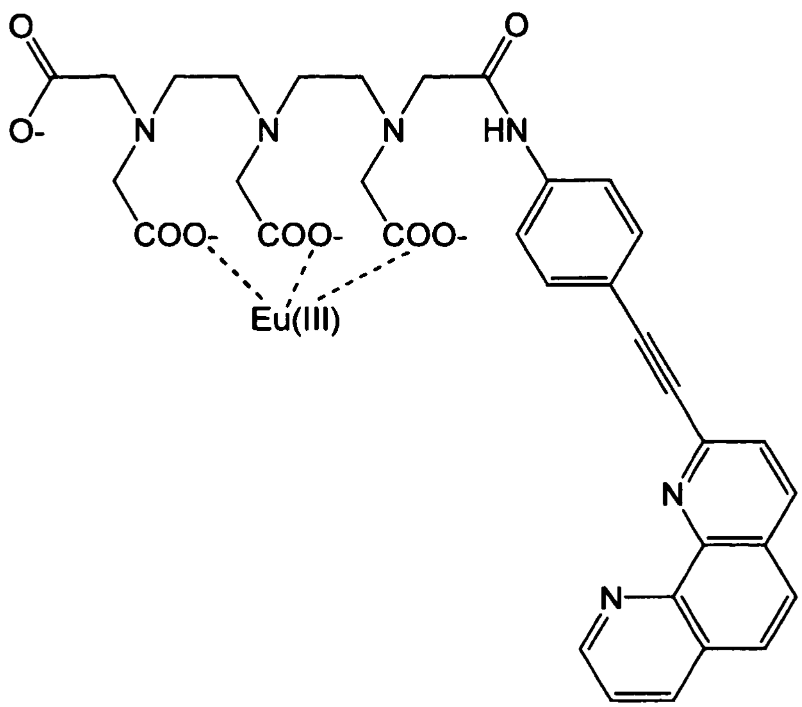

[EuP*BL)]$^-$

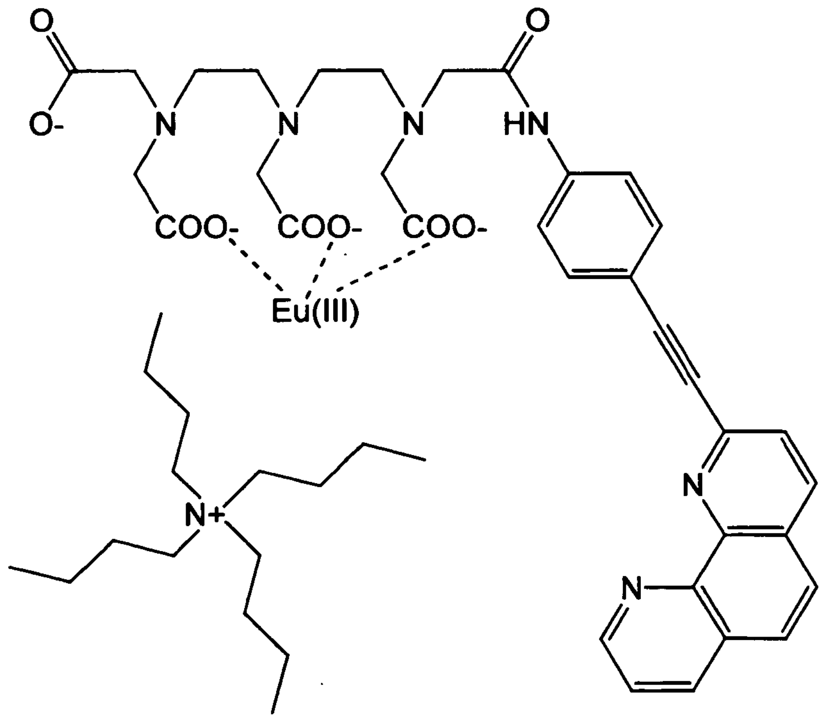

$^{n-}Bu_4N[\ EuP^*BL]$

**8.** Komplex $[TbP^*BL]^-$ und Komplex $^{n-}Bu_4N\ [TbP^*BL]$

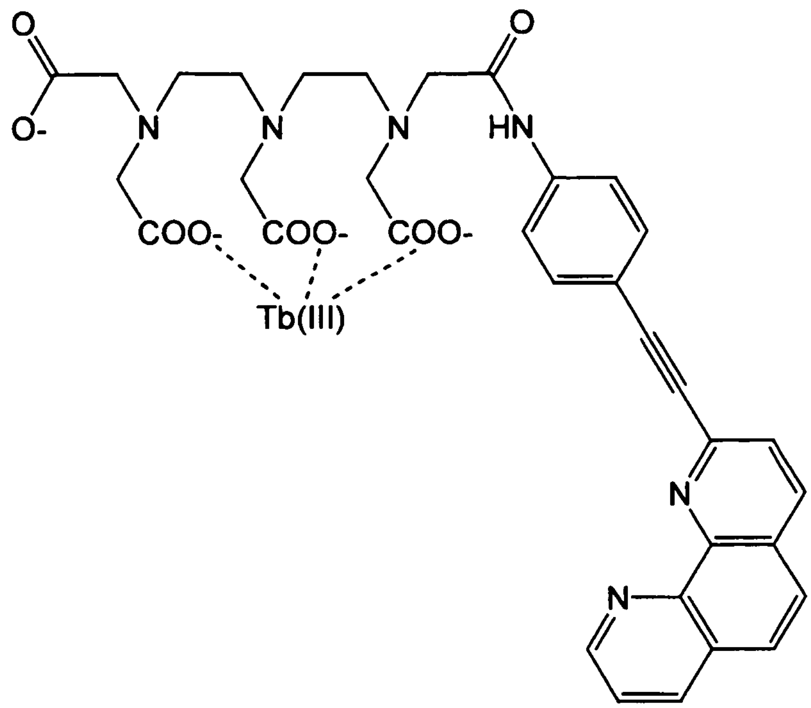

[TbP*BL]$^-$

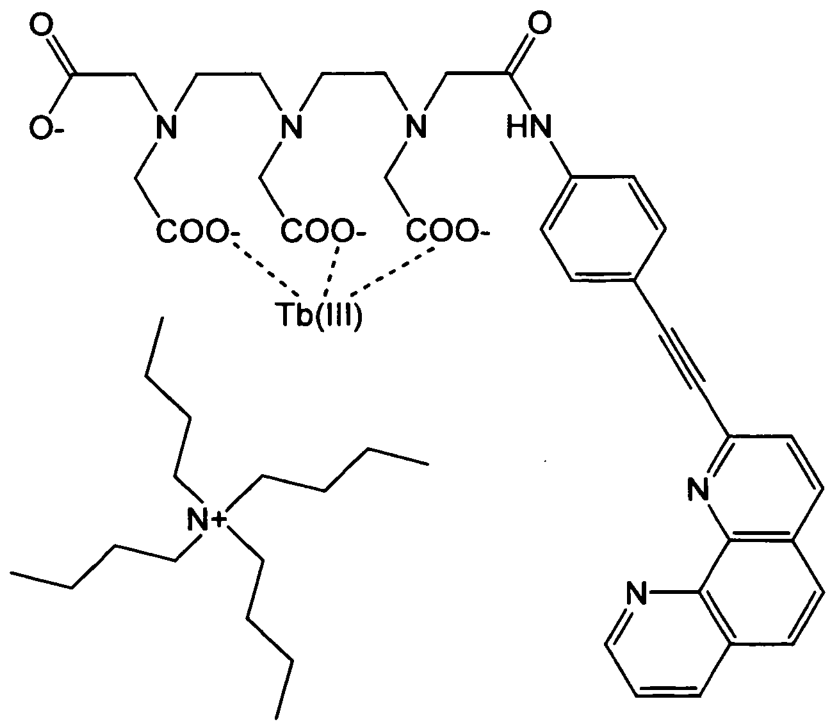

$^{n-}Bu_4N$ [TbP*BL ]

**9.** $P^*BLH_3$-EDA und $P^*BLH_3SP1$

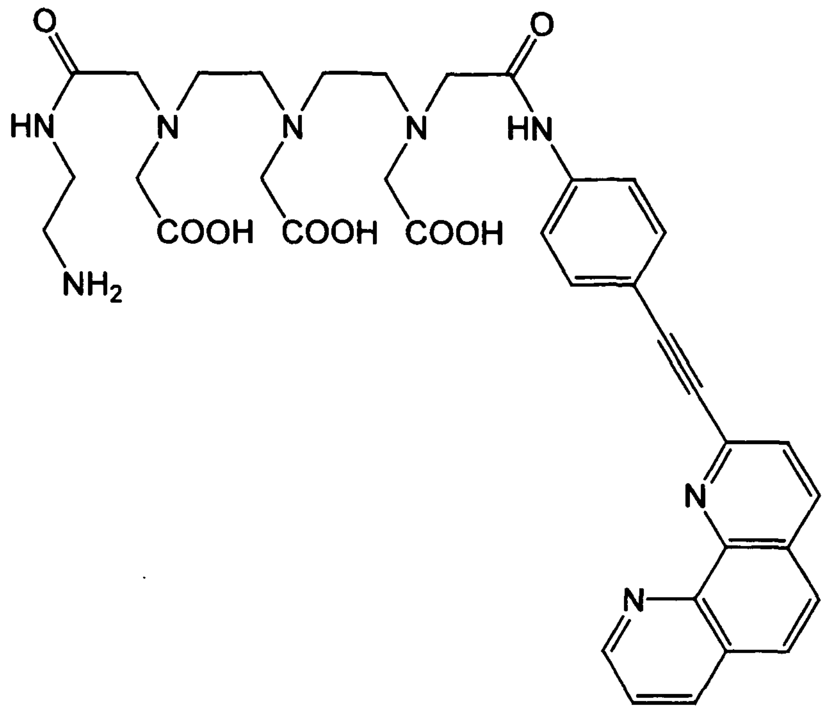

$C_{36}H_{40}N_8O_8$

P*BLH₃-EDA

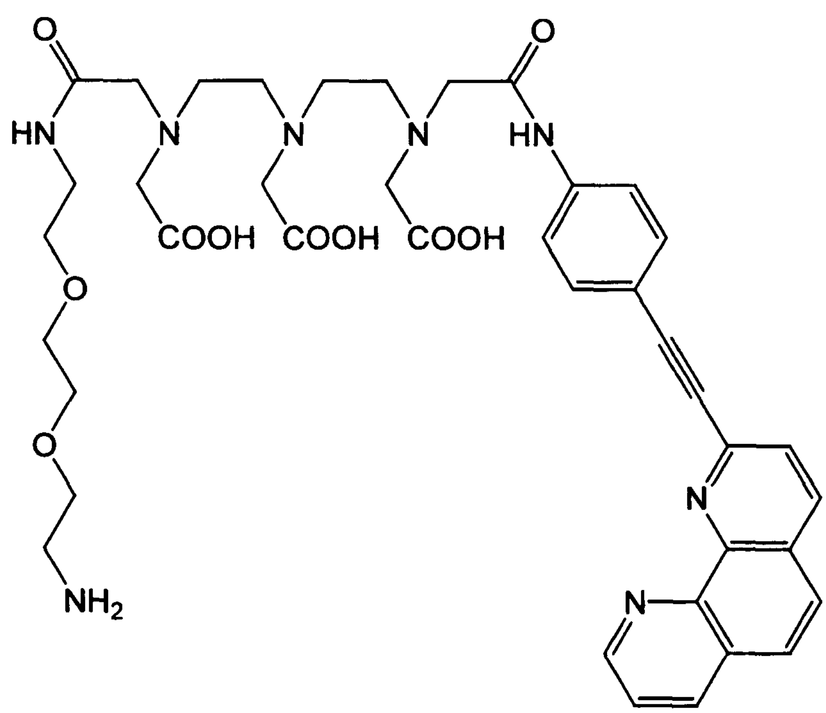

$C_{40}H_{48}N_8$

P*BLH₃-SP1

**10.** Antennenkomplex EuP*BL-EDA und Antennenkomplex EuP*BL-SP1

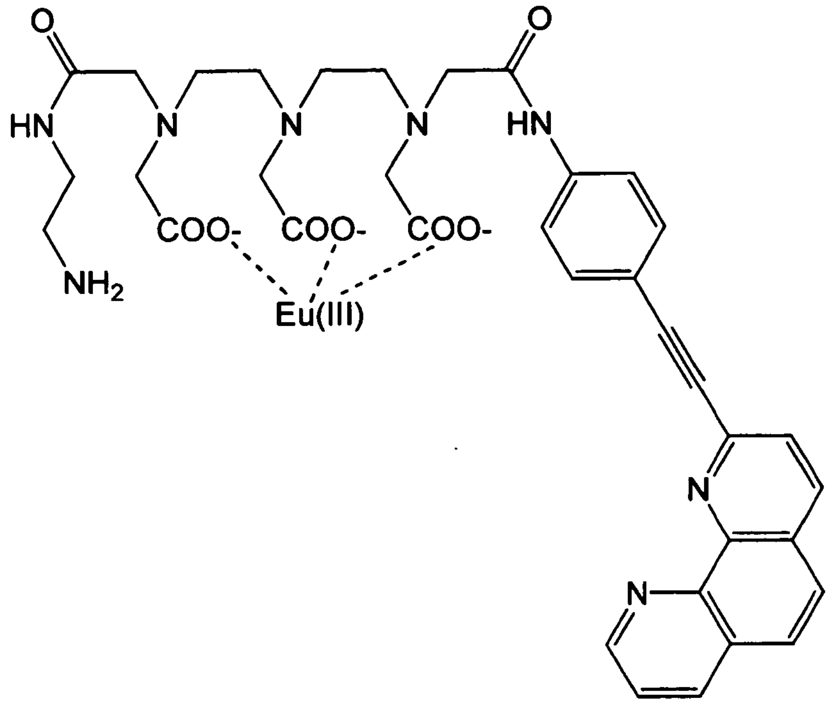

EuP*BL-EDA

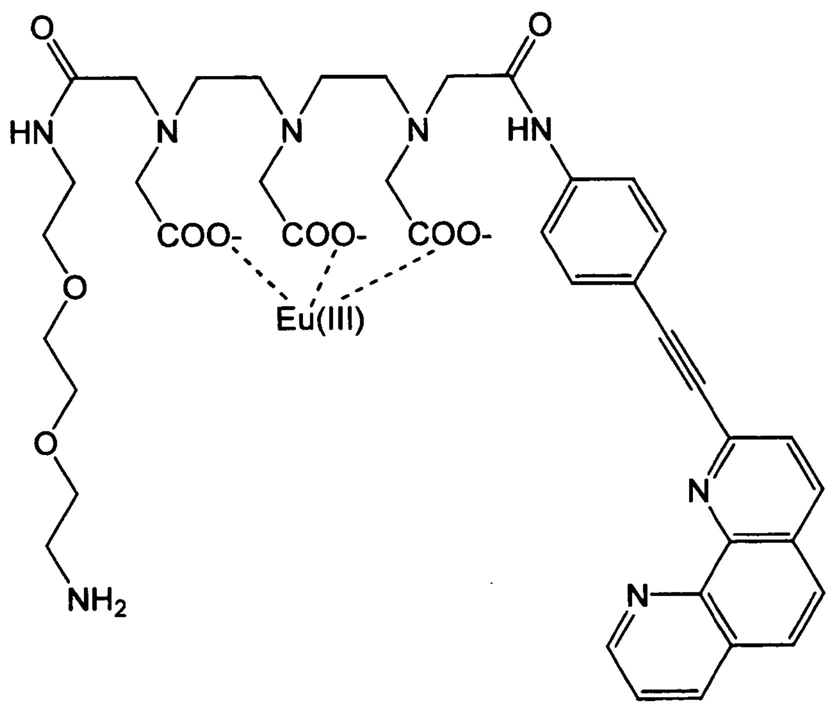

EuP*BL-SP1

**11.** Fluorimetrisches Analysenverfahren auf der Basis von TRF und FRET zum qualitativen Nachweis und zur quantitativen Bestimmung von Biomolekülen,
**dadurch gekennzeichnet, dass** eine Verbindung nach Anspruch 1 durch eine Linkerreaktion kovalent an ein Biomolekül gebunden wird, wobei als zu markierende Biomoleküle Verbindungen aus den Substanzklassen der Peptide, Proteine, Oligonucleotide, Nucleinsäuren wie DNA und RNA, Oligo- und Polysaccharide, Glycoproteine, Phospholipide, niedermolekulare Substrate von Enzymen und niedermolekulare Liganden von Proteinen eingesetzt werden und der Nachweis des Analyten durch eine der folgenden Methoden erfolgt:

a. direkte Messung der Fluoreszenz des Lanthanoid(III)-Ions nach der TRF-Methode oder
b. nach Zugabe eines organischen Farbstoffs aus der Klasse der Polymethinfarbstoffe, gebunden an ein Biomolekül wie vorstehend bezeichnet, zur Messreaktion die von dem Donormolekül auf den organischen Farbstoff, nämlich das Akzeptormolekül übertragene Energie durch Messung der Fluoreszenzemission des Akzeptormoleküls bestimmt wird.

**12.** Verwendung von Ethinylanilin nach den Ansprüchen 1 bis 11 in einem FRET-Bioassay, bestehend aus

- einem Antennenliganden mit dem Linker Ethylendiamin EDA als Feststoff
- N,N'-Carbonyldümidazol CDI oder N-Ethyl-N'-(3-diaminopropyl)-carbodiimid CDD und Triethanolamin/HCl-Puffer zur Markierung
- Eu(III)chlorid als $10^{-3}$ mol/l Lösung in Wasser
- Cy5 Farbstoff mit Säurefunktion als Feststoff, gegebenenfalls gebunden an einen sekundären Antikörper oder Protein A oder Protein G,

wobei

- als Antennenligand P*$BLH_4$ verwendet wird

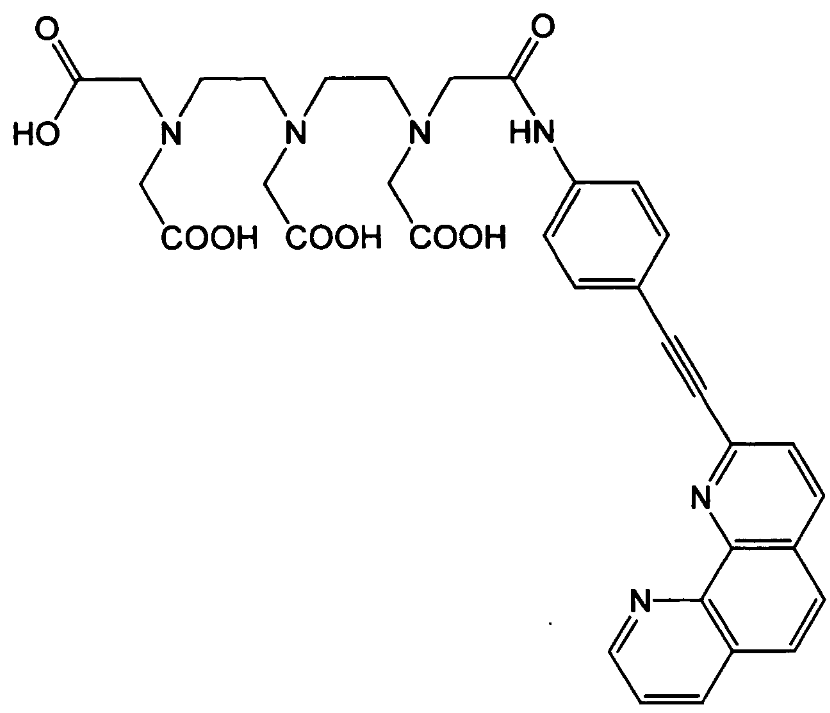

**P*$BLH_4$**

**$C_{34}H_{34}N_6O_9$**

wobei $LH_4$ für Diethylentriaminpentaessigsäure steht und $LH_3$, $LH_2$, LH, L für weitere Dissoziationsstufen der Diethylentriaminpenta-essigsäure stehen,

- der Cy5-Farbstoff mit Säurefunktion Cy5P oder Cy5A oder Cy5E oder Cy5eNCS ist

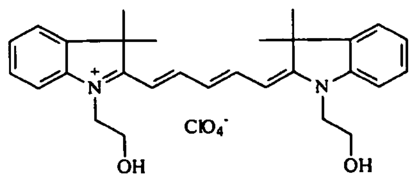

$C_{29}H_{35}N_2O_6Cl$

**Polymethinfarbstoff Cy5E**

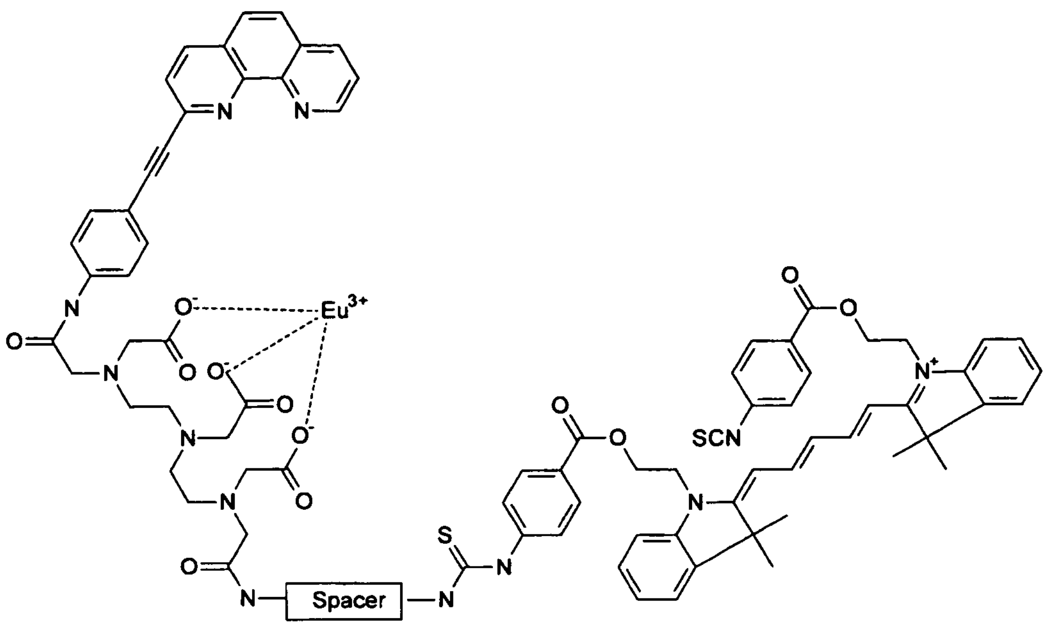

**Akzeptor (Polymethinfarbstoff Cy5ENCS)**

- und die Anregungswellenlänge $\lambda_{exc}$ 360 nm und die Detektionswellenlänge $\lambda_{em}$ 665 nm beträgt.

## Claims

1. Ethinyl aniline corresponding to the formula

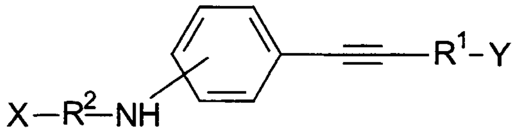

in which

**R¹** is an antenna function selected from the following:
1,10-phenanthroline (1) or fluorene (2) or acetophenone (3) or benzophenone (4) or fluoroenone (5) or xantone (6) or azaxantone (7) or anthraquinone (8) or acridone (9) or quinoline (10) or coumarin (11)

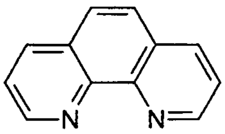
1
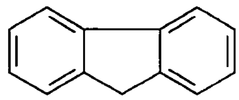
2
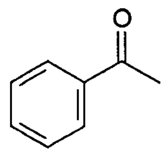
3
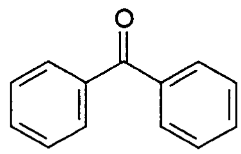
4

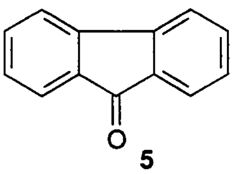
5
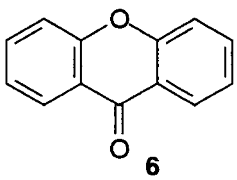
6
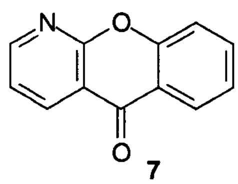
7
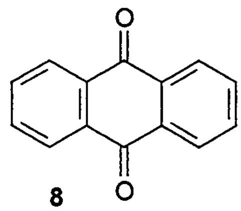
8

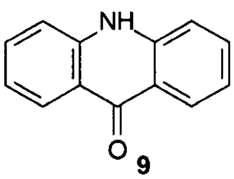
9
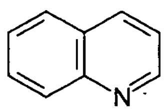
10
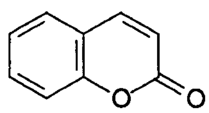
11

**R²** is a chelating agent containing a lanthanoid(III) ion in a coordinated manner, the chelating agent being one of the following:
DTPA (n=1), TTHA (n=2)

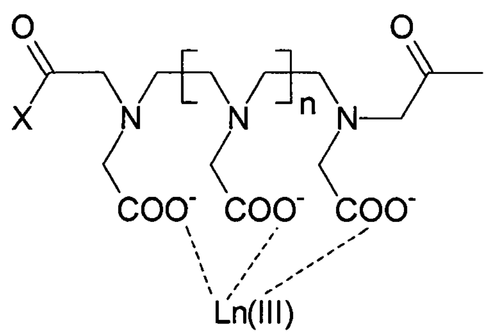

or the TTHA isomer NTTHA

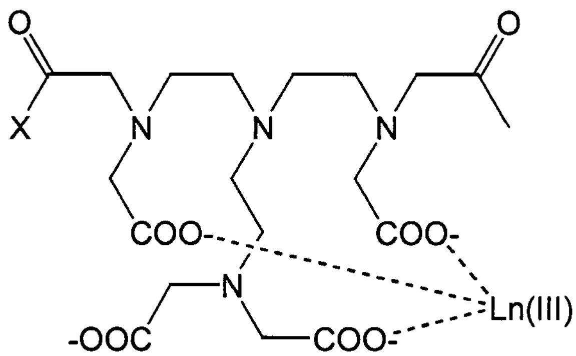

the TTHA derivatives being able to contain a second ethinyl aniline with an antenna function such that three carboxylate radicals are always available for complex formation,
**X** is -OH or a binding group to the biomolecule, which is bonded to a carboxylate radical of the chelating agent via an amide bond, the following being suitable as binding group:
$-NH(CH_2)_2NH_2$, $-NH(CH_2)_2PhNH_2$, $-NH(CH_2)_2PhNCS$, $-NH(CH_2)_nNH(C_3N_3Cl_2)$ with n = 2 to 6 and $-N-H(CH_2)_2O(CH_2)_2NH_2$ (SP1)
**Y** is -H or a binding group to the biomolecule, which is coupled to the antenna function, the binding group being selected from the following:

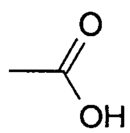

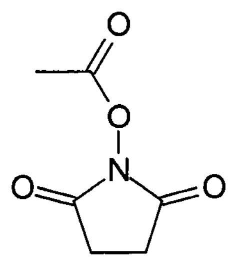

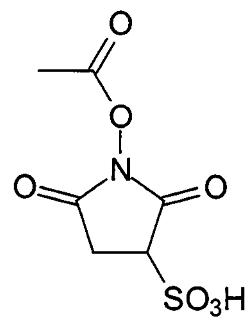

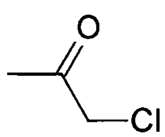

—NCS

$—NH_2$

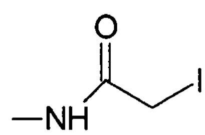

e) the lanthanoid(III) ions being the following: $Eu^{3+}$, $Tb^{3+}$, $Dy^{3+}$, $Sm^{3+}$.

**2.** 2-(4'-Aminophenylethinyl)-1,10-phenanthroline P*B

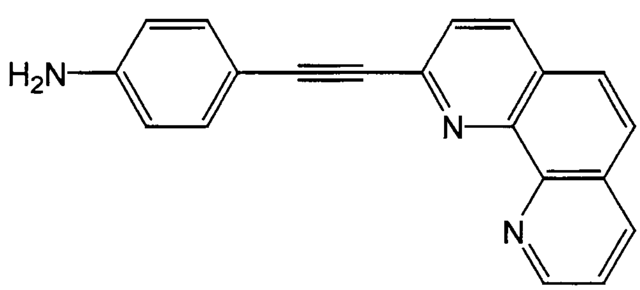

$C_{20}H_{13}N_3$

**3.** Process for the production of 2-(4'-aminophenyl ethinyl)-1,10-phen P*B by reacting 2-chloro-1,10-phenanthroline with p-ethinyl aniline.

**4.** Use of P*B as antenna chromophore

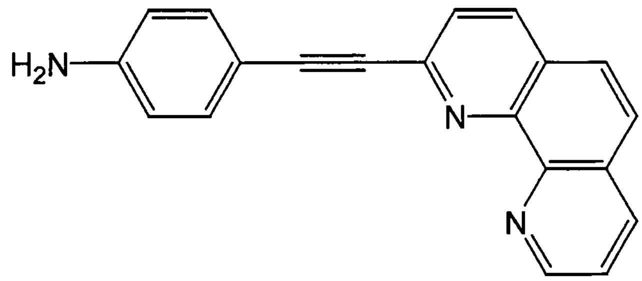

**5.** Chelate ligand $P^*BLH_4$

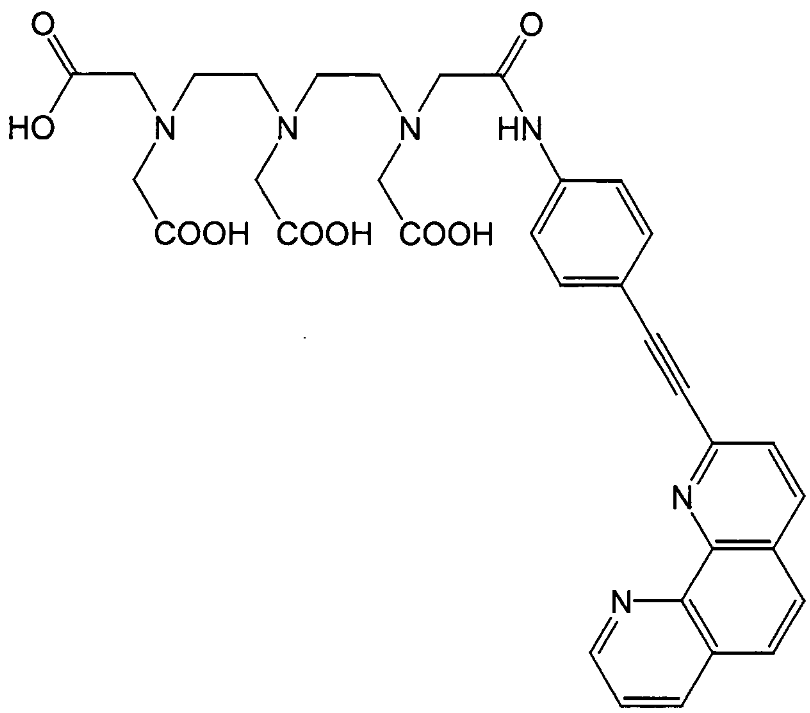

$C_{34}H_{34}N_6O_9$

$LH_4$ representing diethylenetriamine pentaacetic acid and $LH_3$, $LH_2$, LH, L representing further disassociation stages of diethylenetriamine pentaacetic acid.

**6.** Use of the chelate ligand $P^*BLH_4$ as antenna ligand

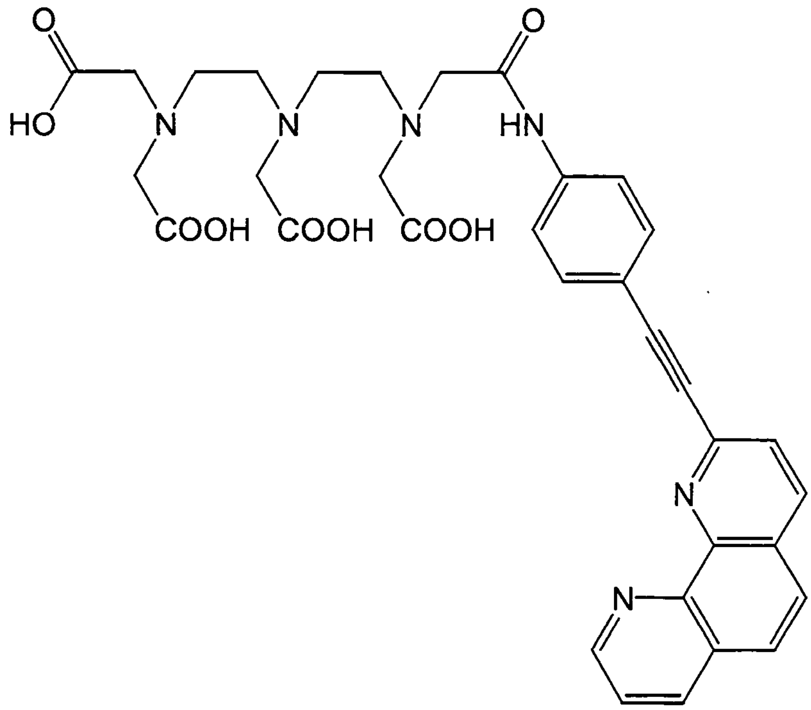

**7.** Complex [EuP*BL)]$^-$ and complex $^{n-}Bu_4N$ [EuP*BL]

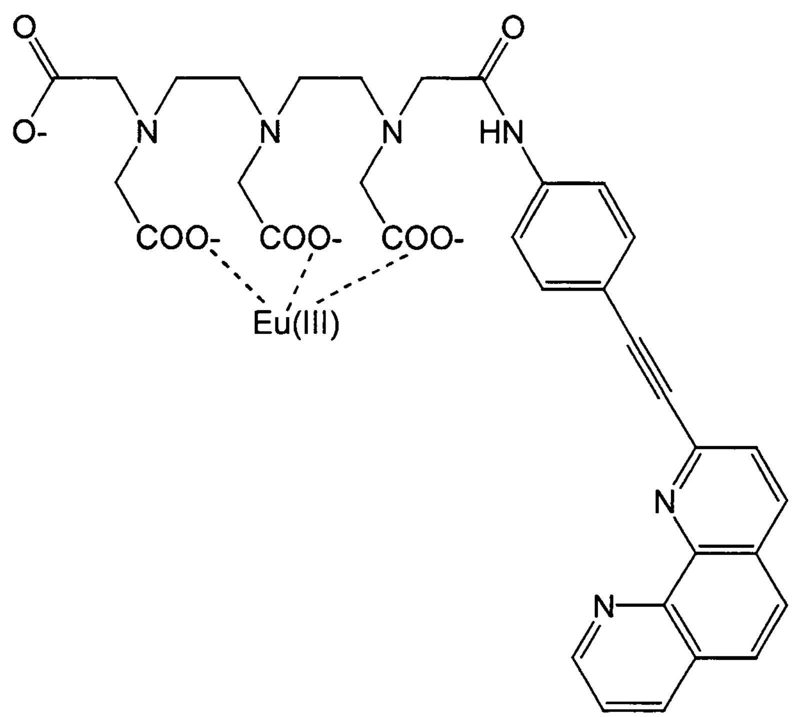

[EuP*BL)]$^-$

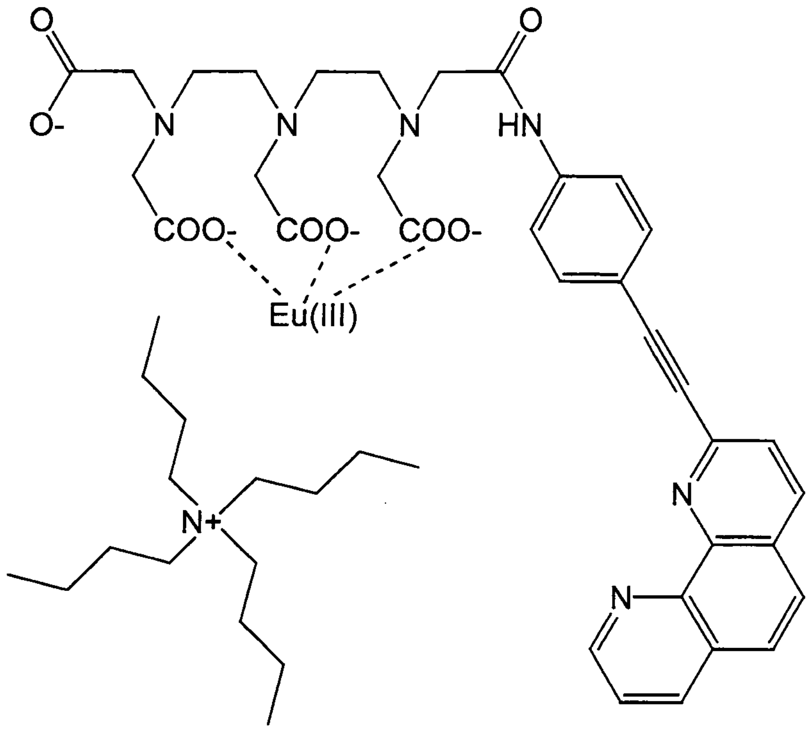

$^{n-}Bu_4N$[ EuP*BL]

**8.** Complex [TbP*BL]$^-$ and complex $^{n-}Bu_4N$ [TbP*BL ]

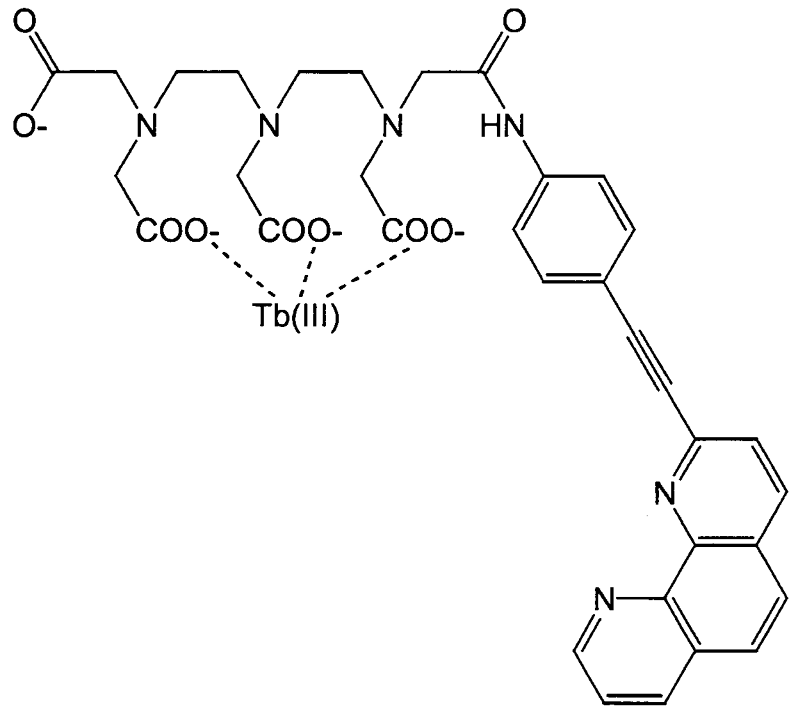

[TbP*BL]$^{-}$

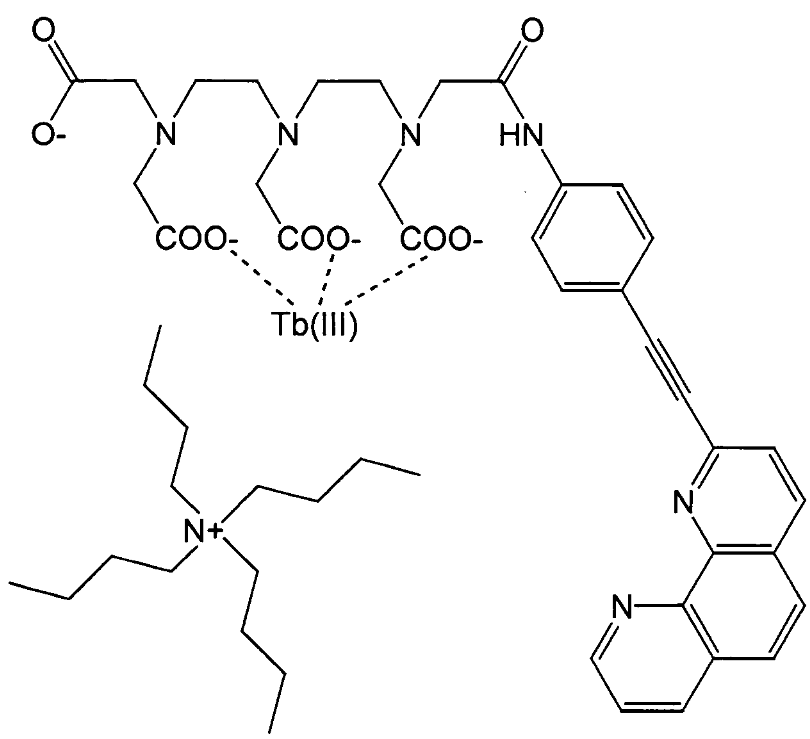

$^{n-}Bu_4N$ [TbP*BL ]

**9.** P*$BLH_3$-EDA und P*$BLH_3$SP1
$C_{36}H_{40}N_8O_8$
P*$BLH_3$-EDA

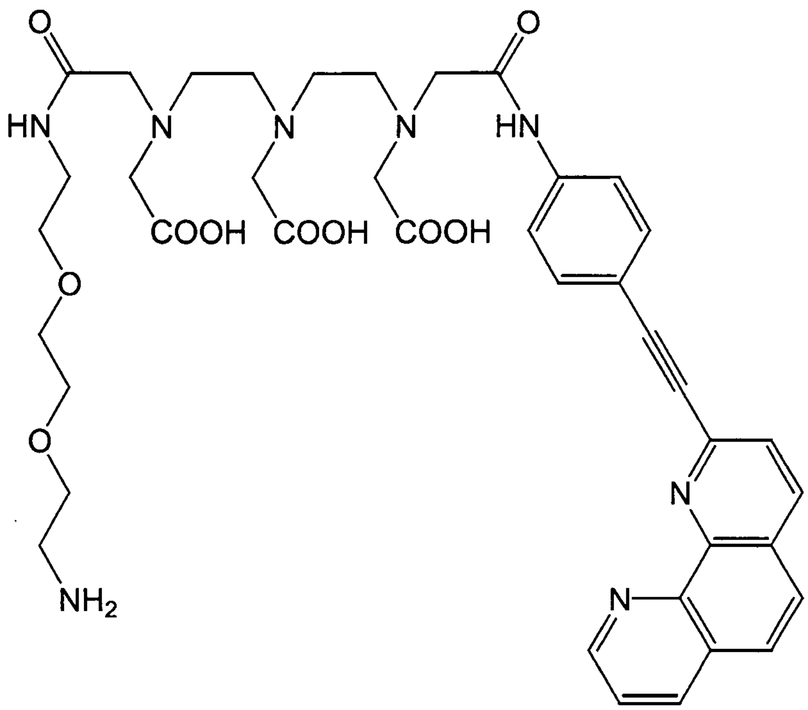

$C_{40}H_{48}N_8$

P*$BLH_3$-SP1

**10.** Antenna complex EuP*BL-EDA and antenna complex EuP*BL-SP1

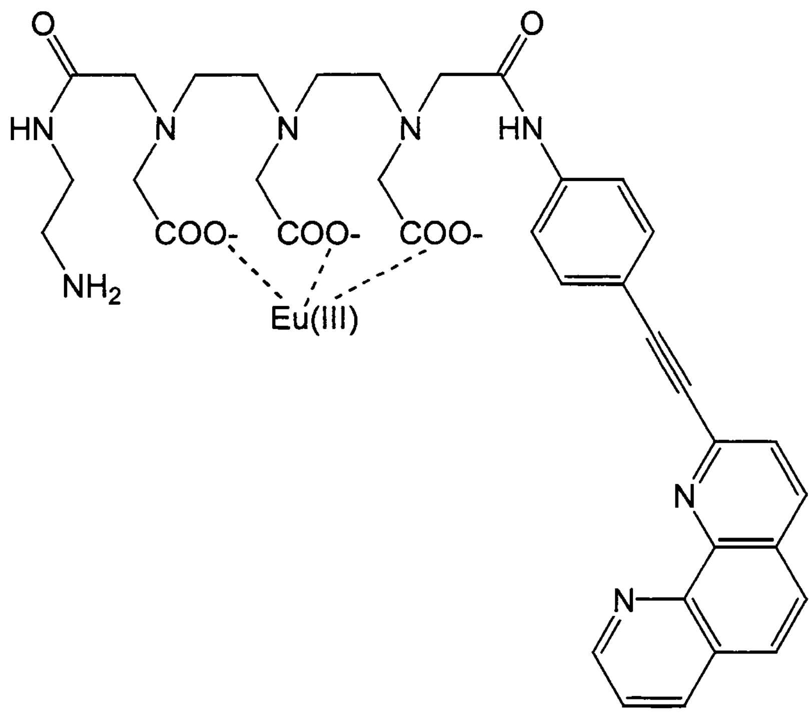

EuP*BL-EDA

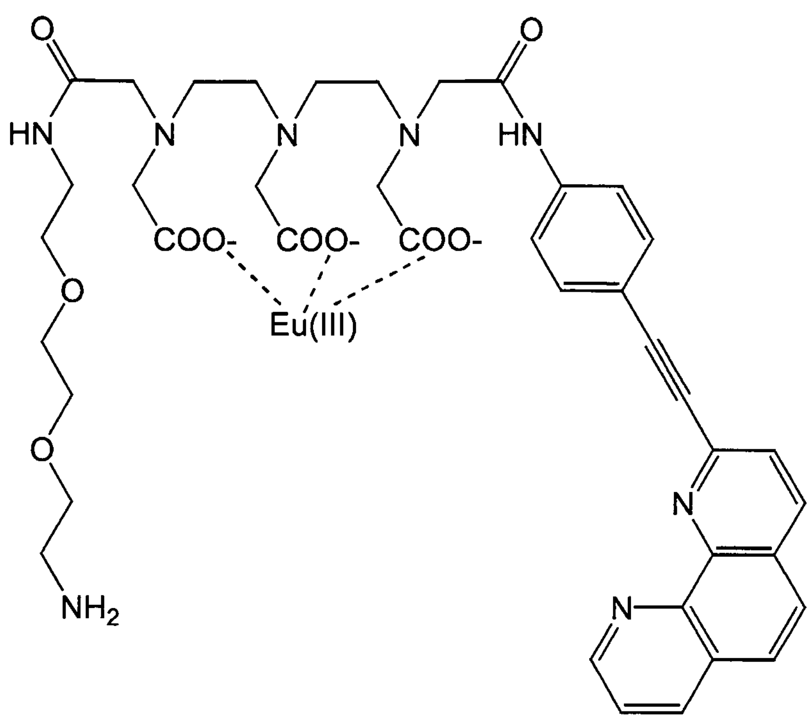

EuP*BL-SP1

**11.** Fluorometric method of analysis based on TRF and FRET for the qualitative detection and the quantitative determination of biomolecules **characterised in that** a compound according to claim 1 is covalently bonded to a biomolecule by a linker reaction, compounds of the group of substances of the peptides, proteins, oligonucleotides, nucleic acids such as DNA and RNA, oligosaccharides and polysaccharides, glycoproteins, phospholipids, low-molecular substrates of enzymes and low molecular ligands of proteins being used as biomolecules to be labelled and the detection of the analyte taking place by one of the following methods:

a. the direct measurement of the fluorescence of the lanthanoid(III) ion according to the TRF method or
b. following the addition of an organic dye from the class of polymethine dyes, bound to a biomolecule, as indicated above, the energy transferred by the donor molecule to the organic dye, namely the acceptor molecule, is determined for the measuring reaction by measuring the fluorescence emission of the acceptor molecule.

**12.** Use of ethinyl aniline according to claims 1 to 11 in a FRET bioassay, consisting of

- an antenna ligand with the linker ethylene diamine EDA as solid
- N,N'-carbonyl diimidazole CDI or N-ethyl-N'-(3-diaminopropyl) carbodiimide CDD and triethanol amine/HCl buffer for labelling
- Eu(III) chloride as $10^{-3}$ mole/l solution in water
- Cy5 dye with an acid function as solid, if necessary bound to a secondary antibody or protein A or protein G,
- P*$BLH_4$ being used as antenna ligand,

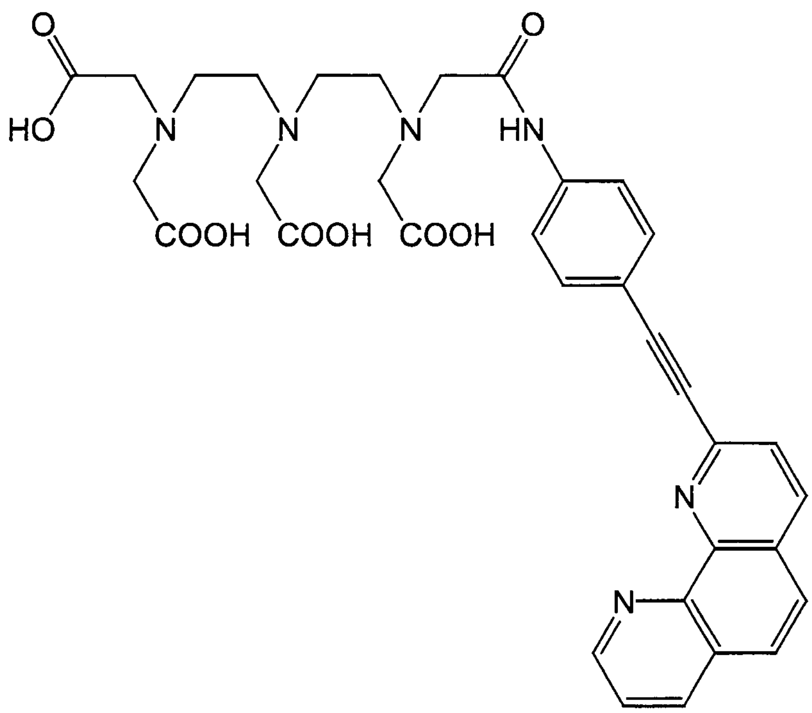

P*$BLH_4$

$C_{34}H_{34}N_6O_9$

$LH_4$ representing diethylenetriamine pentaacetic acid and $LH_3$, $LH_2$, LH, L representing further dissociation stages of diethylenetriamine pentaacetic acid,
- the Cy5 dye with an acid function being Cy5P or Cy5A or Cy5E or Cy5eNCS

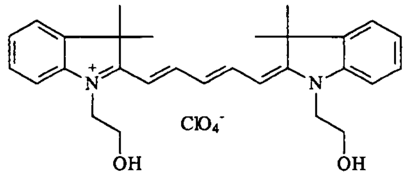

$C_{29}H_{35}N_2O_6Cl$

polymethine dye **Cy5E**

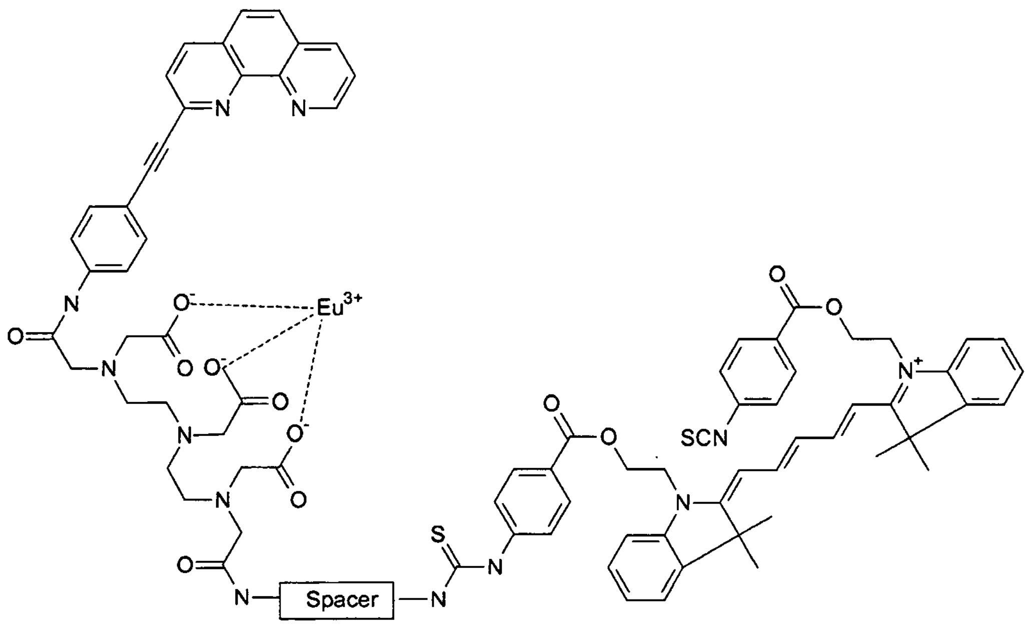

acceptor (polymethine dye **Cy5ENCS**)

- and the excitation wavelength being $\lambda_{exc}$ 360 nm and the detection wavelength $\lambda_{em}$ 665 nm.

## Revendications

**1.** Une éthynylaniline selon la formule

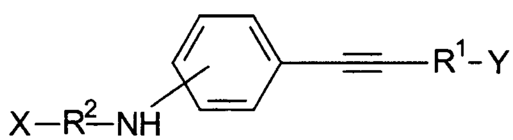

dans laquelle

**$R^1$** est un groupe à fonction d'antenne, choisi parmi les groupes suivants : 1,10-phénanthroline (1) ou fluorène (2) ou acétophénone (3) ou benzophénone (4) ou fluorénone (5) ou xanthone (6) ou azaxanthone (7) ou anthraquinone (8) ou acridone (9) ou quinoléine (10) ou coumarine (11)

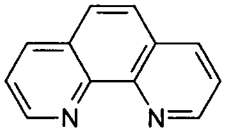
**1**

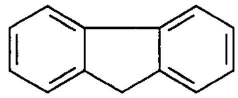
**2**

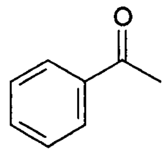
**3**

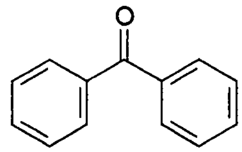
**4**

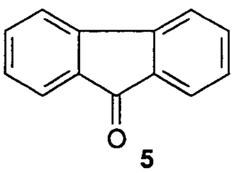
**5**

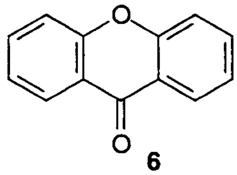
**6**

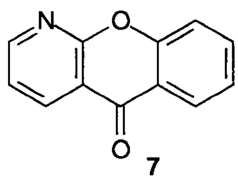
**7**

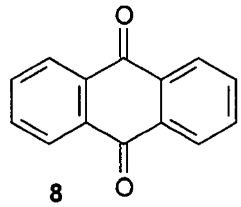
**8**

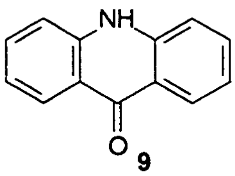
**9**

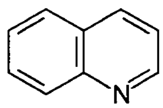
**10**

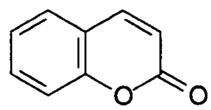
**11**

**$R^2$** est un chélateur comprenant un ion de lanthanides(III) en coordination, ledit chélateur étant choisi parmi les chélateurs suivants :

DTPA (n=1), TTHA (n=2)

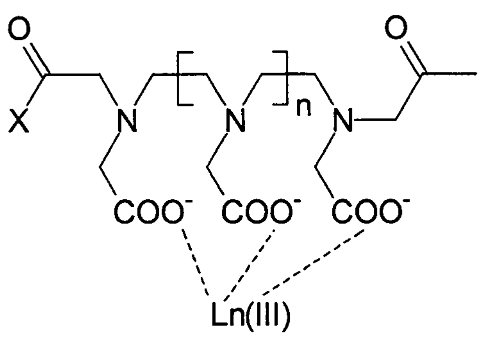

ou l'isomère de TTHA appelé NTTHA

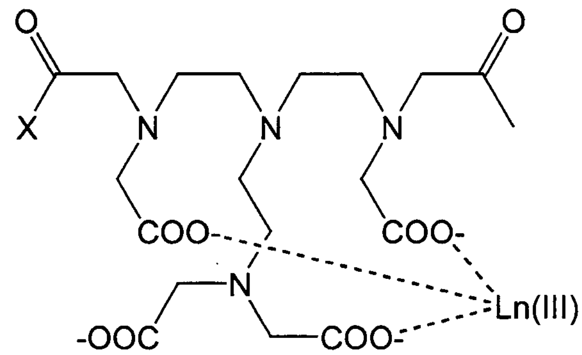

les dérivés de TTHA pouvant contenir une deuxième éthynylaniline munie d'un groupe à fonction d'antenne, de façon à disposer toujours de trois restes carboxyle pour la complexation,

**X** représente -OH ou un groupe promouvant l'adhésion à la biomolécule, ledit groupe étant lié, à travers une liaison amide, à un reste carboxyle du chélateur, ledit groupe promouvant l'adhésion étant choisi parmi les groupes suivants :

$-NH(CH_2)_2NH_2$, $-NH(CH_2)_2PhNH_2$, $-NH(CH_2)_2PhNCS$, $NH(CH_2)_nNH(C_3N_3Cl_2)$ avec n = 2 à 6 ainsi que-NH $(CH_2)_2O\ (CH_2)_2NH_2$(SP1)

**Y** représente -H ou un groupe promouvant l'adhésion à la biomolécule, ledit groupe étant couplé au groupe à fonction d'antenne et étant choisi parmi les groupes suivants :

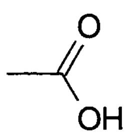

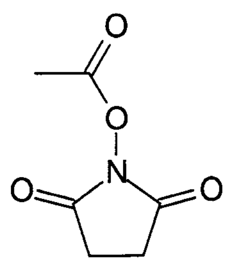

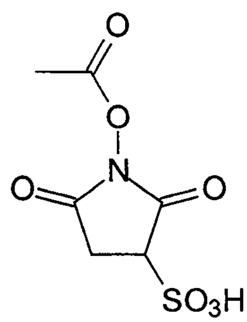

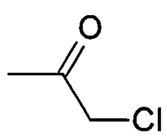

—NCS

$—NH_2$

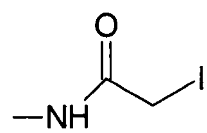

e) les ions d'un lanthanide(III) sont les suivants : $Eu^{3+}$ , $Tb^{3+}$, $Dy^{3+}$, $Sm^{3+}$.

**2.** 2-(4'-Aminophénylethynyl)-1,10-phénanthroline P*B

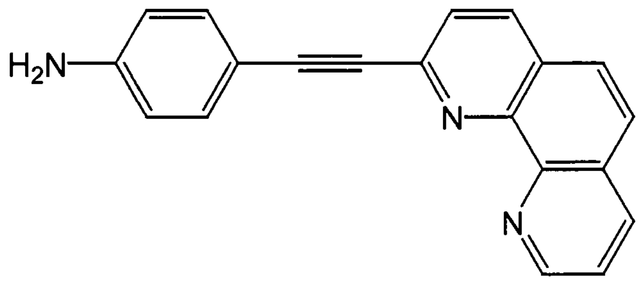

$C_{20}H_{13}N_3$

3. Procédé de synthèse de 2-(4'-aminophénylethynyl)-1,10-phén P*B par réaction de 2-chloro-1,10-phénanthroline avec p-éthynylaniline

4. Utilisation de P*B comme chromophore d'antenne

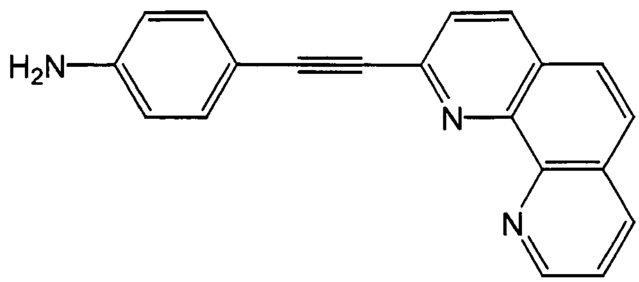

5. Ligand de chélation $P^*BLH_4$

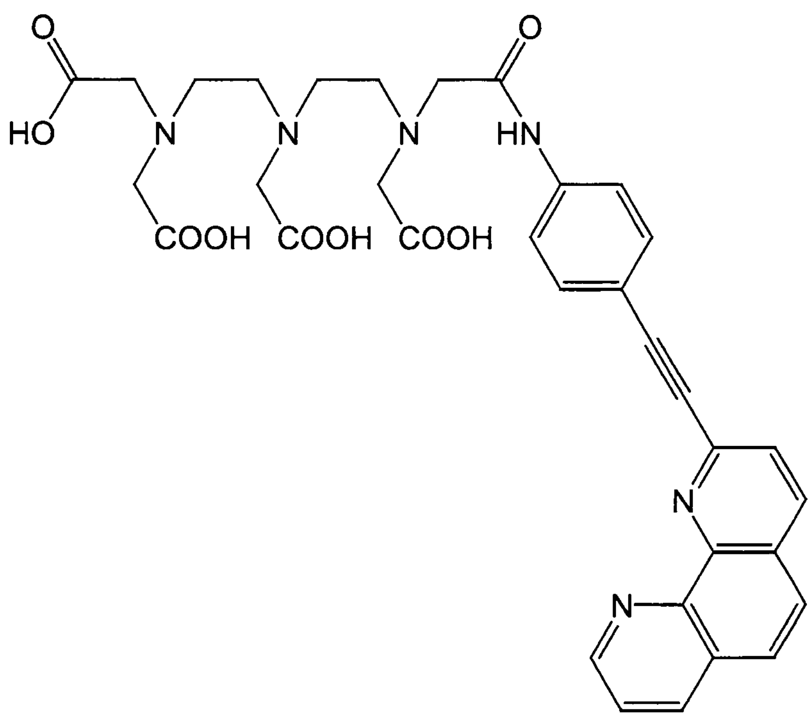

$C_{34}H_{34}N_6O_9$

$LH_4$ représentant l'acide diéthylène-triamine-pentaacétique et $LH_3$, $LH_2$, LH, L représentant d'autres niveaux de dissociation de l'acide diéthylène-triamine-pentaacétique

**6.** Utilisation du ligand de chélation $P^*BLH_4$ comme ligand d'antenne

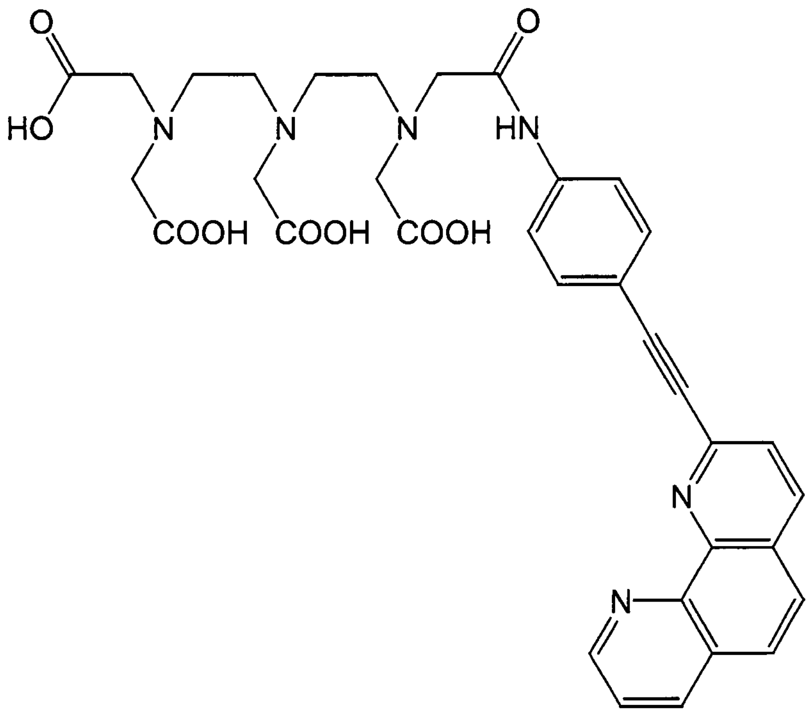

**7.** Complexe $[EuP^*BL)]^-$ et complexe $^{n-}Bu_4N[EuP^*BL]$

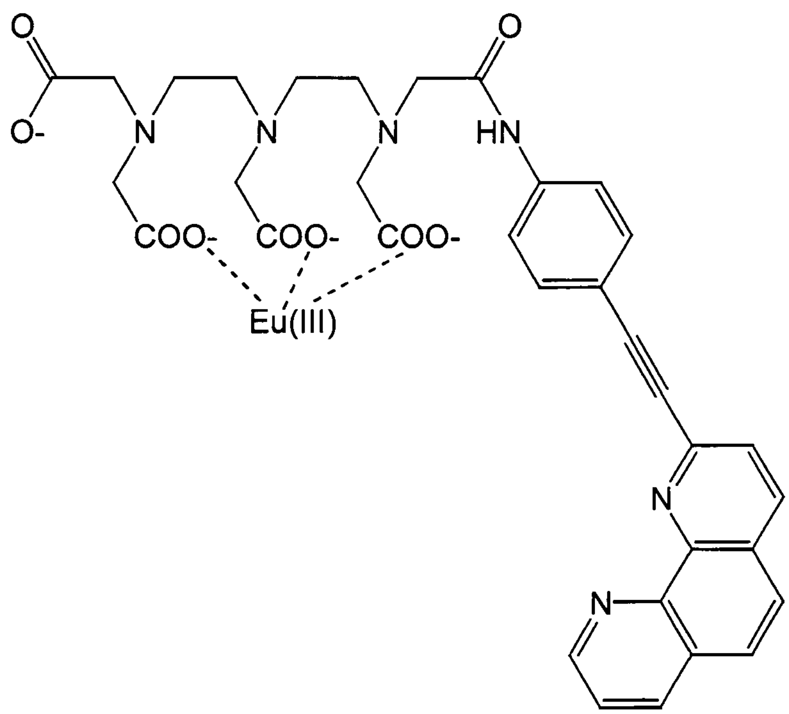

*[EuP*BL)]⁻*

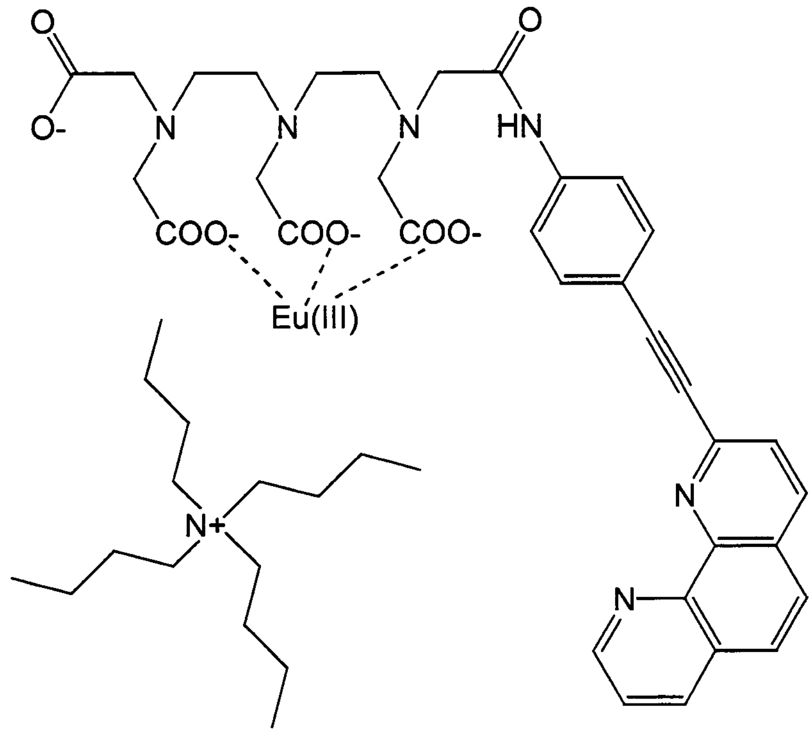

$^{n-}Bu_4N$ [ EuP*BL]

**8.** Complexe $[TbP*BL]^-$ et complexe $^{n-}Bu_4N$ [TbP*BL]

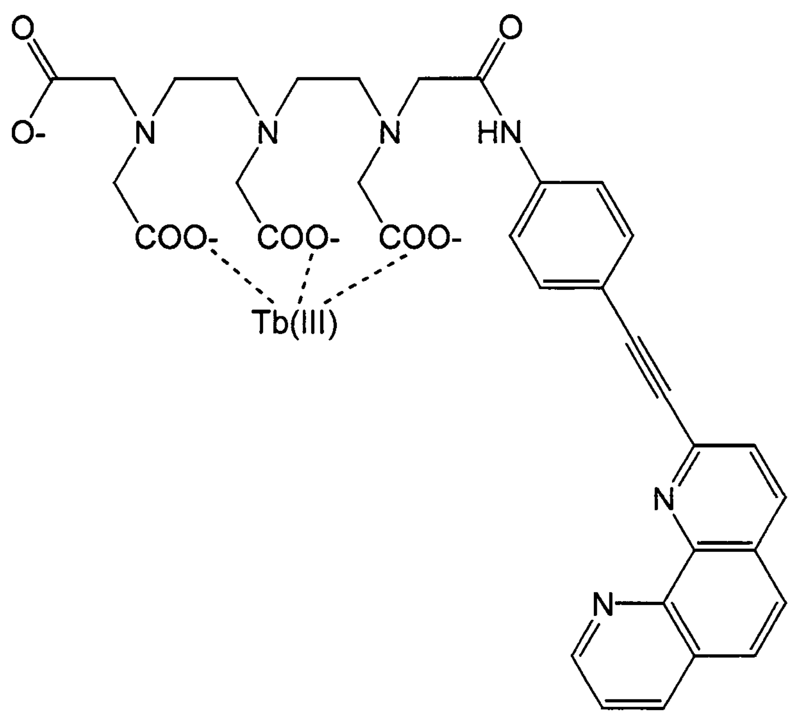

$[TbP*BL]^-$

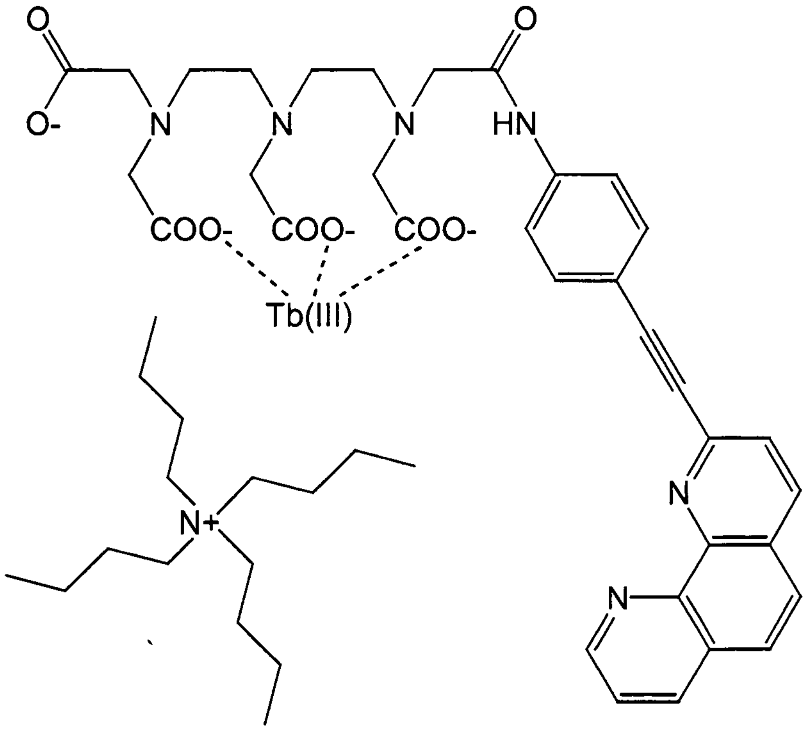

$^{n-}Bu_4N$ [TbP*BL ]

**9.** P*BL$H_3$-EDA et P*BL$H_3$SP1

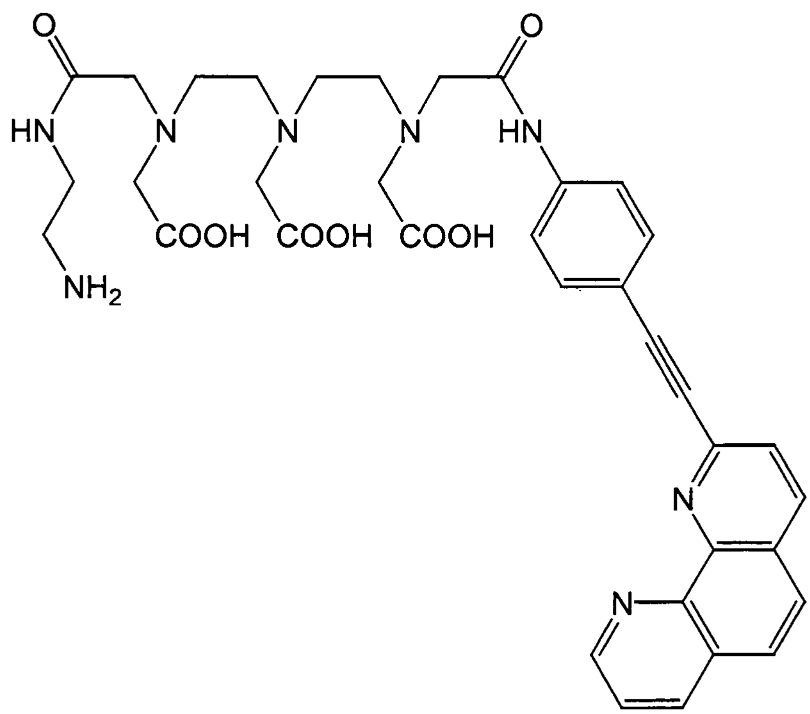

$C_{36}H_{40}N_8O_8$

P*BLH$_3$-EDA

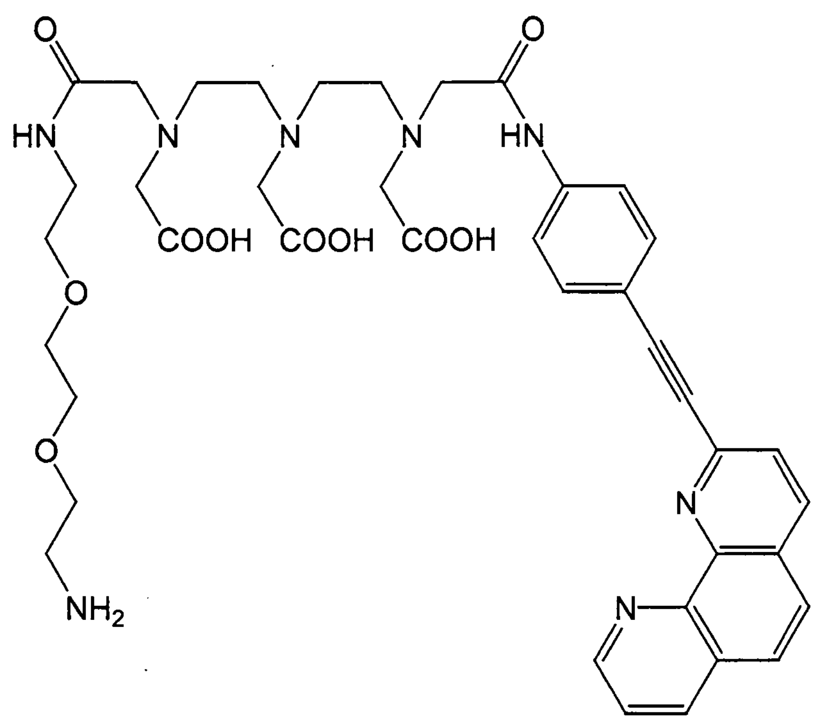

$C_{40}H_{48}N_8$

P*BLH$_3$-SP1

**10.** Complexe d'antenne EuP*BL-EDA et complexe d'antenne EuP*BL-SP1

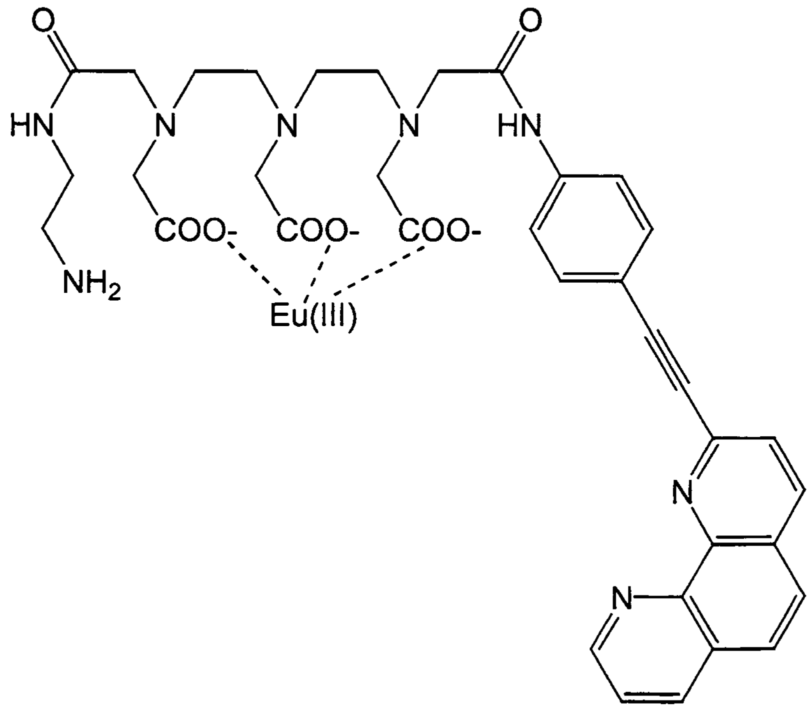

EuP*BL-EDA

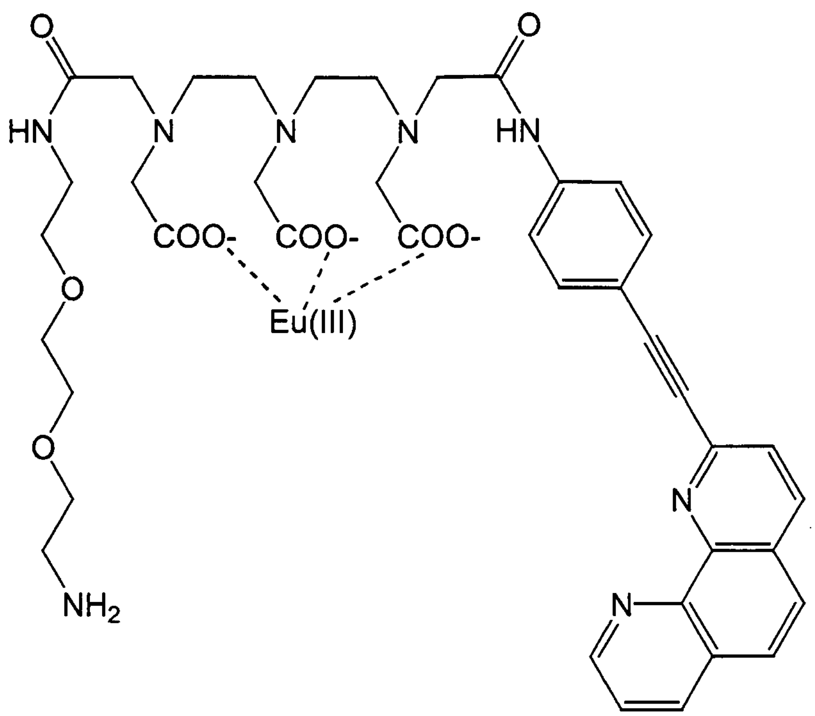

EuP*BL-SP1

**11.** Méthode d'analyse fluorimétrique à base de TRF et FRET destinée à la détection qualitative ainsi qu'au dosage quantitatif de biomolécules, **caractérisée en ce qu'** l'on fait entrer le composé selon la revendication 1 en liaison

covalente avec une biomolécule, la biomolécule à marquer pouvant être issue de classes de substances telles que les peptides, les protéines, les oligonucléotides, les acides nucléiques comme l'ADN et l'ARN, les oligo- et polysaccharides, les glycoprotéines, les phospholipides, les substrats d'enzymes de faible masse moléculaire et les ligands de protéines de faible masse moléculaire, et la détection de l'analyte étant réalisée par une des méthodes suivantes :

c. Mesure directe des la fluorescence de l'ion de lanthanides(III) selon la méthode TRF ou
d. après l'adjonction d'un colorant organique appartenant à la classe des colorants de polyméthine et lié à une biomolécule selon la définition ci-dessus pour que l'énergie soit transférée de la molécule de type donneur à la molécule de type accepteur, à savoir le colorant organique, suivie d'une mesure de l'émission de fluorescence de la molécule de type accepteur.

**12.** Utilisation de l'éthynylaniline selon les revendications 1 à 11 dans un système de dosage biologique FRET, constitué des éléments suivants :

- un ligand d'antenne avec un groupe de liaison éthylène-diamine EDA, sous forme d'un solide
- du N,N'-carbonyldiimidazole CDI ou du N-éthyl-N'-(3-diaminopropyl)-carbodiimide CDD et du tampon triéthanolamine/HCl pour le marquage
- du chlorure d'Eu(III) sous forme d'une solution $10^{-3}$ mol/l dans de l'eau
- un colorant Cy5 pourvu d'un groupe fonctionnel carboxyle sous forme d'un solide, le cas échéant lié à un anticorps secondaire ou une protéine A ou une protéine G

pour lesquels

- le ligand d'antenne utilisé est du P*$BLH_4$

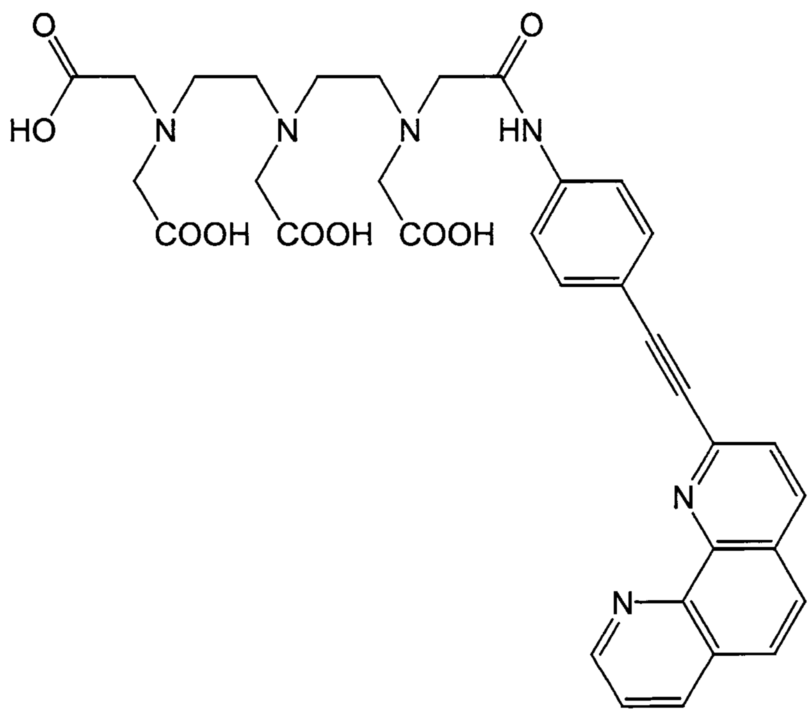

P*$BLH_4$

$C_{34}H_{34}N_6O_9$

$LH_4$ représentant l'acide diéthylène-triamine-pentaacétique et $LH_3$, $LH_2$, LH, L représentant d'autres niveaux de dissociation de l'acide diéthylène-triamine-pentaacétique
- le colorant Cy5 pourvu d'un groupe fonctionnel carboxyle est du Cy5P ou Cy5A ou Cy5E ou Cy5eNCS

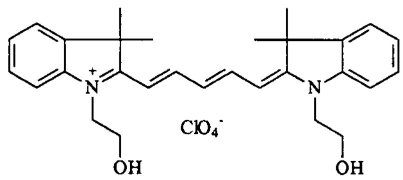

$C_{29}H_{35}N_2O_6Cl$

Colorant de polyméthine **Cy5E**

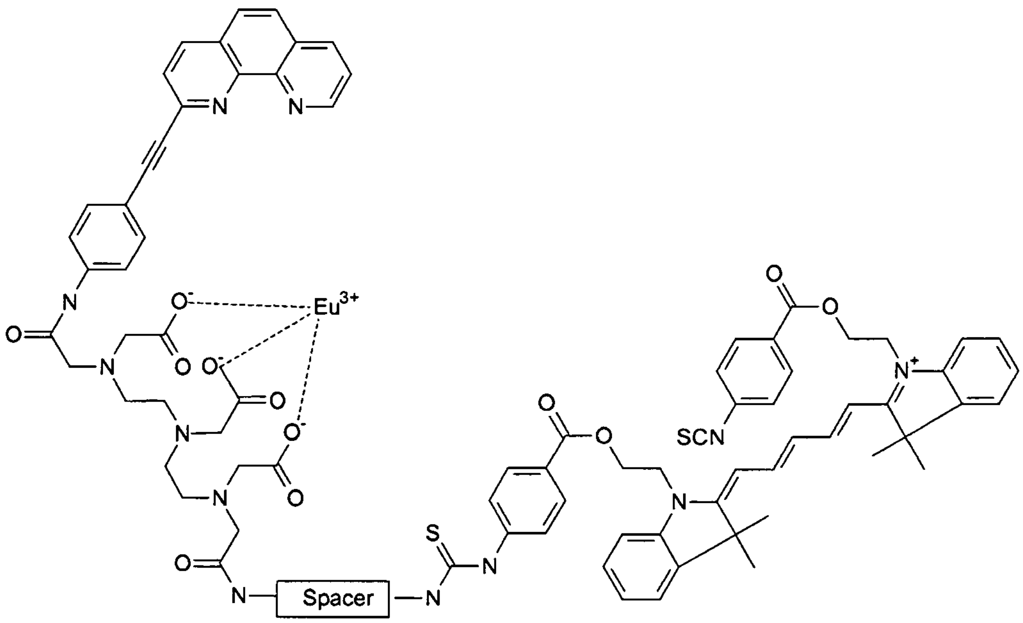

Accepteur (colorant de polyméthine **Cy5ENCS**)

- la longueur d'onde d'excitation $\lambda_{exc}$ est de 360 nm et la longueur d'onde de détection $\lambda_{em}$ est de 665 nm.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4374120 A
- WO 0001663 A
- US 6217848 B
- US 6190641 B
- DE 4222255
- US 5032677 A
- DE 69813850
- DE 10259677


### In der Beschreibung aufgeführte Nicht-Patentliteratur


- **MATSUMOTO, K. et al.** *RIKEN Review 35,* Mai 2001
- **TAKALO,H.** *Journal of Alloys and Compounds,* 1995, vol. 225, 511-14
- **HEMMILÄÄ, I. ; WEBB, S.** *DDT,* 1997, vol. 2, 373-381